# EUROPEAN PATENT APPLICATION

(11) **EP 4 748 401 A1**
(43) Date of publication of application: **27.05.2026**
(21) Application number: 24842397.2
(22) Date of filing: 17.07.2024
(51) Int. Cl.: A61K 48/00, C12N 15/63, C12N 15/81

(54) **REPORTING SYSTEM FOR SCREENING DRUGS TARGETING RNA REPEAT SEQUENCES**

(30) Priority: 17.07.2023 CN 202310875071
(71) Applicant: Shanghai Institute of Nutrition and Health, Chinese Academy of Sciences, Shanghai 200031 (CN)
(72) Inventor: MAO, Miaowei, Shanghai 200031 (CN); WANG, Zefeng, Shanghai 200031 (CN); WEI, Tong, Shanghai 200031 (CN)
(74) Representative: dompatent
(86) International application number: PCT/CN2024/106008
(87) International publication number: WO 2025/016408

(57) **Abstract**

Provided is a reporting system which may be used to screen drugs targeting pathogenic RNA repeat sequences, specifically relating to a reporting system simultaneously containing RNA repeat sequences of different lengths, which may be used to screen drugs targeting pathogenic RNA repeat sequences, and related basic research and development work. Provided are a nucleic acid construct group for screening drugs treating diseases related to long RNA repeat sequences, as well as a vector and a screening system based on the nucleic acid construct group.

## Description

### Technical field

The present invention relates to the field of biotechnology, in particular to a reporting system usable for screening drugs targeting RNA repeat sequences.

### Background

RNA repeat sequences refer to repetitive sequences present in RNA molecules, which are widely distributed in the human transcriptome. Under certain circumstances, the length of such repeat sequences may reach a certain level, thereby leading to the occurrence of specific disease-related RNA repeat sequence-associated diseases. Such diseases are typically caused by inconsistent lengths of RNA repeat sequences carried by the two alleles in the human body, and result from abnormal expansion of microsatellite sequences in the genome. One allele with short repeat sequences is a gene with normal functions, while the other allele with an aberrant length of repeat sequences may cause diseases.

Huntington's disease is an inherited neurodegenerative disease, and its pathogenesis is associated with the expansion of long CAG repeat sequences in the *HTT* gene. The length of CAG repeats varies in the population, with 35 CAG repeats as the critical threshold; a number greater than 35 CAG repeats tends to cause the disease. This disease not only leads to the deterioration of intellectual, motor and mental functions, but also has no radical cure at present. Myotonic Dystrophy Type 1 (DM1) is an inherited muscular disease caused by the presence of long CUG repeat sequences with abnormal length in the 3'UTR region of the *DMPK* gene. Patients with DM1 exhibit severe abnormalities in muscle, heart, endocrine, nervous systems and the like, and the treatment thereof is highly challenging. However, there are currently no effective therapeutic methods for such RNA repeat sequence-associated diseases, which brings enormous distress to patients.

The pathogenic mechanisms of these RNA repeat sequence-associated diseases share certain commonalities, including corresponding RNA-protein complexes, ribosomes and the like, while some specific mechanisms also exist that require further in-depth research and understanding.

Current drug screening systems for such diseases only evaluate the clearance effects on pathogenic long RNA repeat sequences, but do not simultaneously consider the off-target effects on normal short RNA repeat sequences. An optimal system should be able to characterize or screen drugs with strong clearance activity against pathogenic long RNA repeat sequences, while also characterizing their off-target effects on normal short RNA repeat sequences, thereby accelerating the screening process, improving the selectivity of the resulting candidate drugs or molecules, and providing better therapeutic effects for patients.

Therefore, there is an urgent need in the art to develop a screening or reporting system capable of rapidly and accurately screening potential drugs for the treatment of RNA repeat sequence-associated diseases.

### Summary of the invention

The objective of the present invention is to provide a screening or reporting system capable of rapidly and accurately screening potential drugs for the treatment of RNA repeat sequence-associated diseases.

In the first aspect of the present invention, it provides a set of nucleic acid constructs, comprising: a first nucleic acid construct and a second nucleic acid construct;
wherein the first nucleic acid construct comprises a first expression cassette, the first expression cassette expresses a long RNA repeat sequence, and the first expression cassette comprises a repeat sequence unit of 3-6 nucleotides, wherein the number of repetitions of the repeat sequence is 41-2000, preferably 45-1500, and more preferably 50-1000;
and the second nucleic acid construct comprises a second expression cassette, the second expression cassette expresses a short RNA repeat sequence, and the second expression cassette comprises a repeat sequence unit of 3-6 nucleotides corresponding to the first expression cassette, wherein the number of repetitions of the repeat sequence is 2-40, preferably 5-40.

In another preferred embodiment, the first expression cassette further comprises a first promoter and a coding sequence of a first detectable label.

In another preferred embodiment, the second expression cassette further comprises a second promoter and a coding sequence of a second detectable label.

In another preferred embodiment, the repeat sequence unit is selected from the group consisting of: CAG, GCG, CGG, CTG, CCG, GAA, CCCCGCCCCGCG, ATTCT/ATTGT, CCTG, TGGAA, GGCCTG, GGGGCC, CCCTCT, ATTTC, AAGGG, GCA, and different equivalent sequences (e.g., CAG repeats and AGC repeats, CTG repeats and TGC repeats, etc., are equivalent sequences), and combinations thereof.

In another preferred embodiment, the "corresponding to" means that:
the first expression cassette comprises a repeat sequence unit CTG, and the second expression cassette comprises the repeat sequence unit CTG;
the first expression cassette comprises a repeat sequence unit CCTG, and the second expression cassette comprises the repeat sequence unit CCTG;
the first expression cassette comprises a repeat sequence unit CAG, and the second expression cassette comprises the repeat sequence unit CAG;
the first expression cassette comprises a repeat sequence unit TGGAA, and the second expression cassette comprises the repeat sequence unit TGGAA;
the first expression cassette comprises a repeat sequence unit GGGGCC, and the second expression cassette comprises the repeat sequence unit GGGGCC;
the first expression cassette comprises a repeat sequence unit CGG, and the second expression cassette comprises the repeat sequence unit CGG;
the first expression cassette comprises a repeat sequence unit GCG, and the second expression cassette comprises the repeat sequence unit GCG;
the first expression cassette comprises a repeat sequence unit CCG, and the second expression cassette comprises the repeat sequence unit CCG;
the first expression cassette comprises a repeat sequence unit GAA, and the second expression cassette comprises the repeat sequence unit GAA;
the first expression cassette comprises a repeat sequence unit GGCCTG, and the second expression cassette comprises the repeat sequence unit GGCCTG;
the first expression cassette comprises a repeat sequence unit ATTTC, and the second expression cassette comprises the repeat sequence unit ATTTC;
the first expression cassette comprises a repeat sequence unit AAGGG, and the second expression cassette comprises the repeat sequence unit AAGGG;
the first expression cassette comprises a repeat sequence unit GCA, and the second expression cassette comprises the repeat sequence unit GCA;
the first expression cassette comprises a repeat sequence unit ATTCT/ATTGT, and the second expression cassette comprises the repeat sequence unit ATTCT/ATTGT;
the first expression cassette comprises a repeat sequence unit CCCCGCCCCGCG, and the second expression cassette comprises the repeat sequence unit CCCCGCCCCGCG.

In another preferred embodiment, the first promoter and second promoter each independently comprise a eukaryotic promoter or a prokaryotic promoter.

In another preferred embodiment, the first promoter and second promoter each independently comprise a constitutive promoter or an inducible promoter.

In another preferred embodiment, the first promoter and second promoter are each independently selected from the group consisting of: CMV promoter, CAG promoter, PGK promoter, EF-1α promoter, SV40 promoter, chicken beta-actin promoter, HSV-TK promoter, Ubc promoter, TRE promoter, UAS promoter, Ac5 promoter, Polyhedrin promoter, CaMKIIa promoter, IL-2 promoter, RSV promoter, GAL1 promoter, TEF1 promoter, TDH3 promoter, ADH1 promoter, T7 promoter, T3 promoter, Sp6 promoter, araBAD promoter, trp promoter, lac promoter, pL promoter, and combinations thereof.

In another preferred embodiment, the first detectable label and second detectable label are each independently selected from the group consisting of: fluorescent moiety, luciferase, luminescent label, quantum dot, β-galactosidase LacZ, and combinations thereof.

In another preferred embodiment, the fluorescent moiety is selected from the group consisting of: mCherry, GFP and derivatives thereof EGFP, dTomato, BFP, YFP, and combinations of other fluorescent proteins.

In another preferred embodiment, the luciferase is selected from the group consisting of: Firefly luciferase (FLuc), Renilla Luciferase (RLuc), Gaussia Luciferase (GLuc), Nano-Glo Luciferase (NLuc), Cypridina Luciferase (CLuc), and combinations of other luciferases.

In another preferred embodiment, the first detectable label and the second detectable label are different.

In another preferred embodiment, the first nucleic acid construct and the second nucleic acid construct are linked by a linking element.

In another preferred embodiment, the first expression cassette and the second expression cassette are located on the expression product of the same nucleic acid construct.

In another preferred embodiment, the first nucleic acid construct has a structure of Formula I or Formula I':

Z1-P1-Z2-pA1 (I)

pAl-Z2-P1-Z1 (I')

wherein,
Z1 is a first promoter;
P1 is a coding sequence of a first detectable label;
Z2 is a repeat sequence unit of 3-6 nucleotides, and the number of repetitions of the repeat sequence is 41-2000, preferably 45-1500, more preferably 50-1000;
pA1 is one or more nucleic acid fragments comprising a poly(A) signal element or other nucleic acid fragments for transcription termination, and is preferably selected from the group consisting of: CaMV poly(A) signal element, EF-1α poly(A) signal element, BGH (bovine growth hormone)-poly(A) signal element, hGH poly(A) signal element, SV40-poly(A) signal element, human β-globin poly(A) signal element, and combinations thereof;
each "-" is absent or a nucleotide linker sequence.

In another preferred embodiment, the second nucleic acid construct has a structure of Formula II or Formula II':

Z3-P2-Z4-pA2 (II)

pA2-Z4-P2-Z3 (II')

wherein,
Z3 is a second promoter;
P2 is a coding sequence of a second detectable label;
Z4 is a repeat sequence unit of 3-6 nucleotides corresponding to Z2, and the number of repetitions of the repeat sequence is 2-40, preferably 5-40;
pA2 is one or more nucleic acid fragments comprising a poly(A) signal element or other nucleic acid fragments for transcription termination, and is preferably selected from the group consisting of: CaMV poly(A) signal element, EF-1α poly(A) signal element, BGH (bovine growth hormone)-poly(A) signal element, hGH poly(A) signal element, SV40-poly(A) signal element, human β-globin polyadenylation signal element, and combinations thereof;
each "-" is absent or a nucleotide linker sequence.

In another preferred embodiment, pA1 and pA2 may be the same or different.

In another preferred embodiment, the first promoter and the second promoter are the same or have close expression strengths.

In another preferred embodiment, "close expression strengths" means that the ratio (T1/T2) of the expression strength T1 of the first promoter to the expression strength T2 of the second promoter is 0.8-1.2.

In another preferred embodiment, the first promoter is selected from the group consisting of: CMV promoter, CAG promoter, PGK promoter, EF-1α promoter, SV40 promoter, chicken beta-actin promoter, HSV-TK promoter, Ubc promoter, TRE promoter, UAS promoter, Ac5 promoter, Polyhedrin promoter, CaMKIIa promoter, IL-2 promoter, RSV promoter, GAL1 promoter, TEF1 promoter, TDH3 promoter, ADH1 promoter, T7 promoter, T3 promoter, Sp6 promoter, araBAD promoter, trp promoter, lac promoter, pL promoter, and combinations thereof.

In another preferred embodiment, the second promoter is selected from the group consisting of: CMV promoter, CAG promoter, PGK promoter, EF-1α promoter, SV40 promoter, chicken beta-actin promoter, HSV-TK promoter, Ubc promoter, TRE promoter, UAS promoter, Ac5 promoter, Polyhedrin promoter, CaMKIIa promoter, IL-2 promoter, RSV promoter, GAL1 promoter, TEF1 promoter, TDH3 promoter, ADH1 promoter, T7 promoter, T3 promoter, Sp6 promoter, araBAD promoter, trp promoter, lac promoter, pL promoter, and combinations thereof.

In another preferred embodiment, the first detectable label and the second detectable label are different.

In another preferred embodiment, the first detectable label and the second detectable label may be excited by lasers with different excitation wavelengths to emit fluorescence of different colors.

In another preferred embodiment, the first detectable label and the second detectable label may emit light of different colors by adding different substrates.

In another preferred embodiment, the repeat sequence unit of Z2 is CTG and the repeat sequence unit of Z4 is CTG; or
the repeat sequence unit of Z2 is CCTG and the repeat sequence unit of Z4 is CCTG; or
the repeat sequence unit of Z2 is CAG and the repeat sequence unit of Z4 is CAG; or
the repeat sequence unit of Z2 is TGGAA and the repeat sequence unit of Z4 is TGGAA; or
the repeat sequence unit of Z2 is GGGGCC and the repeat sequence unit of Z4 is GGGGCC; or
the repeat sequence unit of Z2 is CGG and the repeat sequence unit of Z4 is CGG;
the repeat sequence unit of Z2 is GCG and the repeat sequence unit of Z4 is GCG;
the repeat sequence unit of Z2 is CCG and the repeat sequence unit of Z4 is CCG;
the repeat sequence unit of Z2 is GAA and the repeat sequence unit of Z4 is GAA;
the repeat sequence unit of Z2 is GGCCTG and the repeat sequence unit of Z4 is GGCCTG;
the repeat sequence unit of Z2 is ATTTC and the repeat sequence unit of Z4 is ATTTC;
the repeat sequence unit of Z2 is AAGGG and the repeat sequence unit of Z4 is AAGGG;
the repeat sequence unit of Z2 is GCA and the repeat sequence unit of Z4 is GCA;
the repeat sequence unit of Z2 is ATTCT/ATTGT and the repeat sequence unit of Z4 is ATTCT/ATTGT;
the repeat sequence unit of Z2 is CCCCGCCCCGCG and the repeat sequence unit of Z4 is CCCCGCCCCGCG.

In another preferred embodiment, the lengths of the first nucleic acid construct and the second nucleic acid construct each independently comprise 10 to 1000 repeat units, preferably 15 to 500 repeat units, and more preferably 20 to 500 repeat units.

In another preferred embodiment, the set of constructs has a structure of Formula III (or III' or III" or III"' or III‴′ or III‴ʺ or III‴‴ or III‴‴′:

Z1-P1-Z2-pA1-Z3-P2-Z4-pA2 (III);

Z3-P2-Z4-pA2-Z1-P1-Z2-pA1 (III');

Z1-P1-Z2-pA1-pA2-Z4-P2-73 (III");

Z3-P2-Z4-pA2-pA1-Z2-P1-Z1 (III"');

pA1-Z2-P1-Z1-Z3-P2-Z4-pA2 (III‴′);

pA2-Z4-P2-Z3-Z1-P1-Z2-pA1 (III‴ʺ);

pA1-Z2-P1-Z1-pA2-Z4-P2-Z3 (III‴‴);

pA2-Z4-P2-Z3-pAl-Z2-P1-Z1 (III‴‴′);

wherein each element is as defined above.

In another preferred embodiment, the set of nucleic acid constructs comprises 10 to 1000 repeat units, preferably 15 to 500 repeat units, and more preferably 20 to 500 repeat units.

In the second aspect of the present invention, it provides a recombinant vector comprising the set of nucleic acid constructs according to the first aspect of the present invention.

In another preferred embodiment, the vector comprises a DNA vector, an RNA vector, a viral vector, a plasmid, a transposon, other gene transfer systems, or a combination thereof; preferably, the vector is a plasmid or a viral vector.

In another preferred embodiment, the vector is circular or linear.

In the third aspect of the present invention, it provides a genetically engineered cell comprising the set of nucleic acid constructs according to the first aspect of the present invention, or the recombinant vector according to the second aspect of the present invention.

In another preferred embodiment, the genetically engineered cell comprises a eukaryotic cell.

In another preferred embodiment, the genetically engineered cell comprises a mammalian cell.

In another preferred embodiment, the genetically engineered cell comprises human embryonic kidney HEK293 cells, human skeletal muscle cells, human HeLa cells, human corneal endothelial cells, mouse NIH 3T3 cell line, monkey COS-7 cell line, and other commonly used mammalian cell lines.

In the fourth aspect of the present invention, it provides a kit comprising: a container and the set of nucleic acid constructs according to the first aspect of the present invention located in the container.

In another preferred embodiment, the kit is used for screening drugs for treating long RNA repeat sequence-associated diseases.

In the fifth aspect of the present invention, it provides a method for screening a drug for treating long RNA repeat sequence-associated diseases, comprising the steps of:
(a) co-introducing the set of nucleic acid constructs according to the first aspect of the present invention and a drug to be screened into a cell;
(b) determining whether the drug to be screened is a drug for treating long RNA repeat sequence-associated diseases according to the intensity changes of the first detectable label and the second detectable label in the set of nucleic acid constructs.

In another preferred embodiment, the cell comprises a mammalian cell.

In another preferred embodiment, the cell comprises human skeletal muscle cells, human neural stem cells, human fibroblast stem cells, human myoblasts, human cardiac cells, human renal epithelial cells, human corneal endothelial cells, human tumor cells, human lung cells, human hepatocytes, human splenic cells, human colon cells, human embryonic stem cells, human bone marrow cells, human thyroid cells, human pancreatic cells, human lymphocytes, human T cells, human colon gland cells, human retinal cells, human gastric cells, or other human-derived cells.

In another preferred embodiment, the cell comprises HEK293T, Hela, u2os, N2a, U87, NIH3T3, B4G12, COS-7, SY5Y, A549, or other human-derived stable cell lines.

In another preferred embodiment, the cell is an *in vitro* cultured cell.

In another preferred embodiment, when the expression level or intensity of the first detectable label is significantly reduced, and the expression level or intensity of the second detectable label is slightly changed or substantially unchanged, it indicates that the drug to be screened can be used as a candidate drug for treating long RNA repeat sequence-associated diseases.

In another preferred embodiment, when the reduction extent of the expression level or intensity of the first detectable label is small, it indicates that the drug to be screened is not suitable for treating long RNA repeat sequence-associated diseases.

In another preferred embodiment, the significant reduction in the expression level or intensity of the first detectable label means that the reduction extent of the expression level or intensity of the first detectable label is ≥25%, preferably ≥50%, and more preferably ≥75%.

In another preferred embodiment, the small reduction extent of the expression level or intensity of the first detectable label means that the reduction extent of the expression level or intensity of the first detectable label is ≤25%, preferably ≤20%, and more preferably ≤15%.

In another preferred embodiment, the slight change or substantial unchangedness of the expression level or intensity of the second detectable label means that the reduction extent of the expression level or intensity of the second detectable label is ≤25%, preferably ≤15%, more preferably ≤5%, and even more preferably 0.

In another preferred embodiment, the introduction method comprises one or more methods selected from the group consisting of: calcium phosphate-mediated transfection, nucleofection, electroporation, cationic polymer transfection, viral transduction, virosome transfection, virion transfection, liposome transfection, cationic liposome transfection, immunoliposome transfection, non-liposome transfection, dendrimer transfection, heat shock transfection, magnetofection, lipofection, gene gun delivery, impalefection, sonoporation, and optical transfection.

In another preferred embodiment, the drug to be screened comprises a drug capable of reducing the expression level or intensity of the first detectable label without reducing the expression level or intensity of the second detectable label.

In another preferred embodiment, the drug to be screened comprises a CRISPR agent.

In another preferred embodiment, the CRISPR agent comprises a gene-editing protein and an optional guide RNA.

In another preferred embodiment, the gene-editing protein can recognize the long RNA repeat sequence and cleave the recognized long RNA repeat sequence.

In another preferred embodiment, the guide RNA directs the gene-editing protein to specifically bind to a DNA portion capable of expressing the long RNA repeat sequence.

In another preferred embodiment, the gene-editing protein is selected from the group consisting of: Cas13a, Cas13b, Cas13d, Cas13x, Cas13y, RNA-targeting Cas9 (RCas9, dead Cas9 from *Streptococcus pyogenes*), dSaCas9 (dead Cas9 from *Staphylococcus aureus*), and a combination thereof.

In another preferred embodiment, the drug to be screened comprises an artificial RNA degrading enzyme.

In another preferred embodiment, the RNA degrading enzyme has a structure of Formula IV or IV' from the N-terminus to the C-terminus of the protein:

D1-A-L1-B-D2 (IV);

D1-B-L1-A-D2 (IV')

wherein each "-" is independently a linker peptide or a peptide bond;
A is a PUF protein fragment;
B is the full length of an RNase or an RNA helicase, or an active fragment thereof;
L1 is absent or a linker peptide;
D1 is absent or a localization peptide;
D2 is absent or a localization peptide.

In another preferred embodiment, the PUF protein fragment is a fragment of the domain in a PUF protein that can recognize and bind to RNA.

In another preferred embodiment, the PUF protein comprises a PUF protein and its homologous proteins.

In another preferred embodiment, the PUF protein is derived from a mammal, preferably a primate, and more preferably a human.

In another preferred embodiment, the PUF protein fragment comprises n recognition units, each of which is used to recognize one RNA base, wherein n is a positive integer of 5-30.

In another preferred embodiment, n is 6-24, more preferably 8-20, e.g., 6, 7, 8, 9, 10, 11, 12, 13, 14, 15, 16, 17, 18, 19, 20, 21, 22, 23, 24.

In another preferred embodiment, each recognition unit is an α-helical repeat sequence, and three amino acids at specific positions on the repeat sequence are responsible for binding to the RNA base.

In another preferred embodiment, the n recognition units are connected in tandem to form an RNA base recognition region.

In another preferred embodiment, the PUF protein fragment further comprises optional protective regions respectively located on both sides of the RNA base recognition region, for protecting the RNA base recognition region.

In another preferred embodiment, the PUF protein fragment comprises the domain of the PUF protein upstream of the RNA base recognition region (excluding the protective regions), but may or may not comprise the domain of the PUF protein downstream of the RNA base recognition region (excluding the protective regions).

In another preferred embodiment, the protective regions are non-functional repeat sequences at both ends of the RNA base recognition region.

In another preferred embodiment, the localization peptide sequence comprises a nuclear localization signal sequence, a mitochondrial localization signal sequence, or a nuclear export signal sequence.

In another preferred embodiment, the linker peptide and/or the localization peptide are each independently located between the PUF protein fragment and the RNase or RNA helicase.

In another preferred embodiment, the RNA is single-stranded.

In another preferred embodiment, the PUF protein fragment is selected from the group consisting of:
(a) a polypeptide having the amino acid sequence as set forth in SEQ ID NO: 1;
(b) a polypeptide having ≥80%, 81%, 82%, 83%, 84%, 85%, 86%, 87%, 88%, 89%, 90%, 91%, 92%, 93%, 94%, 95%, 96%, 97%, 98%, 99% or 99.5% homology (or identity) with the amino acid sequence as set forth in SEQ ID NO: 1, and the polypeptide has the biological function of the polypeptide as set forth in SEQ ID NO: 1;
(c) a derivative polypeptide formed by substitution, deletion or addition of one or more (preferably 1-20, more preferably 1-10, even more preferably 1-5) amino acid residues in the amino acid sequence as set forth in SEQ ID NO: 1, and retaining the biological function of the polypeptide as set forth in SEQ ID NO: 1.

In another preferred embodiment, the PUF protein fragment has the amino acid sequence as set forth in SEQ ID NO: 1.

GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLG (SEQ ID NO: 1)

In another preferred embodiment, the full length of the RNase or RNA helicase, or an active fragment thereof, is selected from the group consisting of: PIN (from SMG6, UNIPROT: Q86US8), ANG (UNIPROT: P03950), RNASEL (UNIPROT: Q05823), RRP44 (UNIPROT: Q9Y2L1), RNASE1 (UNIPROT: P07998), RNASET2 (UNIPROT: O00584), SUPV3L1 (UNIPROT: Q8IYB8), PNPT1 (UNIPROT: Q8TCS8), REXO2 (UNIPROT: Q9Y3B8), MRPL44 (UNIPROT: Q9H9J2), LACTB2 (UNIPROT: Q53H82), TSEN15 (UNIPROT: Q8WW01), ZC3H12A (UNIPROT: Q5D1E8), an active region thereof, and sequence combination thereof.

In another preferred embodiment, the full length of the RNase or RNA helicase, or an active fragment thereof, is selected from the group consisting of: ANG (UNIPROT: P03950), RNASE1 (UNIPROT: P07998), RNASET2 (UNIPROT: O00584), SUPV3L1 (UNIPROT: Q8IYB8), PNPT1 (UNIPROT: Q8TCS8), REXO2 (UNIPROT: Q9Y3B8), and a combination thereof.

In another preferred embodiment, the RNase or RNA helicase comprises an RNase or RNA helicase having at least 80%, preferably at least 85% or 90%, more preferably at least 95%, most preferably at least 98% or 99% homology with the above-mentioned RNase or RNA helicase.

In another preferred embodiment, the nucleotide sequence encoding the RNA degrading enzyme is a codon-optimized nucleotide sequence.

In another preferred embodiment, the nucleotide sequence encoding the RNA degrading enzyme is a nucleotide sequence codon-optimized using the GENEWIZ algorithm, Genescript algorithm, or Thermo algorithm.

In another preferred embodiment, the artificial RNA degrading enzyme is selected from the group consisting of:
(a) a polypeptide having the amino acid sequence as set forth in any one of SEQ ID NOs: 95-111 and 116-119;
(b) a polypeptide having ≥80%, 81%, 82%, 83%, 84%, 85%, 86%, 87%, 88%, 89%, 90%, 91%, 92%, 93%, 94%, 95%, 96%, 97%, 98%, 99% or 99.5% homology (or identity) with the amino acid sequence as set forth in any one of SEQ ID NOs: 95-111 and 116-119, and the polypeptide has the biological function of the polypeptide as set forth in any one of SEQ ID NOs: 99-115 and 120-123;
(c) a derivative polypeptide formed by substitution, deletion or addition of one or more (preferably 1-20, more preferably 1-10, even more preferably 1-5) amino acid residues in the amino acid sequence as set forth any one of SEQ ID NOs: 95-111 and 116-119, and retaining the biological function of the polypeptide as set forth in any one of SEQ ID NOs: 95-111 and 116-119.

In another preferred embodiment, the linker peptide is absent, a flexible peptide, or a rigid peptide.

In another preferred embodiment, the linker peptide is selected from the group consisting of: XTEN peptide, GGGGS, and other short peptides.

In another preferred embodiment, the length of the linker peptide is 0-40 aa, preferably 2-20 aa.

In the sixth aspect of the present invention, it provides a system for screening a drug for treating long RNA repeat sequence-associated diseases, wherein the system comprises:
(a) the set of nucleic acid constructs according to the first aspect of the present invention;
(b) a drug to be screened;
wherein, when the expression level or intensity of the first detectable label in the set of nucleic acid constructs is significantly reduced, and the expression level or intensity of the second detectable label is slightly changed or substantially unchanged, it indicates that the drug to be screened is a candidate or potential drug usable for treating long RNA repeat sequence-associated diseases.

In another preferred embodiment, the first detectable label is driven to express by a first promoter (e.g., CMV promoter, CAG promoter, PGK promoter, EF-1α promoter, SV40 promoter, chicken beta-actin promoter, HSV-TK promoter, Ubc promoter, TRE promoter, UAS promoter, Ac5 promoter, Polyhedrin promoter, CaMKIIa promoter, IL-2 promoter, RSV promoter, or other eukaryotic cell promoters).

In another preferred embodiment, the second detectable label is driven to express by a second promoter (e.g., CMV promoter, CAG promoter, PGK promoter, EF-1α promoter, SV40 promoter, chicken beta-actin promoter, HSV-TK promoter, Ubc promoter, TRE promoter, UAS promoter, Ac5 promoter, Polyhedrin promoter, CaMKIIa promoter, IL-2 promoter, RSV promoter, or other eukaryotic cell promoters).

In another preferred embodiment, the first promoter and the second promoter are the same or have close expression strengths.

In another preferred embodiment, "close expression strengths" means that the ratio (T1/T2) of the expression strength T1 of the first promoter to the expression strength T2 of the second promoter is 0.8-1.2.

In another preferred embodiment, when the expression level or intensity of the first detectable label is significantly reduced, the expression level or intensity of the second detectable label is slightly changed or substantially unchanged.

In another preferred embodiment, the first detectable label and the second detectable label are respectively expressed via the same promoter.

In the seventh aspect of the present invention, it provides a screening composition for screening a drug for treating long RNA repeat sequence-associated diseases, wherein the screening composition comprises:
(a) a drug to be screened;
(b) the set of nucleic acid constructs according to the first aspect of the present invention;
wherein, when the expression level or intensity of the first detectable label in the set of nucleic acid constructs is significantly reduced, and the expression level or intensity of the second detectable label is slightly changed or substantially unchanged, it indicates that the drug to be screened is a candidate or potential drug usable for treating long RNA repeat sequence-associated diseases.

In the eighth aspect of the present invention, it provides a use of the system according to the sixth aspect of the present invention or the screening composition according to the seventh aspect of the present invention, for screening a drug for treating long RNA repeat sequence-associated diseases.

In another preferred embodiment, the drug for treating long RNA repeat sequence-associated diseases comprises a CRISPR agent.

In another preferred embodiment, the CRISPR agent comprises a gene-editing protein and an optional guide RNA.

In another preferred embodiment, the gene-editing protein can recognize the long RNA repeat sequence and cleave the recognized long RNA repeat sequence.

In another preferred embodiment, the guide RNA directs the gene-editing protein to specifically bind to a DNA portion capable of expressing the long RNA repeat sequence.

In another preferred embodiment, the gene-editing protein is selected from the group consisting of: Cas13a, Cas13b, Cas13d, Cas13x, Cas13y, RNA-targeting Cas9 (RCas9, dead Cas9 from *Streptococcus pyogenes*), dSaCas9 (dead Cas9 from *Staphylococcus aureus*), and a combination thereof.

In another preferred embodiment, the drug for treating long RNA repeat sequence-associated diseases comprises an artificial RNA degrading enzyme.

In another preferred embodiment, the RNA degrading enzyme has a structure of Formula IV or IV' from the N-terminus to the C-terminus:

D1-A-L1-B-D2 (IV);

D1-B-L1-A-D2 (IV')

wherein each "-" is independently a linker peptide or a peptide bond;
A is a PUF protein fragment;
B is the full length of an RNase or an RNA helicase, or an active fragment thereof;
L1 is absent or a linker peptide;
D1 is absent or a localization peptide;
D2 is absent or a localization peptide.

In another preferred embodiment, the nucleotide sequence encoding the RNA degrading enzyme is a codon-optimized nucleotide sequence.

In another preferred embodiment, the nucleotide sequence encoding the RNA degrading enzyme is a nucleotide sequence codon-optimized using the GENEWIZ algorithm, Genescript algorithm, or Thermo algorithm.

In another preferred embodiment, the artificial RNA degrading enzyme is an RNA degrading enzyme obtained by encoding with a codon-optimized nucleotide sequence (e.g., codon-optimized using the GENEWIZ algorithm, Genescript algorithm, or Thermo algorithm).

In the ninth aspect of the present invention, it provides a composition comprising:
(a) a drug for treating long RNA repeat sequence-associated diseases obtained by the method according to the fifth aspect of the present invention or the system according to the sixth aspect of the present invention, wherein the drug for treating long RNA repeat sequence-associated diseases comprises an artificial RNA degrading enzyme.

In another preferred embodiment, the RNA degrading enzyme has a structure of Formula IV or IV' from the N-terminus to the C-terminus:

D1-A-L1-B-D2 (IV);

D1-B-L1-A-D2 (IV')

wherein each "-" is independently a linker peptide or a peptide bond;
A is a PUF protein fragment;
B is the full length of an RNase or an RNA helicase, or an active fragment thereof;
L1 is absent or a linker peptide;
D1 is absent or a localization peptide;
D2 is absent or a localization peptide.

In the tenth aspect of the present invention, it provides a method for preparing an animal model for screening a drug for treating long RNA repeat sequence-associated diseases, wherein the method comprises the steps of:
(a) providing a cell of a non-human mammal, and introducing the set of nucleic acid constructs according to the first aspect of the present invention into the cell, thereby obtaining a cell that simultaneously expresses a long RNA repeat sequence and a short RNA repeat sequence;
(b) using the cell that simultaneously expresses a long RNA repeat sequence and a short RNA repeat sequence obtained in step (a), thereby preparing an animal model for screening a drug for treating long RNA repeat sequence-associated diseases.

In another preferred embodiment, the cell comprises a somatic cell, a germ cell and/or an embryonic stem cell.

In another preferred embodiment, the embryonic stem cell further comprises an embryo of less than 14 days and/or a fertilized egg.

In another preferred embodiment, the cell is selected from a non-human mammal, preferably the non-human mammal comprises a rodent or a primate, and more preferably the non-human mammal comprises a mouse, rat, rabbit and/or monkey, chimpanzee.

In another preferred embodiment, the cell is a fertilized egg of a non-human mammal.

In the eleventh aspect of the present invention, it provides a use of a non-human mammalian model prepared by the method according to the tenth aspect of the present invention, wherein the model is used for screening or identifying a substance (therapeutic agent) for treating long RNA repeat sequence-associated diseases.

In the twelfth aspect of the present invention, it provides a method for screening or identifying a potential therapeutic agent of a drug for treating long RNA repeat sequence-associated diseases, comprising the steps of:
(a) in a test group, administering a test drug to a non-human mammalian model prepared by the method according to the ninth aspect of the present invention in the presence of the test drug, and analyzing the expression level or intensity of the first detectable label and the second detectable label of the animal model in the test group, respectively; and in a control group without administering the test drug but under otherwise identical conditions, analyzing the expression level or intensity of the first detectable label and the second detectable label of the animal model in the control group;
(b) comparing the expression level or intensity of the first detectable label and the second detectable label of the animal models between the test group and the control group, wherein, compared with the control group, if the expression level or intensity of the first detectable label is significantly reduced and the expression level or intensity of the second detectable label is substantially unchanged in the animal model administered with the test drug, it indicates that this test compound can be used as a potential therapeutic agent for treating long RNA repeat sequence-associated diseases.

In another preferred embodiment, the long RNA repeat sequence-associated diseases comprise the disease types shown in Table 1 and Table 2.

In another preferred embodiment, the long RNA repeat sequence-associated diseases comprise: Frontotemporal dementia (FTD), Huntington disease (HD), Myotonic dystrophy Type 1/2 (DM1/DM2), Amyotrophic lateral sclerosis (ALS), Fuchs endothelial corneal dystrophy (FECD), Autosomal-dominant cerebellar ataxias (ADCAs), and Fragile X syndrome (FXS).

In another preferred embodiment, the method is non-diagnostic and non-therapeutic.

In another preferred embodiment, the method comprises step (c): administering the potential therapeutic agent screened or identified in step (b) to the non-human mammalian model prepared by the method according to the tenth aspect of the present invention, so as to determine its effect on the expression level or intensity of the first detectable label and the second detectable label in the animal model.

In the thirteenth aspect of the present invention, it provides a non-human mammalian model, which is prepared by the method according to the tenth aspect of the present invention.

In the fourteenth aspect of the present invention, it provides a use of the genetically engineered cell according to the third aspect of the present invention, for preparing a biological agent for constructing a non-human mammalian animal model for screening a drug for treating long RNA repeat sequence-associated diseases.

In another preferred embodiment, the biological agent is a liquid formulation.

It should be understood that within the scope of the present invention, the above-mentioned technical features of the present invention and the technical features specifically described in the following (such as the examples) can be combined with each other to form a new or preferred technical solution, which is not redundantly repeated one by one herein due to space limitation.

### Description of the drawings

Figure 1. Schematic diagrams of dual-reporter gene systems with different RNA repeat sequences. (A) CUG repeat sequence; (B) CAG repeat sequence; (C) CCUG repeat sequence; (D) TGGAA repeat sequence.
Figure 2. Induced expression results of the dual-reporter gene system with CUG repeat sequence in Flp-In T-REX 293 cells. (A) Expression of reporter gene fluorescent proteins in cells without the addition of an inducer; (B) Expression of reporter gene fluorescent proteins in cells with the addition of 1 µg/mL of the inducer doxycycline; (C) Expression imaging results of the dual-reporter gene system with CUG repeat sequence in cells.
Figure 3. Flowchart of the comparative experiment for the effects of existing systems targeting RNA repeat sequences.
Figure 4. Flow cytometry detection results of existing systems targeting RNA repeat sequences.
Figure 5. RT-qPCR detection results of existing systems targeting RNA repeat sequences.
Figure 6. Screening of novel artificial RNA degrading enzymes using dual-reporter gene systems with different RNA repeat sequences. (A) Schematic diagram of the structure of artificial RNA degrading enzymes; (B) Screening and detection results (RT-qPCR) of novel artificial RNA degrading enzymes.
Figure 7. Flow cytometry detection results of the newly constructed artificial RNA degrading enzymes on the RNA repeat sequence dual-reporter system.
Figure 8. Abnormal RNA aggregates formed in the nucleus by fluorescent RNA-labeled RNA repeat sequences. (A) Schematic diagram of fluorescent RNA-labeled RNA repeat sequences; (B) Number of abnormal RNA aggregates formed in the nucleus by RNA repeat sequences of different lengths; (C) Imaging results of RNA repeat sequences of different lengths in cells; (D) Distribution of fluorescent RNA labeling CUG repeat sequences is consistent with that of fluorescent proteins targeting CUG repeat sequences.
Figure 9. Clearance of intranuclear aggregation of RNA repeat sequences by different systems. (A) Regulation of intranuclear aggregation of RNA repeat sequences by non-PUF systems; (B) Regulation of intranuclear aggregation of RNA repeat sequences by different PUF systems; (C) Statistical results of all systems capable of regulating intracellular RNA repeat sequence aggregation (n=50).
Figure 10. (A) Construction of MBNL1-overexpressing cell lines to detect the distribution of pathogenic RNA in the nucleus. (B) Expression strategy of PUF protein-based artificial RNA degrading enzyme expression vector and pathogenic repeat RNA fluorescent vector. (C) Clearance of intranuclear aggregation of RNA repeat sequences and alleviation of disease molecular phenotypes by artificial RNA degrading enzymes. (D) Statistical results of systems in which artificial RNA degrading enzymes can regulate intracellular RNA repeat sequence aggregation (n=50).

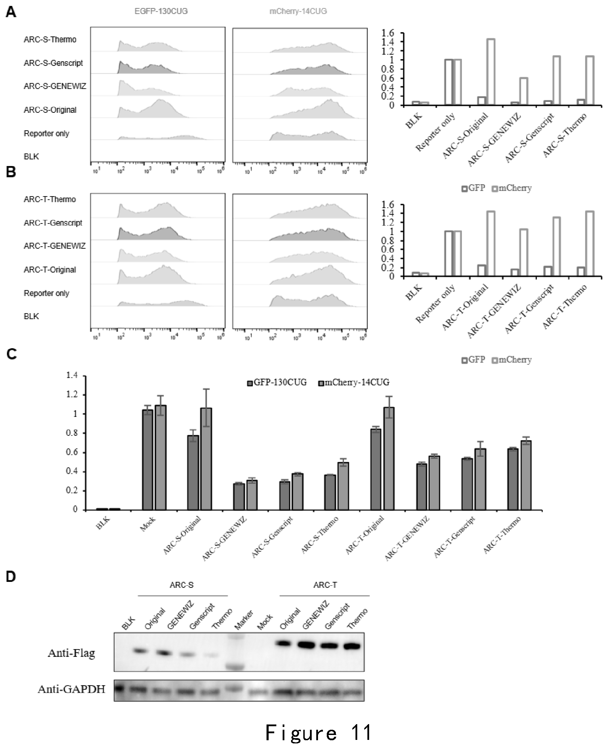

Figure 11. (A) Flow cytometry detection results of ARC-S artificial RNA degrading enzymes optimized for expression by different DNA sequence optimization methods. (B) Flow cytometry detection results of ARC-T artificial RNA degrading enzymes optimized for expression by different DNA sequence optimization methods. (C) RT-qPCR detection results of ARC-S and ARC-T artificial RNA degrading enzymes optimized for expression by different DNA sequence optimization methods. (D) Expression level detection results of ARC-S and ARC-T artificial RNA degrading enzymes optimized for expression by different DNA sequence optimization methods.
Figure 12. (A) Protein purification strategy for ARC-S and ARC-T artificial RNA degrading enzymes. (B) Purity identification of protein purification products of ARC-S and ARC-T artificial RNA degrading enzymes. (C) *In vitro* evaluation experiments of the enzymatic activities of ARC-S and ARC-T artificial RNA degrading enzymes.
Figure 13. Strategy for constructing mouse models of dual-reporter gene systems with CUG repeat sequences of different lengths.
Figure 14. Strategy for constructing mouse models of dual-reporter gene systems with CAG repeat sequences of different lengths.
Figure 15. Imaging identification results of the construction of dual-reporter gene mice with different CUG repeat sequence lengths.
Figure 16. (A) Comparison of the effects of existing systems using dual-reporter gene mouse models. Schematic diagram of the mouse experimental protocol, in which control AAV viral particles were injected into the left leg of mice and experimental AAV viral particles were injected into the right leg of mice, with the experiment lasting for 2 months. (B) RT-qPCR detection results of the reporter gene with long CUG sequence (mCherry-160CUG). (C) RT-qPCR detection results of the reporter gene with short CUG sequence (EGFP-14CUG).
Figure 17. (A) Influence of different targeting systems on changes in normal gene expression when targeting pathogenic RNA repeat sequences in mouse muscle tissue. (B) Schematic diagram of the experimental protocol for isolation of T cells from mouse peripheral blood. (C) RT-qPCR detection results of changes in expression levels of immune response marker genes in CD4 and CD8 cells.
Figure 18. Crystal structure diagram of the binding between the PUF domain and target RNA (PDB ID: 2YJY) (left). Binding code between PUF and RNA (right).

### Detailed description

Through extensive and in-depth research, the inventors of the present invention have for the first time developed a set of nucleic acid constructs for screening drugs for treating long RNA repeat sequence-associated diseases, as well as vectors and screening systems based on the set of nucleic acid constructs. Using the set of nucleic acid constructs, as well as the vectors and the screening systems, drugs for treating long RNA repeat sequence-associated diseases can be screened or identified in a high-throughput manner. Based on the screening method targeting RNA repeat sequences developed by the present invention, potential drugs for treating RNA repeat sequence-associated diseases can be screened quickly and accurately. The screening method of the present invention combines technologies in multiple fields such as bioinformatics, and can comprehensively analyze and evaluate the potential therapeutic ability of various drugs. On this basis, the present invention has been completed.

### Long RNA repeat sequence

As used herein, the terms "long RNA repeat sequence" and "long repeat RNA sequence" may be used interchangeably and both refer to a long sequence carrying an abnormal number of RNA repeat units, which can trigger abnormal intracellular RNA metabolism, thereby leading to the occurrence of diseases. It is caused by the abnormal expansion of microsatellite sequences in the genome.

The correspondence between disease types caused by long RNA repeat sequences and long RNA repeat sequences is shown in Table 1 and Table 2 (Reference: Depienne, C., & Mandel, J.-L. (2021). 30 years of repeat expansion disorders: What have we learned and what are the remaining challenges? The American Journal of Human Genetics, 108(5), 764-785.)

**Table 1 List of coding repeat expansion disorder sequences (sorted by year of discovery)**

| **Year of Identifi cation** | **Diso rder** | **Inherit ance** | **Chromo somal Locatio n** | **Gene** | **Repea t Unit** | **Nor mal Rep eat Ran ge** | **Pathol ogical Repeat Range** | **Patholo gical Mechan ism(s)** | **Identifi cation Method** | **Referenc e / Discover y Team** |
|---|---|---|---|---|---|---|---|---|---|---|
| 1993 | HD | AD | 4p 16.3 | *HTT* | CAG | 6-35 | ≥36-250 | polyQ, RNA | L, ExScr | The Huntingt on's Disease Collabor ative Research Group |
| 1993 | SCA 1 | AD | 6p22 | *ATXN* 1 | CAG | 6-38 | ≥39-88 | polyQ, RNA | L, ExScr | Orr et al. |
| 1994 | DRP LA | AD | 12p13.3 1 | *ATN1* | CAG | 3-35 | ≥48-93 | polyQ, RNA | L, ExScr | Koide et al.,Nag afuchi et al. |
| 1994 | SCA 3 | AD | 14q32 | *ATXN* 3 | CAG | 12-44 | ≥55-87 | polyQ, RNA (foci) | L, cl | Kawaguc hi et al. |
| 1996 | SCA 2 | AD | 12q24 | *ATXN* 2 | CAG | 13-31 | ≥32-500 | polyQ, RNA | L, cl | Pulst et al. |
| 1996 | SCA 7 | AD | 3p21 | *ATXN* 7 | CAG | 4-33 | >37-460 | polyQ, RNA | L, cl | Lindblad et al. |
| 1996 | SPD 1 | AD | 2q31.1 | *HOX D13* | GCG | 15 | 24 | polyA (DN) | L, CGA | Akarsu et al. |
| 1997 | SCA 6 | AD | 19p13 | *CACN A1A* | CAG | 4-18 | ≥20-33 | polyQ, RNA | L, ExScr | Zhuchen ko et al. |
| 1997 | BCC D | AD | 6p21 | *RUNX* 2 | GCN | 17 | 27 | polyA (DN/Lo F) | L, CGA | Mundlos et al. |
| 1998 | OPM D | AD | 14q11.2 | *PABP N1* | GCG | 6-10 | ≥12-17 | polyA (DN/Lo F) | L, ExScr | Brais et al. |
| 1999 | SCA 17 | AD | 6q27 | *TBP* | CAG (or CAG/ CAA) | 25-40 | ≥43-66 | polyQ, RNA | L, CGA | Koide et al. |
| 2000, 2002 | HFG S | AD | 7p15.2 | *HOX A13* | tract 1: GCN | 14 | 22 | polyA (DN/Lo F) | L, CGA | Goodma n et al., Uts ch et al. |
| | | | | | tract 2: GCN | 12 | 18 | | | |
| | | | | | tract 3: GCN | 18 | 24-30 | | | |
| 2001 | HDL 2 | AD | 16q24.2 | *JPH3* | CAG | 6-28 | ≥41-58 | polyQ, RNA (foci) | L, RED | Margolis et al. |
| 2001 | BPE S | AD | 3q23 | *FOXL* 2 | GCN | 14 | 19-24 | polyA (LoF) | L, CGA | De Baere et al. |
| 2001 | HPE 5 | AD | 13q32 | *ZIC2* | GCN | 15 | 25 | polyA (LoF) | L, CGA | Brown et al. |
| 2002 | EIEE 1 | XLR | Xp21.3 | *ARX* | tract 1: GCN | 16 | 23 | polyA (DN) | L, CGA | Stremme et al. |
| | | | | | tract 2: GCN | 12 | 20 | | | |
| 2002 | MRG H | XLR | Xq26.3 | *SOX3* | GCN | 15 | 26 | polyA (LoF) | L, CGA | Laumonn ier et al. |
| 2003 | CCH S | AD | 4p13 | *PHO X2B* | GCN | 20 | 25-29 | polyA (DN) | L, CGA | Amiel et al. |

**Disorder:** BCCD, brachydactyly and cleidocranial dysplasia; BPES, blepharophimosis, ptosis and epicanthus inversus; CCHS, congenital central hypoventilation syndrome; DRPLA, dentatorubral-pallidoluysian atrophy; EIEE1, early infantile epileptic encephalopathy type 1; HD, Huntington disease; HDL2, Huntington disease-like 2; HFGS, hand-foot-genital syndrome; HPE5, holoprosencephaly type 5; MRGH, mental retardation with isolated growth hormone deficiency; OPMD, oculopharyngeal muscular dystrophy; SBMA, spinal and bulbar muscular atrophy; SPD1, synpolydactyly type 1; SCA, spinocerebellar ataxia.

**Inheritance:** AD, autosomal dominant; XLR, X-linked recessive.

**Pathological Mechanism(s):** polyA (DN), polyAlanine tract associated with dominant-negative effect; polyA (LoF), polyAlanine tract leading to a loss of function of the protein; polyQ, polyGlutamine protein toxicity; polyG, polyGlycine peptide toxicity; RNA, RNA toxicity; RNA (foci), RNA foci toxicity.

**Identification Method:** cl, cloning; CGA, candidate gene analysis; ExScr, expansion screening; L, linkage; RED, repeat expansion detection.

**Table 2 List of non-coding repeat expansion disorder sequences (sorted by year of discovery)**

| **Year of Identi ficati on** | **Disor der** | **Inher itance** | **Chro moso mal Locat ion** | **Gene** | **Locat ion** | **Repe at Unit** | **Differ ent Patho genic Motif s** | **Norm al Repe at Rang e** | **Patho logica l Repe at Rang e** | **Patho logica l Mech anism (s)** | **Identi ficati on Meth od** | **Refer ence / Disco very Team** |
|---|---|---|---|---|---|---|---|---|---|---|---|---|
| 1991 | FXS | XLD | Xq27. 3 | *FMR1* | 5' UTR | CGG | - | 5-50 | >200 | Me/G S | K, FS, L, Me, cl | Oberl é et al., Verke rk et al. |
| 1992 | DM1 | AD | 19q13 .32 | *DMP K* | 3' UTR | CTG | - | 5-37 | >50-10,00 0 | RNA (foci, PS), RAN (poly Q) | L, cl | Maha devan et al. |
| 1993 | FRA XE | XLR | Xq28 | *AFF2* | 5' UTR | CCG | - | 4-39 | ≥200-900 | Me/G S | K, L | Knigh t et al. |
| 1996 | FRD A | AR | 9q21. 11 | *FXN* | intron | GAA | - | 5-34 | ≥66-1300 | EGS | L, ExScr | Camp uzano et al. |
| 1997 | EPM1 | AR | 21q22 .3 | *CSTB* | 5' UTR | CCC CGC CCC GCG | - | 2-3 | ≥30-75 | EGS | L, cl, exp | Laliot i et al. |
| 1998 | FXPO I | XL | Xq27. 3 | *FMR1* | 5' UTR | CGG | - | 5-50 | 55-200 | RNA (foci), RAN | Fam, ExScr | Conw ay et al. |
| 1999 | SCA8 | AD | 13q21 | *ATXN* 8/ *ATXN 8OS* | 3' UTR | CAG/ CTG | - | 15-50 | >74-250 | RNA (foci), RAN | L, cl | Koob et al. |
| 1999 | SCA1 2 | AD | 5q31 | *PPP2 R2B* | 5' UTR | CAG | - | 4-32 | ≥43-78 | RAN (poly G)? | L, RED | Holm es et al. |
| 2000 | SCA1 0 | AD | 22q13 | *ATXN* 10 | intron | ATTC T / ATT GT | - | 10-32 | >280-4,500 | RNA (foci) | L, ExScr | Matsu ura et al. |
| 2001 | DM2 | AD | 3q21. 3 | *CNBP* | intron | CCT G/ CAG G | - | 11-30 | >50-11,00 0 | RNA (foci) | L, ExScr | Liquo ri et al. |
| 2001 | FXTA S | XLR | Xq27. 3 | *FMR1* | 5' UTR | CGG | - | 5-50 | 55-200 | RNA (foci), RAN (poly G) | Fam | Hager man et al. |
| 2009 | SCA3 1 | AD | 16q22 | *BEAN 1*/ *TK2* | intron | TAA AA | TGG AA (+TA GAA) | variab le GAA) GAA) | ≥110-760 | RNA (foci, PS), RAN? | L | Sato et al. |
| 2011 | SCA3 6 | AD | 20p13 | *NOP5* 6 | intron | GGC CTG | - | 5-14 | ≥650-2,500 | RNA (foci) | L, ExScr | Koba yashi et al. |
| 2011 | FTD/ ALS | AD | 9p21. 2 | *C9OR F72* | 5' UTR / intron | GGG GCC | - | 3-25 | >30 | RNA (foci), RAN (poly G) | L, WGS | Rento n et al., DeJes us-Herna ndez et al. |
| 2015 | FECD 3 | AD | 18q21 .2 | *TCF4* | intron | CTG | - | 5-31 | >50 | unkno wn | L, GWA S | Moot ha et al. |
| 2017 | XDP | XLR | Xq13. 1 | *TAF1* | intron (retrot ransp oson) | CCCT CT | - | none | 30-55 | unkno wn | L, exp | Bragg et al. |
| 2017 | SCA3 7 | AD | 1p32 | *DAB1* | intron | ATTT T | ATTT C | 7-400 (ATT TT) | ≥31-75 ATTT C | RNA | L, ExScr | Seixas et al. |
| 2018 | FAM E1 | AD | 8q24 | *SAM D12* | intron | ATTT T | ATTT C | 7-exp (ATT TT) | ≥440-3,680 ATTT C | RNA? | L, ExScr | Ishiur a et al. |
| 2018 | FAM E6 | AD | 16p12 .1 | *TNRC 6A* | intron | ATTT T | ATTT C | N/A | N/A | RNA? | TRhis t | Ishiur a et al. |
| 2018 | FAM E7 | AD | 4q32. 1 | *RAPG EF2* | intron | ATTT T | ATTT C | N/A | N/A | RNA? | TRhis t | Ishiur a et al. |
| 2019 | BSS | AR | 16p12 .3 | *XYLT* 1 | 5' UTR | CGG | - | 9-20 | 120-800 | Me/G S | ADO, Me | LaCro ix et al. |
| 2019 | CAN VAS | AR | 4p14 | *RFC1* | intron | AAA AG / AAA GG / AAG AG / AGA GG | AAG GG | variab le | ≥400-2,000 | unkno wn | L, WGS | Cortes e et al., Rafeh i et al. |
| 2019 | GDP AG | AR | 2q32. 2 | *GLS* | 5' UTR | GCA | - | 8-16 | ≥680-1,400 | EGS | CGA | van Kuile nburg et al. |
| 2019 | NIID | AD | 1q21. 2 | *NOTC H2NL C* | *5'* UTR / exon 1 | CGG | - | 7-60 | ≥61-500 | Me/G S, RAN (poly G)? | TRhis t, L, WGS | Ishiur a et al., Sone et al., Tian et al. |
| 2019 | OPD M1 | AD | 8q22. 3 | *LRP1 2* | 5' UTR | CGG | - | 13-45 | 90-130 | RAN (poly G)? | TRhis t | Ishiur a et al. |
| 2019 | OPM L1 | AD | 10q22 .3 | *NUT M2B-AS1* | nonco ding RNA gene | CGG/ CCG | - | 3-16 | 40-60 | RAN (poly G)? | TRhis t | Ishiur a et al. |
| 2019 | FAM E4 | AD | 3q27. 1 | *YEAT S2* | intron | ATTT T | ATTT C | 7-400 (ATT TT) | N/A | RNA? | L, ExScr | Yeeto ng et al. |
| 2019 | FAM E2 | AD | 2q11. 2 | *STAR D7* | intron | ATTT T | ATTT C | 9-20 | ≥661-735 | RNA? | L, ExScr | Corbe tt et al. |
| 2019 | FAM E3 | AD | 5p15. 2 | *MAR CHF6* | intron | ATTT T | ATTT C | 10-30 | ≥660-2,800 | RNA? | L, ExScr | Floria n et al. |
| 2020 | OPD M2 | AD | 19p13 .12 | *GIPC* 1 | 5' UTR | CGG | - | 12-32 | ≥97-120 | unkno wn | L, WGS, LRS | Deng et al. |

**Disorder:** BSS, Baratela-Scott syndrome; CANVAS, cerebellar ataxia, neuropathy and vestibular areflexia syndrome; DM1, myotonic dystrophy type 1; DM2, myotonic dystrophy type 2; EPM1, progressive myoclonus epilepsy type 1 (Unverricht-Lundborg disease); FAME, familial adult myoclonic epilepsy; FECD3, Fuchs endothelial corneal dystrophy type 3; FRAXE, fragile XE syndrome; FRDA, Friedreich ataxia; FTD/ALS, frontotemporal dementia/amyotrophic lateral sclerosis; FXPOI, Fragile X-associated premature ovarian infertility; FXS, fragile X syndrome; FXTAS, fragile X-associated tremor ataxia syndrome; GDPAG, global developmental delay, progressive ataxia, and elevated glutamine; NIID, neuronal intranuclear inclusion disease; OPDM1, oculopharyngodistal myopathy type 1; OPDM2, oculopharyngodistal myopathy type 2; OPML1, oculopharyngeal myopathy with leukoencephalopathy type 1; SCA, spinocerebellar ataxia; XDP, X-linked dystonia parkinsonism.

**Inheritance:** AD, autosomal dominant; XL, X-linked; XLR, X-linked, recessive; XLD, X-linked, dominant.

**Pathological Mechanism(s):** EGS, epigenetic gene silencing; Me/GS, gene silencing associated with hypermethylation; PS, protein sequestration; RNA, RNA toxicity; RNA (foci), RNA foci toxicity; RAN, repeat-associated non-AUG translation.

**Identification Method:** ADO, allele drop-out; cl, cloning; CGA, candidate gene analysis; exp, expression; ExScr, expansion screening; Fam, clinical analyses in fragile X-affected families; FS, fragile site; GWAS, genome-wide association study; K, karyotyping; L, linkage; LRS, long-read sequencing; RED, repeat expansion detection; WGS, whole-genome sequencing.

### Short RNA repeat sequence

As used herein, the terms "short RNA repeat sequence" and "short repeat RNA sequence" may be used interchangeably and both refer to a short sequence carrying a normal number of RNA repeat units, which does not cause abnormal intracellular RNA metabolism.

### Gene-editing protein

In the present invention, the gene-editing protein mainly refers to CRISPR proteins. The active ones that have been discovered and verified include Cas13a, Cas13b, Cas13d, Cas13x, Cas13y, SpCas9, SaCas9, and the like.

### PUF protein

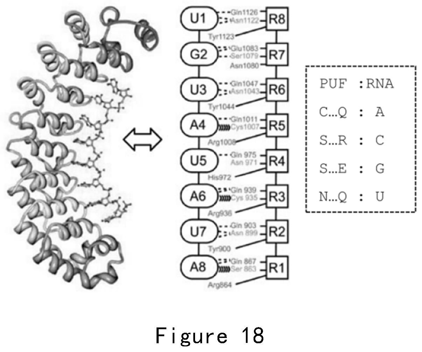

*Drosophila melanogaster* Pumilio and *Caenorhabditis elegans* fem-3 mRNA-binding factor (PUF) are sequence-specific RNA-binding proteins that recognize mRNA targets via repeat motifs within the protein. A typical PUF protein has a C-terminal RNA-binding domain (also known as the PUF domain), which contains eight tandem repeat sequences, as well as N- and C-terminal regions. The eight PUF repeat sequences are arranged in an arc, with each repeat sequence consisting of three α-helices. RNA binds to the concave surface of the PUF domain in an antiparallel manner, with each base specifically recognized by one repeat sequence. The second α-helix of each repeat sequence contains a conserved RNA recognition code consisting of 5 amino acid residues, denoted as "12XX5" (X represents a random residue), wherein residues at positions 1 and 5 directly interact with RNA bases via polar interactions, and residue at position 2 stacks with the bases. Stacking residues in each PUF repeat sequence play an important role in binding affinity, while two polar or charged residues determine the specificity of the PUF repeat. Thus, a set of specific recognition codes exists in the native PUF protein: cysteine and glutamine bind adenine; asparagine and glutamine bind uracil; serine and glutamate bind guanine. In 2011, two independent research groups successfully deciphered the missing cytosine recognition code in the PUF code (serine and arginine bind cytosine) via the yeast three-hybrid system. Since then, PUF proteins can recognize any 8-nt RNA by altering their RNA recognition codes, as shown in Figure 18.

The unique structure and relatively short length of the PUF domain make it an ideal RNA-binding scaffold for engineered proteins to regulate RNA processing. Several engineered PUF regulators fused with different functional domains have been successfully developed and applied in various fields. Existing engineered PUF factors are being used in biomedical research and novel therapeutics for RNA-related diseases. Despite successful early applications, the use of custom PUF domains as programmable RNA-binding scaffolds has several limitations. One major limitation is that native PUF proteins recognize 8-nt RNA sequences. However, in some cases, PUF proteins may be required to recognize target sequences of different lengths. For example, when binding to a longer RNA within the entire transcriptome is desired, PUFs with more repeats will minimize off-target effects. Conversely, shorter recognition sites are more practical for some *in vitro* applications to bind RNA substrates at multiple sites. Recent structural studies of PUF domains with different lengths indicate that the RNA recognition capacity of PUF is maximized with 9-10 repeats. Increasing or decreasing the number of repeats still allows binding to the target sequence, but with reduced affinity.

### Screening system and method of the present invention

The present invention constructs for the first time a system for screening a drug for treating long RNA repeat sequence-associated diseases, wherein the system comprises:
(a) a drug to be screened;
(b) the set of nucleic acid constructs according to the first aspect of the present invention;
wherein, when the expression level or intensity of the first detectable label is significantly reduced, and the expression level or intensity of the second detectable label is slightly changed or substantially unchanged, it indicates that the drug to be screened is a drug for treating long RNA repeat sequence-associated diseases.

The system of the present invention mainly consists of plasmids capable of simultaneously expressing RNAs with two different repeat lengths at equal levels.

For ease of screening, RNAs with two different repeat lengths can be linked to different reporter genes, including but not limited to various fluorescent proteins, luciferases, and other reporter genes that can be used to characterize gene expression levels.

The reporter gene system or screening system disclosed in the present invention allows simultaneous detection of the clearance of two different lengths of RNAs by different drug molecules via a variety of detection means, including but not limited to real-time fluorescent quantitative PCR (RT-qPCR), RNA-seq, fluorescence microscopy, microplate reader, Western Blotting, Northern Blotting, flow cytometry, and the like.

The system of the present invention can be used at the mammalian cell level to characterize the clearance effect of different tools on long repeat sequences, and can also simultaneously observe the clearance effect of each system on short repeat sequences.

The system of the present invention can be used at the model animal (zebrafish, mouse) level to characterize the clearance effect of different tools on long repeat sequences, and can also simultaneously observe the clearance effect of each system on short repeat sequences.

Further, according to the present invention, RNAs with two different repeat lengths can be expressed simultaneously at equal (or similar) levels, facilitating subsequent detection and evaluation.

According to the present invention, available expression modes include bicistronic expression or dual-gene divergent expression, using identical (or similarly strong) eukaryotic promoters, including but not limited to commonly used CMV promoter, CAG promoter, PGK promoter, EF-1α promoter, SV40 promoter, and other alternative promoters.

According to the present invention, the two simultaneously expressed RNAs contain RNA repeat sequences of two different lengths, one being a normal-length RNA with a shorter RNA repeat sequence, and the other being an abnormal-length RNA containing a longer RNA repeat sequence.

The present invention develops a reporter system for screening drugs targeting long RNA repeat sequences. Based on the screening method targeting RNA repeat sequences developed by the present invention, potential drugs for treating RNA repeat sequence-associated diseases can be screened quickly and accurately. The screening method of the present invention integrates technologies from multiple fields such as bioinformatics, enabling comprehensive analysis and evaluation of the potential therapeutic capacity of various drugs.

The development of the reporter system of the present invention will help to further understand the pathophysiological mechanisms of long RNA repeat sequence-associated diseases and promote the development and progress of therapeutic methods for such diseases. Meanwhile, the application of this reporter system in drug screening for RNA repeat sequence-associated diseases will have broad prospects in the whole research field, bringing more opportunities and possibilities for the treatment of RNA-related diseases. By further investigating the molecular mechanisms and clinical manifestations of RNA repeat sequence-associated diseases, we will have the opportunity to design and discover more innovative and therapeutically effective drugs for such diseases.

Furthermore, the present invention establishes a system capable of simultaneously expressing RNAs with two different repeat lengths at equal levels. This system simulates the *in vivo* situation where alleles are expressed at similar levels but carry different repeat lengths, making it highly suitable for drug screening against such diseases.

### Main advantages of the present invention include:

(1) The present invention develops for the first time a set of nucleic acid constructs for screening drugs for treating long RNA repeat sequence-associated diseases, as well as vectors and screening systems based on the set of nucleic acid constructs. Using the set of nucleic acid constructs, vectors, and screening systems, drugs for treating long RNA repeat sequence-associated diseases can be screened or identified in a high-throughput manner.
(2) Based on the screening method targeting RNA repeat sequences developed by the present invention, potential drugs for treating RNA repeat sequence-associated diseases can be screened quickly and accurately. The screening method of the present invention integrates technologies from multiple fields such as bioinformatics, enabling comprehensive analysis and evaluation of the potential therapeutic capacity of various drugs. The present invention has been completed on this basis.
(3) Using the system of the present invention, drugs targeting long repeat sequences can be characterized or screened while their effects on short repeat sequences are also evaluated. Moreover, due to the use of identical promoter elements, the two lengths of RNAs are expressed at similar levels, which closely resembles the *in vivo* condition of diseases caused by long repeat sequences in humans.
(4) At present, no commercial similar system is available. Existing systems generally only focus on characterizing effects on long repeat sequences while neglecting characterization of short repeat sequences.
(5) The system of the present invention can also be used in cells and model animals (zebrafish, mice).
(6) The system of the present invention can be used to screen and obtain drugs for treating long RNA repeat sequence-associated diseases, such as artificial RNA degrading enzymes having the specific Formula IV or IV' structure of the present invention.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturers. Percentages and parts are calculated by weight unless otherwise stated.

Unless otherwise specified, materials and reagents in the examples are all commercially available products.

### Example 1 Construction of Reporter Plasmids Containing CTG Repeat Sequences

First, DNA fragments expressing mCherry and EGFP were obtained by PCR amplification, and the pcDNA5-FRT/TO plasmid (purchased from Thermo Fisher) was linearized by PCR. The mCherry fragment and the linearized pcDNA5-FRT/TO vector were circularized by homologous recombination using Gibson Assembly Master Mix (purchased from New England Biolabs). The circularized product was transformed into DH5α competent cells (purchased from Beijing TransGen Biotech Co., Ltd.) and cultured according to the manufacturer's instructions. The resulting plasmids were confirmed by Sanger sequencing as pcDNA5-FRT/TO-mCherry and pcDNA5-FRT/TO-EGFP.

The synthetic (CTG)₁₄ DNA fragment (purchased from Genewiz) was inserted into the pre-constructed pcDNA5-FRT/TO-mCherry by PCR at the C-terminus of the mCherry gene, thereby obtaining the plasmid pcDNA5-FRT/TO-mCherry-(CTG)₁₄ expressing mCherry-(CTG)₁₄.

The restriction enzyme SapI site was inserted into the pre-constructed pcDNA5-FRT/TO-EGFP by PCR at the C-terminus of the EGFP gene, thereby obtaining pcDNA5-FRT/TO-EGFP-IN.

The synthetic (CTG)₅₀ DNA fragment was digested with EarI and ligated into the SapI-digested pcDNA5-FRT/TO-EGFP-IN vector to obtain pcDNA5-FRT/TO-EGFP-(CTG)₅₀ (denoted as 50CUG). This digestion-ligation process was repeated to obtain pcDNA5-FRT/TO-EGFP-(CTG)₁₀₀ (denoted as 100CUG), pcDNA5-FRT/TO-EGFP-(CTG)₁₅₀ (denoted as 150CUG), and pcDNA5-FRT/TO-EGFP-(CTG)₂₀₀ (denoted as 200CUG). During construction, due to contraction of CTG repeat sequences during *E. coli* culture, the plasmid pcDNA5-FRT/TO-EGFP-(CTG)₁₃₀ (denoted as 130CUG) was also obtained.

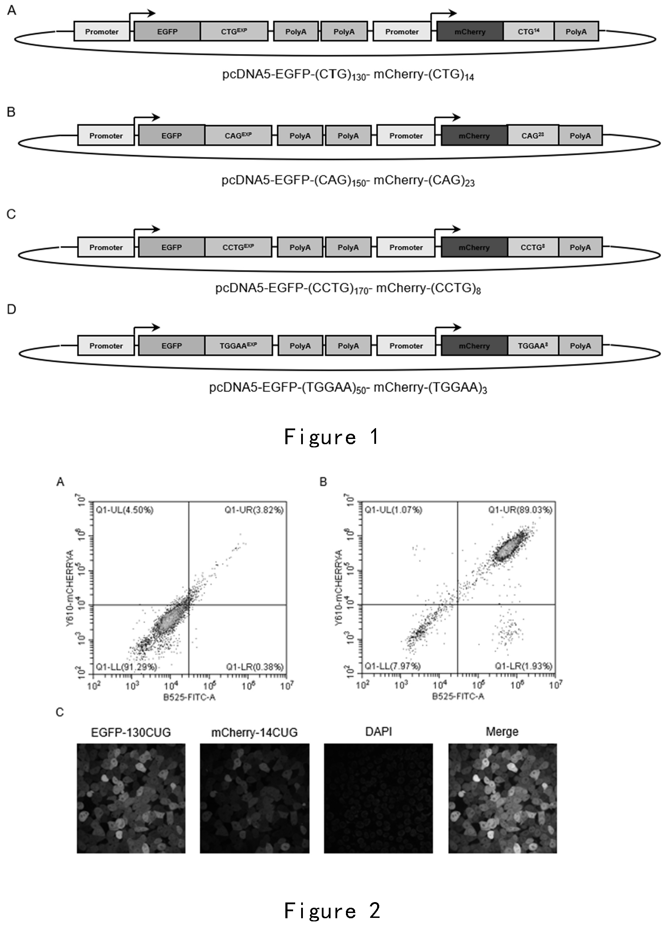

The pcDNA5-FRT/TO-mCherry-(CTG)₁₄ plasmid was modified by PCR to introduce unique NotI and KpnI restriction sites to generate a vector. DNA fragments containing EGFP and different lengths of CUG repeats were obtained by NotI and KpnI digestion of 50CUG, 100CUG, 130CUG, 150CUG, 200CUG, etc. Homologous recombination was performed to obtain vectors capable of simultaneously expressing two different lengths of CUG repeat sequences at equal levels, which express two different fluorescent proteins as indicators of RNA expression levels (Figure 1, panel A).

### Example 2 Construction of Reporter Plasmids Containing CCTG Repeat Sequences

The synthetic (CCTG)₈ DNA fragment was inserted into the pre-constructed pcDNA5-FRT/TO-mCherry by PCR at the C-terminus of the mCherry gene, thereby obtaining pcDNA5-FRT/TO-mCherry-(CCTG)₈.

The restriction enzyme BbsI site was inserted into the pre-constructed pcDNA5-FRT/TO-EGFP by PCR at the C-terminus of the EGFP gene, thereby obtaining pcDNA5-FRT/TO-EGFP-IN.

The synthetic (CCTG)₅₀ DNA fragment was digested with BbsI and Esp3I and ligated into the BbsI-digested pcDNA5-FRT/TO-EGFP-IN vector to obtain pcDNA5-FRT/TO-EGFP-(CCTG)₅₀ (denoted as 50CCUG). This digestion-ligation process was repeated to obtain pcDNA5-FRT/TO-EGFP-(CCTG)₁₀₀ (denoted as 100CCUG), pcDNA5-FRT/TO-EGFP-(CCTG)₁₅₀ (denoted as 150CCUG), and pcDNA5-FRT/TO-EGFP-(CCTG)₂₀₀ (denoted as 200CCUG). Similarly, due to contraction of CCTG repeat sequences during *E. coli* culture, the plasmid pcDNA5-FRT/TO-EGFP-(CCTG)₁₇₀ (denoted as 170CCUG) was also obtained.

The pcDNA5-FRT/TO-mCherry-(CCTG)₈ plasmid was modified by PCR to introduce unique NotI and KpnI restriction sites to generate a vector. DNA fragments containing EGFP and different lengths of CCUG repeats were obtained by NotI and KpnI digestion of 50CCUG, 100CCUG, 150CCUG, 200CCUG, etc. Homologous recombination was performed to obtain vectors capable of simultaneously expressing two different lengths of CCUG repeat sequences at equal levels, which express two different fluorescent proteins as indicators of RNA expression levels (Figure 1, panel B).

### Example 3 Construction of Reporter Plasmids Containing CAG Repeat Sequences

The synthetic (CAG)₂₃ DNA fragment was inserted into the pre-constructed pcDNA5-FRT/TO-mCherry by PCR at the C-terminus of the mCherry gene, thereby obtaining pcDNA5-FRT/TO-mCherry-(CAG)₂₃.

The restriction enzyme BbsI site was inserted into the pre-constructed pcDNA5-FRT/TO-EGFP by PCR at the C-terminus of the EGFP gene, thereby obtaining pcDNA5-FRT/TO-EGFP-IN.

The synthetic (CAG)₅₀ DNA fragment was digested with BbsI and Esp3I and ligated into the BbsI-digested pcDNA5-FRT/TO-EGFP-IN vector to obtain pcDNA5-FRT/TO-EGFP-(CAG)₅₀ (denoted as 50CAG). This digestion-ligation process was repeated to obtain pcDNA5-FRT/TO-EGFP-(CAG)₁₀₀ (denoted as 100CAG), pcDNA5-FRT/TO-EGFP-(CAG)₁₅₀ (denoted as 150CAG), and pcDNA5-FRT/TO-EGFP-(CAG)₂₀₀ (denoted as 200CAG).

The pcDNA5-FRT/TO-mCherry-(CAG)₂₃ plasmid was modified by PCR to introduce unique NotI and KpnI restriction sites to generate a vector. DNA fragments containing EGFP and different lengths of CAG repeats were obtained by NotI and KpnI digestion of 50CAG, 100CAG, 150CAG, 200CAG, etc. Homologous recombination was performed to obtain vectors capable of simultaneously expressing two different lengths of CAG repeat sequences at equal levels, which express two different fluorescent proteins as indicators of RNA expression levels (Figure 1, panel C).

### Example 4 Construction of Reporter Plasmids Containing TGGAA Repeat Sequences

The synthetic (TGGAA)₃ DNA fragment was inserted into the pre-constructed pcDNA5-FRT/TO-mCherry by PCR at the C-terminus of the mCherry gene, thereby obtaining pcDNA5-FRT/TO-mCherry-(TGGAA)₃.

The restriction enzyme BbsI site was inserted into the pre-constructed pcDNA5-FRT/TO-EGFP by PCR at the C-terminus of the EGFP gene, thereby obtaining pcDNA5-FRT/TO-EGFP-IN.

The synthetic (TGGAA)₅₀ DNA fragment was digested with BbsI and Esp3I and ligated into the BbsI-digested pcDNA5-FRT/TO-EGFP-IN vector to obtain pcDNA5-FRT/TO-EGFP-(TGGAA)₅₀ (denoted as 50TGGAA). This digestion-ligation process was repeated to obtain pcDNA5-FRT/TO-EGFP-(TGGAA)₁₀₀ (denoted as 100TGGAA).

The pcDNA5-FRT/TO-mCherry-(TGGAA)₃ plasmid was modified by PCR to introduce unique NotI and KpnI restriction sites to generate a vector. DNA fragments containing EGFP and different lengths of TGGAA repeats were obtained by NotI and KpnI digestion of 50TGGAA, 100TGGAA, etc. Homologous recombination was performed to obtain vectors capable of simultaneously expressing two different lengths of TGGAA repeat sequences at equal levels, which express two different fluorescent proteins as indicators of RNA expression levels (Figure 1, panel D).

### Example 5 Construction of Cell Lines Stably Expressing Reporter Plasmids

The above-obtained plasmids containing different lengths of CTG/CCTG/CAG/TGGAA repeat sequences and the plasmid pOG44 (purchased from Thermo Fisher) were co-transfected into Flp-In T-Rex 293 cells (purchased from Thermo Fisher) at a ratio of 1:9. 48 hours after transfection, cells were washed and plated in fresh medium (<25% confluency), and cultured overnight at 37°C under 5% CO₂. Subsequently, cells were cultured in fresh medium containing 100 µg/mL Hygromycin (purchased from Thermo Fisher) every 3 days until single resistant cell colonies appeared. 3-4 individual colonies were selected for further use. The correctness of the inserted sequence was verified by Sanger sequencing.

The resulting cell lines stably expressing the reporter plasmids can be induced for expression by the addition of doxycycline (purchased from Sigma-Aldrich) or tetracycline at a final concentration of 1 µg/mL. The expression of the reporter system at the cellular level can be detected using a fluorescence microscope, flow cytometer, or other detection methods. Figure 2 shows the induced expression results of the dual-reporter gene system with CUG repeats in Flp-In T-REX 293 cells. Figure 2, panel A shows that both fluorescent reporter genes were not expressed without the addition of the inducer; after induction with 1 µg/mL doxycycline (Figure 2, panel B), both fluorescent proteins showed high expression. Figure 2, panel C shows the fluorescence microscopy results. The results in Figure 2 demonstrate that stable cell lines carrying reporter genes with different lengths of RNA repeat sequences can stably express the two different reporter genes and the carried RNA repeat sequences.

### Example 6 Comparison of Existing Regulatory Methods Targeting RNA Repeat Sequences at the Cellular Level

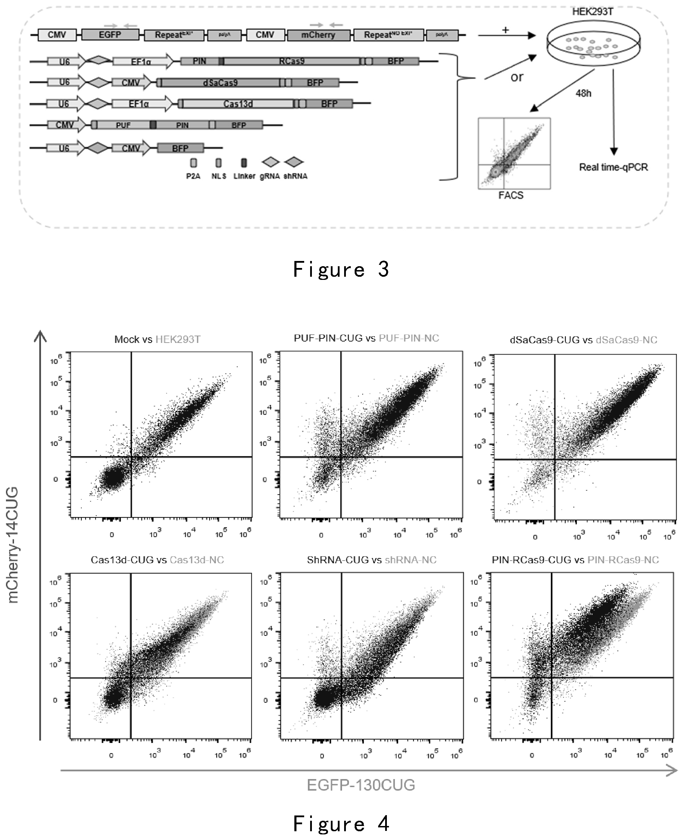

Plasmids capable of expressing the Cas13d system targeting CUG repeat sequences (purchased from Addgene), PIN-RCas9 system plasmid (purchased from Addgene), ASRE(CUG) plasmid (in-house), shRNA(CUG) plasmid (purchased from Addgene), antisense oligonucleotide (ASO) (purchased from GenePharma), and the control empty vector pcDNA3.1 (purchased from Thermo Fisher) were co-transfected with the 150CUG plasmid of the present invention into HEK 293T cells (purchased from ATCC) using Lipofectamine 3000 (purchased from Thermo Fisher) at a ratio of 1:10. The specific steps followed the Lipofectamine 3000 manual. 48 hours after transfection, adherent cells were digested with trypsin, collected by centrifugation at 800 g, and washed with PBS (experimental design and workflow shown in Figure 3).

Cells were then divided equally into three parts. The first part was used for flow cytometry to detect the fluorescence levels of EGFP and mCherry (results shown in Figure 4). Figure 4 shows that, using the screening and comparison system provided by the present invention, among the existing regulatory methods, only the PIN-RCas9 system significantly reduced the long RNA repeat sequence EGFP-130CUG, as indicated by decreased EGFP protein expression level.

The second part was collected by centrifugation again, lysed with RIPA buffer (purchased from Thermo Fisher), supplemented with 2× Laemmli Sample Buffer (purchased from Bio-Rad), heated at 99°C for 10 min, and used for Western Blotting identification.

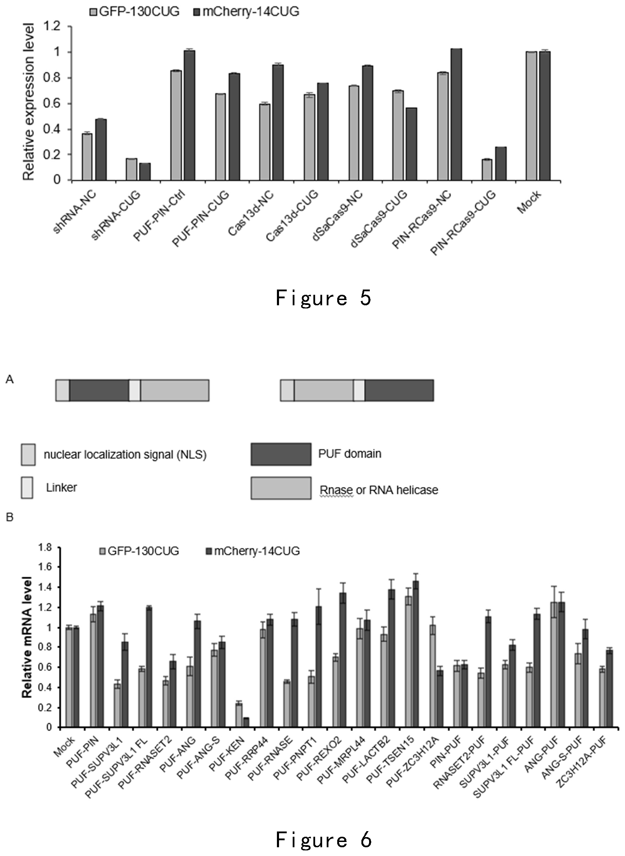

The third part was lysed with TRIzol Reagent (purchased from Thermo Fisher), and total RNA was extracted according to the manual. Reverse transcription was performed using the PrimeScript RT Reagent Kit with gDNA Eraser (TAKARA) or other commercial reverse transcription kits. Subsequently, quantitative PCR was performed using 2× Universal SYBR Green Fast qPCR Mix reagents (purchased from ABclonal) to compare the clearance effects and off-target effects of the regulatory methods used (results shown in Figure 5). Figure 5 shows that among all compared methods, only shCUG and PIN-RCas9(CUG) achieved clearance of the target long RNA repeat sequence, but shCUG showed poor selectivity.

Primer sequences for quantitative PCR were as follows: EGFP-F1: 5'-AGTGCTTCAGCCGCTACCC -3'; EGFP-R1: 5'- GTTGTACTCCAGCTTGTGCC - 3'; mCherry-F1: 5'- CTTCAAGTGGGAGCGCGTG -3'; mCherry-R1: 5'-CAGCCTCTGCTTGATCTCGCC -3'; GAPDH-F: 5'-TTGCCATCAATGACCCCTTCA -3'; GAPDH-R: 5'-CGCCCCACTTGATTTTGGA-3'.

### Example 7 Construction and Detection of Novel Artificial RNA Enzymes

The plasmid expressing ASRE(CUG) was linearized by PCR, using the following primers: PUF-R: 5'-CTGAGAGCCGTTGCCGGTGTCGACCCCTAAGTCAACACCGTTC -3'; cDNA-F: 5'- GCTAGCGTTTAAACTTAAGCTTGGTACC -3'. The structure of the selected ASRE(CUG) artificial RNA enzyme portion was NLS-PUF(CUG)-Linker-PIN.

Full-length DNA or an active portion DNA fragment thereof of the following RNases or RNA helicases was amplified from HEK 293T cDNA by PCR amplification method, including but not limited to: ANG (UNIPROT: P03950), RNASEL (UNIPROT: Q05823), RRP44 (UNIPROT: Q9Y2L1), RNASE1 (UNIPROT: P07998), RNASET2 (UNIPROT: O00584), SUPV3L1 (UNIPROT: Q8IYB8), PNPT1 (UNIPROT: Q8TCS8), REXO2 (UNIPROT: Q9Y3B8), MRPL44 (UNIPROT: Q9H9J2), LACTB2 (UNIPROT: Q53H82), TSEN15 (UNIPROT: Q8WW01), ZC3H12A (UNIPROT: QSD1E8). All amplified fragments contained DNA bases (>20 bp) complementary to the linearized ASRE(CUG) plasmid. These DNA fragments and the linearized ASRE(CUG) vector were circularized using Gibson Assembly Master Mix (purchased from New England Biolabs) to replace the original domain and generate novel artificial RNA enzymes: PUF-ANG, PUF-ANG-S, PUF-RNASEL, PUF-SUPV3L1, PUF-SUPV3L1 FL, PUF-RNASET2, PUF-RRP44, PUF-RNASE1, PUF-PNPT1, PUF-REXO2, PUF-MRPL44, PUF-LACTB2, PUF-TSEN15, and PUF-ZC3H12A. Similarly, ANG-PUF, ANG-S-PUF, SUPV3L1-PUF, SUPV3L1 FL-PUF, RNASET2-PUF, RNASE1-PUF, PNPT1-PUF, REX02-PUF, and ZC3H12A-PUF may also constructed by PCR and homologous recombination (the structure of the resulting novel artificial RNA enzyme elements is shown in Figure 6, panel A, which is schematic only).

1 µg of the novel artificial RNA enzyme plasmid and 0.1 µg of the reporter plasmid pcDNA5-EGFP-CTG130-mCherry-CTG14 were co-transfected into HEK 293T cells (cell confluency about 80%) using Lipofectamine 3000 (purchased from Thermo Fisher) according to the manual. Transfected cells were collected 48 hours after transfection. The obtained cells were lysed with TRIzol Reagent (purchased from Thermo Fisher), and total RNA was extracted according to the manual. Reverse transcription was performed using PrimeScript RT Reagent Kit with gDNA Eraser (TAKARA) or other commercial reverse transcription kits. Subsequently, quantitative PCR was performed using 2× Universal SYBR Green Fast qPCR Mix reagents (purchased from ABclonal) (results shown in Figure 6, panel B). The results in Figure 6, panel B show that PUF-RNASE1, PUF-SUPV3L1 (ARC-S), PUF-SUPV3L1 FL, PUF-RNASET2, PUF-PNPT1, PUF-REXO2, RNASE1-PUF, PNPT1-PUF, REXO2-PUF, SUPV3L1-PUF, SUPV3L1 FL-PUF, and RNASET2-PUF (ARC-T; ARC: Artificial RNA Cleaver, Artificial RNA Enzyme) exhibited better selectivity for the two different lengths of CTG repeat sequences, with strong reduction effects on long CUG repeat sequences.

Subsequently, ARC-S and ARC-T, which showed outstanding detection results, were co-transfected with the 130CUG plasmid of the present invention into HEK 293T cells (purchased from ATCC) using Lipofectamine 3000 (purchased from Thermo Fisher) at a ratio of 1: 10. The specific steps followed the Lipofectamine 3000 manual. 48 hours after transfection, adherent cells were digested with trypsin, collected by centrifugation at 800 g, and washed with PBS (experimental design and workflow shown in Figure 3).

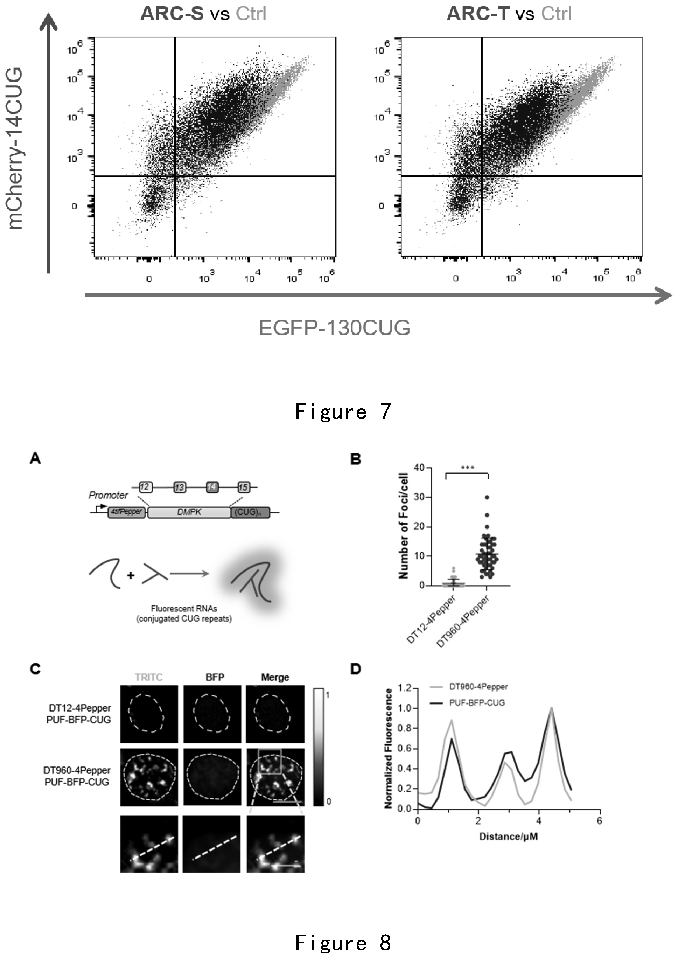

Cells were then used for flow cytometry to detect the fluorescence levels of EGFP and mCherry (results shown in Figure 7). Figure 7 shows that, using the screening and comparison system provided by the present invention, the newly constructed artificial RNA enzymes ARC-S and ARC-T significantly reduced the long RNA repeat sequence EGFP-130CUG (indicated by decreased EGFP protein expression level) without obvious effects on mCherry-14CUG containing short repeat sequences.

### Example 9

Based on the reported imaging results of fluorescent RNA, the DNA sequence with a fluorescent RNA segment (4Pepper, provided by East China University of Science and Technology) was inserted into plasmids DT12 (purchased from Addgene, Cat#80416) and DT960 (purchased from Addgene, Cat#80412) by PCR, restriction digestion, and ligation, at a position before DMPK exon 12 and after the CMV promoter, thereby obtaining plasmids DT12-4Pepper and DT960-4Pepper (Figure 8, panel A).

DT12-4Pepper and DT960-4Pepper were co-transfected with the NLS-PUF-BFP-CUG plasmid (constructed according to the method in Example 1) into HEK 293T cells (purchased from ATCC) using Lipofectamine 3000 (purchased from Thermo Fisher), respectively. The specific steps followed the Lipofectamine 3000 manual. 48 hours after transfection, cells were imaged using a confocal fluorescence microscope, and the number of RNA aggregates in the nucleus was counted and detected (Figure 8, panel B) (n=50). The statistical results in Figure 8, panel B show that DT960-4Pepper forms more RNA aggregates in the nucleus (consistent with literature reports), whereas DT12-4Pepper forms only a few or no RNA aggregates. Meanwhile, quantitative analysis of the distribution of fluorescent RNA labeling CUG repeat sequences and fluorescent protein NLS-PUF-BFP targeting CUG repeat sequences was performed (Figure 8, panel C), revealing a positive correlation between their distributions (Figure 8, panel D).

### Example 10 Clearance of Intranuclear Aggregation of RNA Repeat Sequences by Different Systems

Existing different systems targeting RNA repeat sequences (all plasmids were labeled with BFP for imaging detection) were co-transfected with DT960-4Pepper into HEK 293T cells (purchased from ATCC) using Lipofectamine 3000 (purchased from Thermo Fisher). The specific steps followed the Lipofectamine 3000 manual. 48 hours after transfection, cells were imaged using a confocal fluorescence microscope to detect the number of RNA aggregates in the nucleus. In imaging results, the dye SYTO 9 was used to distinguish cytoplasm and nucleus (FITC channel, green), blue fluorescent protein BFP labeled on different system plasmids was used to identify successfully transfected cells, and the TRITC channel was used to select cells successfully transfected with DT960-4Pepper (orange). Cells expressing both BFP and DT960-4Pepper were used to count the number of intranuclear RNA aggregates.

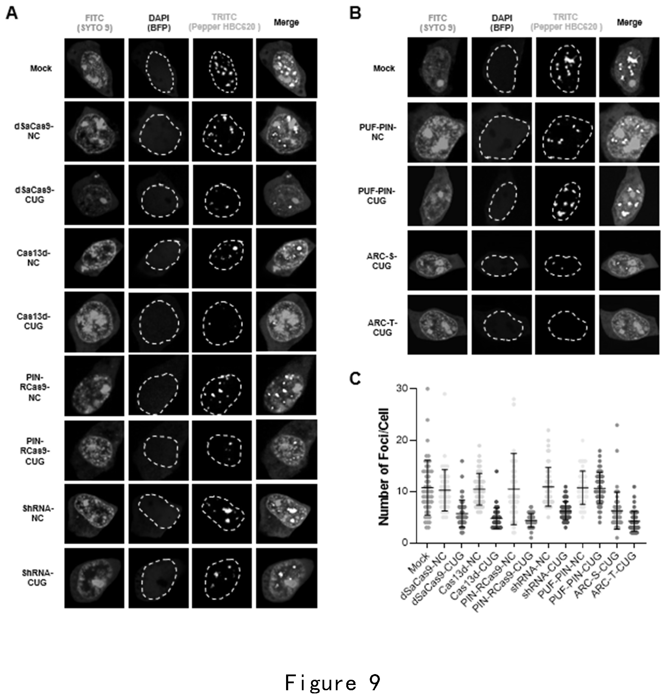

Imaging results show that among non-PUF-related regulatory systems, the PIN-RCas9 system exhibited the best clearance ability (Figure 9, panel A) (n=50). Among PUF-related regulatory systems, the ARC-T constructed in the present application exhibited the best clearance ability (Figure 9, panel B) (n=50). In the comparison of all systems, the PIN-RCas9 system and ARC-T showed similar clearance effects (Figure 9, panel C).

### Example 11 Pathological Lesion Clearance and Molecular Phenotype Alleviation by Novel Artificial RNA Enzymes

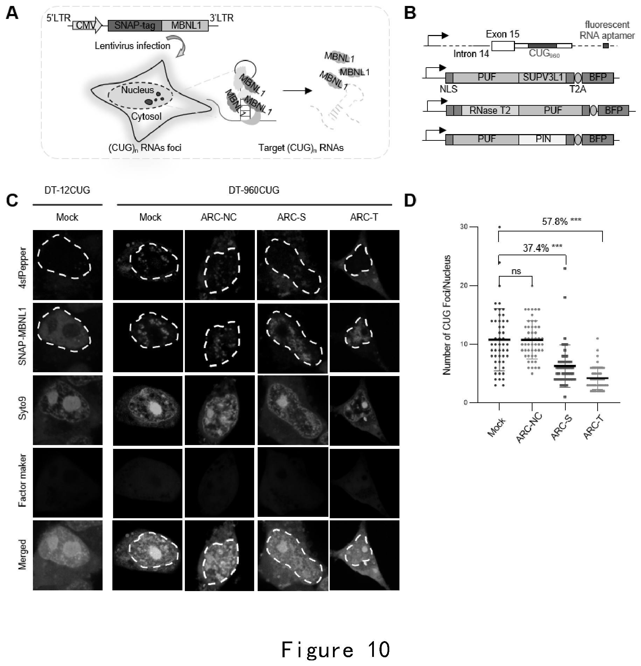

A cell line stably overexpressing MBNL1 was constructed using HEK 293T cells (purchased from ATCC). The synthetic MBNL1 gene sequence (Uniprot: Q9NR56Q) was constructed into the lentiviral expression vector pcDH-Ecmv-Puro (purchased from Addgene) to obtain the target expression plasmid. The envelope plasmid (pMD2G), packing plasmid (pSPAX) (purchased from Addgene), and target plasmid were co-transfected into cells at a ratio of 1:3:4. The specific steps followed the Lipofectamine 3000 manual. Viral supernatant was harvested 48 h after transfection. The packaged viral supernatant was used to infect HEK 293T cells. 48 h after infection, Puromycin (Gibco) at a concentration of 5 µg/ml was added to select successfully infected cells, and a cell line stably overexpressing MBNL1 was finally obtained (Figure 10, panel A).

The obtained novel artificial RNA enzymes (all plasmids were labeled with BFP for imaging detection) were co-transfected with DT960-4Pepper into the above MBNL1-overexpressing cell line using Lipofectamine 3000 (purchased from Thermo Fisher) (Figure 10, panel B). The specific steps followed the Lipofectamine 3000 manual. 48 hours after transfection, cells were imaged using a confocal fluorescence microscope to detect the number of RNA aggregates in the nucleus and observe the nuclear distribution of MBNL1. In imaging results, the dye SYTO 9 (purchased from Thermo Fisher) was used to distinguish cytoplasm and nucleus (FITC channel, green), blue fluorescent protein BFP labeled on different system plasmids was used to identify successfully transfected cells, the TRITC channel was used to select cells successfully transfected with DT960-4Pepper (red), and the snap tag was used to indicate the distribution of MBNL1 protein in the nucleus (purple). Cells expressing both BFP and DT960-4Pepper were used to count the number of intranuclear RNA aggregates.

Imaging results show that, compared with the Mock control group, the novel artificial RNA enzymes ARC-S and ARC-T constructed in the present application not only efficiently cleared pathogenic RNA aggregates (Figure 10, panel C) but also significantly improved the mislocalization of MBNL1, changing its nuclear distribution from abnormal punctate aggregates to diffuse and uniform distribution in the nucleus. In statistical results, the artificial enzyme system significantly reduced the number of pathogenic RNA aggregates (n=50) (Figure 10, panel D).

### Example 12 Sequence-Optimized Novel Artificial RNA Enzymes Show Enhanced Efficiency

The sequences of the novel artificial RNA enzymes constructed in the present application were optimized using publicly available sequence optimization algorithms from GENEWIZ, GenScript, and Thermo, which can enhance the expression level and functional efficiency of the artificial enzymes.

Sequence-optimized novel artificial RNA enzymes were constructed into the same expression vector pcDNA5 (purchased from Thermo Fisher) and co-transfected with the 150CUG plasmid of the present invention into HEK 293T cells (purchased from ATCC) using Lipofectamine 3000 (purchased from Thermo Fisher) at a ratio of 1:10. The specific steps followed the Lipofectamine 3000 manual. 48 hours after transfection, adherent cells were digested with trypsin, collected by centrifugation at 800 g, and washed with PBS (experimental design and workflow similar to those described above).

Cells were divided equally into three parts. The first part was used for flow cytometry to detect the fluorescence levels of EGFP and mCherry (results shown in Figure 11, panel A and Figure 11, panel B). The results show that sequence-optimized artificial RNA enzymes retained the pathogenic RNA targeting and clearance ability claimed in the present application. Both ARC-S and ARC-T significantly reduced the expression of the long RNA repeat sequence EGFP-130CUG, as indicated by decreased EGFP protein expression level.

The second part was collected by centrifugation again, lysed with RIPA buffer (purchased from Thermo Fisher), supplemented with 2× Laemmli Sample Buffer (purchased from Bio-Rad), heated at 99°C for 10 min, and used for Western Blotting (Figure 11, panel D). The results show that different sequence optimization designs affect the expression levels of the two artificial RNA enzymes in the present application; sequences optimized using the GENEWIZ algorithm showed higher protein expression levels than the original sequence and sequences optimized using other optimization methods.

The third part was lysed with TRIzol Reagent (purchased from Thermo Fisher), and total RNA was extracted according to the manual. Reverse transcription was performed using the PrimeScript RT Reagent Kit with gDNA Eraser (TAKARA) or other commercial reverse transcription kits. Subsequently, quantitative PCR was performed using 2× Universal SYBR Green Fast qPCR Mix reagents (purchased from ABclonal) to compare the clearance effects and off-target effects of the regulatory methods (results shown in Figure 11, panel C). The results show that, among all sequence optimization methods, increased expression of artificial RNA enzymes improved pathogenic RNA clearance ability.

Primer sequences for quantitative PCR were as follows: EGFP-F1: 5'-AGTGCTTCAGCCGCTACCC -3'; EGFP-R1: 5'- GTTGTACTCCAGCTTGTGCC - 3'; mCherry-F1: 5'- CTTCAAGTGGGAGCGCGTG -3'; mCherry-R1: 5'-CAGCCTCTGCTTGATCTCGCC -3'; GAPDH-F: 5'-TTGCCATCAATGACCCCTTCA -3'; GAPDH-R: 5'-CGCCCCACTTGATTTTGGA -3'.

### Example 13 Purified Novel Artificial RNA Enzymes Also Exert Pathogenic RNA Cleavage Activity In Vitro

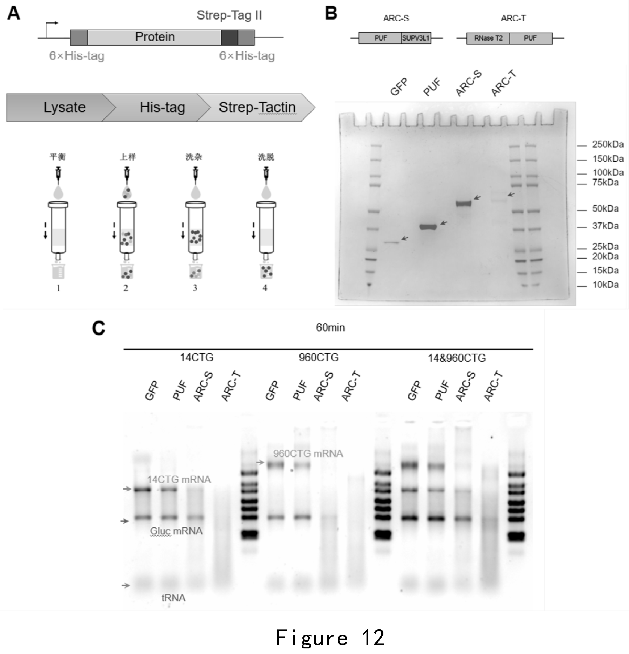

*In vitro* expression and purification of the novel artificial RNA enzyme proteins further support the effectiveness of the present invention / application. The protein-coding sequences of PUF, GFP, ARC-S, and ARC-T were constructed into the prokaryotic expression vector pET28a (purchased from Addgene) and prepared by *in vitro* protein expression through bacterial culture. Bacterial lysates were isolated and purified by two-step chromatography using His-tag and Strep-Tactin resin columns (purchased from Thermo) to obtain high-purity protein products, following the manufacturer's instructions (Figure 12, panel A). *In vitro* purified proteins were analyzed for protein purity by SDS-PAGE, and the results demonstrated that the four purified proteins had correct band sizes and showed a single band, respectively (Figure 12, panel B).

High-purity long and short CUG repeat sequence RNAs were obtained by *in vitro* transcription from the corresponding sequences in aforementioned dual-fluorescent vectors. Gluc mRNA was purchased from Novoprotein, tRNA was purchased from Thermo, and *in vitro* transcription was performed using the MAXIscript^{™} SP6 Transcription Kit (Thermo) according to the manual. 5 µM of PUF, GFP, ARC-S, and ARC-T proteins were incubated with the RNA mixture at 37°C for 60 min. The electrophoresis results showed that PUF and GFP proteins exerted little effect on the degradation of the RNA mixture. ARC-S and ARC-T could significantly degrade mRNA carrying CUG repeat sequences, while having little effect on RNA without CUG repeats. Moreover, the RNA degradation ability of ARC-T was stronger than that of ARC-S (Figure 12, panel C).

### Example 14 Construction of Mouse Strains Stably Expressing Reporter Plasmids

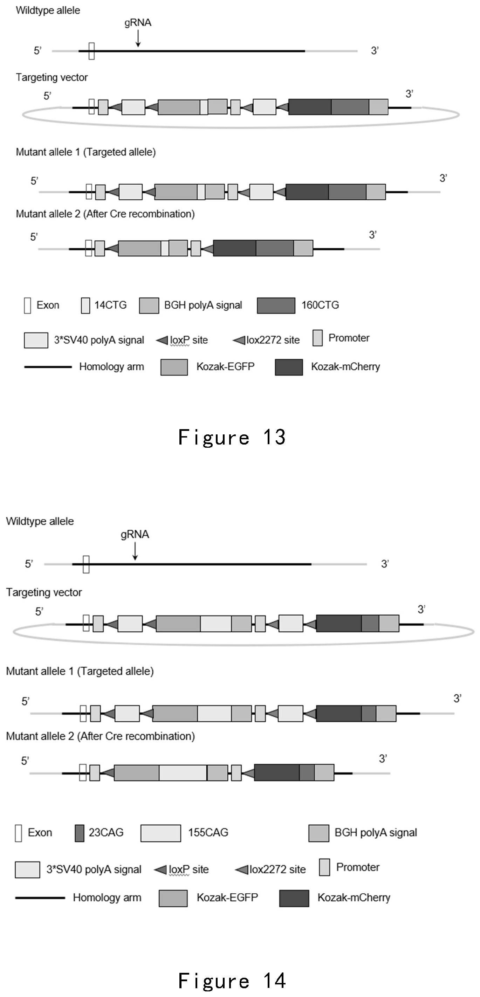

A mouse strain stably expressing the reporter plasmid (denoted as mouse-CUG-R160G14) was constructed via a contract research organization (CRO) (purchased from Cyagen Biosciences). Specifically, a DNA fragment simultaneously expressing EGFP-(CUG)₁₄ and mCherry-(CUG)₁₆₀ RNA sequences was inserted into intron 1 of the ROSA26 gene (NCBI Reference Sequence: NR_027008.1) in the mouse genome (construction strategy shown in Figure 13).

A mouse strain stably expressing the reporter plasmid (denoted as mouse-CAG-G155R23) was constructed via a contract research organization (CRO) (purchased from Cyagen Biosciences). Specifically, a DNA fragment simultaneously expressing EGFP-(CAG)₁₅₅ and mCherry-(CAG)₂₃ RNA sequences was inserted into intron 1 of the ROSA26 gene (NCBI Reference Sequence: NR_027008.1) in the mouse genome (construction strategy shown in Figure 14).

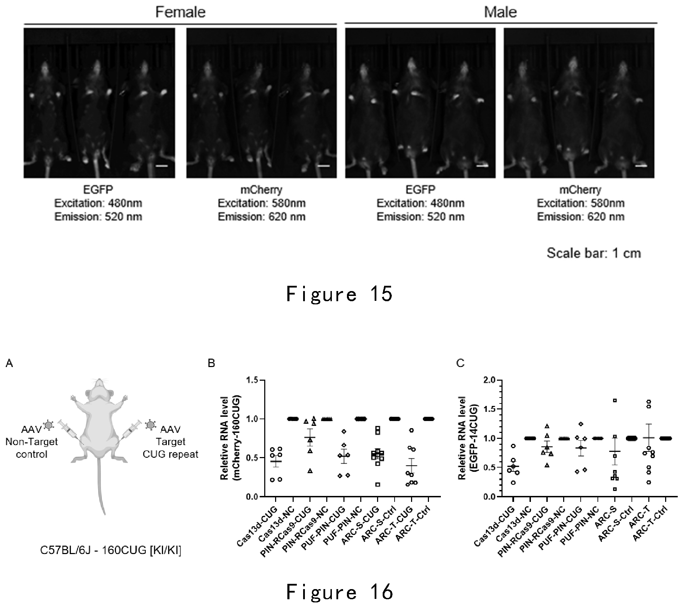

The resulting transgenic mouse mouse-CUG-R160G14 can simultaneously express EGFP-(CUG)₁₄ and mCherry-(CUG)₁₆₀ RNA sequences, thus carrying two different fluorescent signals (Figure 15). This property can be used for the rapid screening and efficacy evaluation of CUG repeat sequence-specific drugs.

### Example 15 Comparison of Existing Regulatory Methods Targeting RNA Repeat Sequences at the In Vivo Level

AAV viral particles capable of expressing the Cas13d system targeting CUG repeat sequences (purchased from OBiO TECH), PIN-RCas9 system plasmid (purchased from OBiO TECH), PUF-PIN (purchased from OBiO TECH), ARC-S (purchased from OBiO TECH), and ARC-T (purchased from OBiO TECH) were injected into the thigh skeletal muscle (right leg) of mouse-CUG-R160G14 mice (purchased from Cyagen Biosciences) at a total dose of 10¹¹ vg per mouse. Meanwhile, the corresponding control AAV viral particles (purchased from OBiO TECH) were injected into the left leg of the same mouse at the same dose (Figure 16, panel A).

Two months after injection, the injected leg muscle was harvested, and total RNA was extracted using TRIzol Reagent (purchased from Thermo Fisher) according to the manual. Reverse transcription was performed using the PrimeScript RT Reagent Kit with gDNA Eraser (TAKARA) or other commercial reverse transcription kits. Subsequently, quantitative PCR was performed using 2× Universal SYBR Green Fast qPCR Mix reagents (purchased from ABclonal) to compare the clearance effects and off-target effects of the regulatory methods (Figure 16, panel B and Figure 16, panel C). Figure 16, panel B shows that ARC-T viral particles exhibited the best clearance effect on mCherry-160CUG with long RNA repeat sequences, followed by Cas13d-CUG, ARC-S, PUF-PIN-CUG, and PIN-RCas9-CUG in sequence. Figure 16, panel C shows that Cas13D-CUG viral particles had the strongest off-target clearance effect on EGFP-14CUG with short RNA repeat sequences (about 50%), while the other four showed comparable and low off-target effects (about 20%), with ARC-T showing the lowest off-target rate.

Primer sequences for quantitative PCR were as follows: EGFP-F2: 5'-CCCGACAACCACTACCTGAG -3'; EGFP-R2: 5'-TGCATCTTACACCAAACTCATCTG -3'; mCherry-F2: 5'-AAGACTATGGGCTGGGAGGC -3'; mCherry-R2: 5'-CTCGTTGTGGGAGGTGATGT -3'; mGAPDH-F: 5'-GTCAAGGCCGAGAATGGGAA -3'; mGAPDH-R: 5'-CTCGTGGTTCACACCCATCA -3'.

### Example 16 In Vivo Evaluation of Specificity and Immunogenicity of Novel Artificial RNA Enzymes

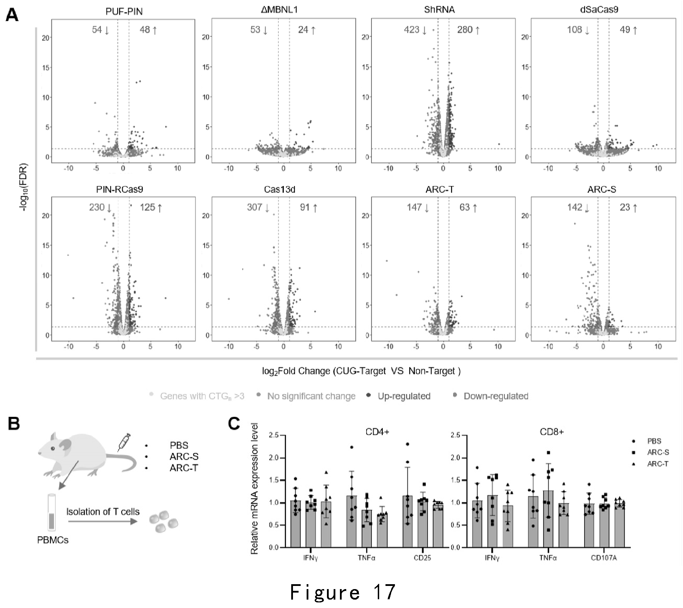

According to the above *in vivo* mouse injection method, different viral particles targeting CUG repeat sequences (purchased from OBiO TECH) were injected into the thigh skeletal muscle (right leg) of mouse-CUG-R160G14 mice (purchased from Cyagen Biosciences) at a total dose of 10¹¹ vg per mouse. Meanwhile, the corresponding control AAV viral particles (purchased from OBiO TECH) were injected into the left leg of the same mouse at the same dose (Figure 16, panel A). Two months after injection, the injected leg muscle was harvested, RNA was extracted as described above, and sequencing was performed. Data analysis results show that all CUG-targeting viral particles caused perturbations in gene expression levels in mouse muscle tissues. Among the different targeting systems, shRNA and Cas13d caused the largest number of upregulated and downregulated genes, followed by PIN-RCas9-CUG, dSaCas9, etc. The two novel artificial RNA enzymes ARC-S and ARC-T claimed in the present invention had minor effects on normal gene expression, showing therapeutic specificity among existing potential disease-targeting tools (Figure 17, panel A).

The novel artificial RNA enzymes claimed in the present application also exhibited safer properties in terms of immunogenicity. ARC-S and ARC-T viral particles (purchased from OBiO TECH) were injected into mice via tail vein at a dose of 10¹² vg. Two months after injection, CD4 and CD8 T cells were isolated from mouse peripheral blood, and common immune response marker genes were detected. Peripheral blood cell isolation was performed using the Dynabeads^{™} FlowComp^{™} Mouse CD4, CD8 Kit (Thermo) according to the manual (Figure 17, panel B). Total RNA was extracted from the isolated CD4 and CD8 cells using TRIzol Reagent (purchased from Thermo Fisher) according to the manual. Reverse transcription was performed using the PrimeScript RT Reagent Kit with gDNA Eraser (TAKARA) or other commercial reverse transcription kits. Subsequently, quantitative PCR was performed using 2× Universal SYBR Green Fast qPCR Mix reagents (purchased from ABclonal). The results show that the two artificial RNA enzymes in the present application did not induce immune responses in mice, thus demonstrating strong safety in therapeutic applications targeting repeat expansion RNA (Figure 17, panel C).

Primer sequences for quantitative PCR were as follows: mCD25-F: 5'-GCGTTGCTTAGGAAACTCCTGG -3'; mCD25-R: 5'-GCATAGACTGTGTTGGCTTCTGC -3'; mIFNG-F: 5'-CAGCAACAGCAAGGCGAAAAAGG -3'; mIFNG-R: 5'-TTTCCGCTTCCTGAGGCTGGAT -3'; mIL-17-F: 5'-CAGACTACCTCAACCGTTCCAC -3'; mIL-17-R: 5'-TCCAGCTTTCCCTCCGCATTGA -3'; mIL-4-F: 5'-ATCATCGGCATTTTGAACGAGGTC -3'; mIL-4-R: 5'-ACCTTGGAAGCCCTACAGACGA -3'; mLAMP1-F: 5'-CCAGGCTTTCAAGGTGGACAGT -3'; mLAMP1-R: 5'-GGTAGGCAATGAGGACGATGAG -3'; mTNFa-F: 5'-GGTGCCTATGTCTCAGCCTCTT -3'; mTNFa-R: 5'-GCCATAGAACTGATGAGAGGGAG -3'.

### Sequence information

SEQ ID NO: 1 PUF protein fragment 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLG
CMV promoter SEQ ID NO: 2 
GTGATGCGGTTTTGGCAGTACATCAATGGGCGTGGATAGCGGTTTGA
CTCACGGGGATTTCCAAGTCTCCACCCCATTGACGTCAATGGGAGTTTGT
TTTGGCACCAAAATCAACGGGACTTTCCAAAATGTCGTAACAACTCCGCC
CCATTGACGCAAATGGGCGGTAGGCGTGTACGGTGGGAGGTCTATATAA
GCAGAGCT
SEQ ID NO: 3 CAG promoter 
GCGTTACATAACTTACGGTAAATGGCCCGCCTGGCTGACCGCCCAAC
GACCCCCGCCCATTGACGTCAATAATGACGTATGTTCCCATAGTAACGCC
AATAGGGACTTTCCATTGACGTCAATGGGTGGAGTATTTACGGTAAACTG
CCCACTTGGCAGTACATCAAGTGTATCATATGCCAAGTACGCCCCCTATT
GACGTCAATGACGGTAAATGGCCCGCCTGGCATTATGCCCAGTACATGAC
CTTATGGGACTTTCCTACTTGGCAGTACATCTACGTATTAGTCATCGCTAT
TACCATGGTCGAGGTGAGCCCCACGTTCTGCTTCACTCTCCCCATCTCCCC
CCCCTCCCCACCCCCAATTTTGTATTTATTTATTTTTTAATTATTTTGTGCA
GCGATGGGGGCGGGGGGGGGGGGGGGGCGCGCGCCAGGCGGGGCGGGG
CGGGGCGAGGGGCGGGGCGGGGCGAGGCGGAGAGGTGCGGCGGCAGCC
AATCAGAGCGGCGCGCTCCGAAAGTTTCCTTTTATGGCGAGGCGGCGGCG
GCGGCGGCCCTATAAAAAGCGAAGCGCGCGGCGGGCG
SEQ ID NO: 4 PGK promoter 
GGGTAGGGGAGGCGCTTTTCCCAAGGCAGTCTGGAGCATGCGCTTTA
GCAGCCCCGCTGGGCACTTGGCGCTACACAAGTGGCCTCTGGCCTCGCAC
ACATTCCACATCCACCGGTAGGCGCCAACCGGCTCCGTTCTTTGGTGGCC
CCTTCGCGCCACCTTCTACTCCTCCCCTAGTCAGGAAGTTCCCCCCCGCCC
CGCAGCTCGCGTCGTGCAGGACGTGACAAATGGAAGTAGCACGTCTCACT
AGTCTCGTGCAGATGGACAGCACCGCTGAGCAATGGAAGCGGGTAGGCC
TTTGGGGCAGCGGCCAATAGCAGCTTTGCTCCTTCGCTTTCTGGGCTCAG
AGGCTGGGAAGGGGTGGGTCCGGGGGCGGGCTCAGGGGCGGGCTCAGGG
 
GCGGGGCGGGCGCCCGAAGGTCCTCCGGAGGCCCGGCATTCTGCACGCTT
CAAAAGCGCACGTCTGCCGCGCTGTTCTCCTCTTCCTCATCTCCGGGCCTT
TCG
SEQ ID NO: 5 EF-1α promoter 
GGCTCCGGTGCCCGTCAGTGGGCAGAGCGCACATCGCCCACAGTCCC
CGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTT
TCCCGAGGGTGGGGGAGAACCGTATATAAGTGCAGTAGTCGCCGTGAAC
GTTCTTTTTCGCAACGGGTTTGCCGCCAGAACACAGGTAAGTGCCGTGTG
TGGTTCCCGCGGGCCTGGCCTCTTTACGGGTTATGGCCCTTGCGTGCCTTG
AATTACTTCCACCTGGCTGCAGTACGTGATTCTTGATCCCGAGCTTCGGGT
TGGAAGTGGGTGGGAGAGTTCGAGGCCTTGCGCTTAAGGAGCCCCTTCGC
CTCGTGCTTGAGTTGAGGCCTGGCCTGGGCGCTGGGGCCGCCGCGTGCGA
ATCTGGTGGCACCTTCGCGCCTGTCTCGCTGCTTTCGATAAGTCTCTAGCC
ATTTAAAATTTTTGATGACCTGCTGCGACGCTTTTTTTCTGGCAAGATAGT
CTTGTAAATGCGGGCCAAGATCTGCACACTGGTATTTCGGTTTTTGGGGC
CGCGGGCGGCGACGGGGCCCGTGCGTCCCAGCGCACATGTTCGGCGAGG
CGGGGCCTGCGAGCGCGGCCACCGAGAATCGGACGGGGGTAGTCTCAAG
CTGGCCGGCCTGCTCTGGTGCCTGGCCTCGCGCCGCCGTGTATCGCCCCG
CCCTGGGCGGCAAGGCTGGCCCGGTCGGCACCAGTTGCGTGAGCGGAAA
GATGGCCGCTTCCCGGCCCTGCTGCAGGGAGCTCAAAATGGAGGACGCG
GCGCTCGGGAGAGCGGGCGGGTGAGTCACCCACACAAAGGAAAAGGGCC
TTTCCGTCCTCAGCCGTCGCTTCATGTGACTCCACGGAGTACCGGGCGCC
GTCCAGGCACCTCGATTAGTTCTCGAGCTTTTGGAGTACGTCGTCTTTAGG
TTGGGGGGAGGGGTTTTATGCGATGGAGTTTCCCCACACTGAGTGGGTGG
AGACTGAAGTTAGGCCAGCTTGGCACTTGATGTAATTCTCCTTGGAATTT
GCCCTTTTTGAGTTTGGATCTTGGTTCATTCTCAAGCCTCAGACAGTGGTT
CAAAGTTTTTTTCTTCCATTTCAGGTGTCGTGA
SEQ ID NO: 6 SV40 promoter 
GGTGTGGAAAGTCCCCAGGCTCCCCAGCAGGCAGAAGTATGCAAAG
CATGCATCTCAATTAGTCAGCAACCAGGTGTGGAAAGTCCCCAGGCTCCC
CAGCAGGCAGAAGTATGCAAAGCATGCATCTCAATTAGTCAGCAACCAT
AGTCCCGCCCCTAACTCCGCCCATCCCGCCCCTAACTCCGCCCAGTTCCGC
CCATTCTCCGCCCCATGGCTGACTAATTTTTTTTATTTATGCAGAGGCCGA
GGCCGCCTCGGCCTCTGAGCTATTCCAGAAGTAGTGAGGAGGCTTTTTTG
GAGGCCTAGGCTTTTGCAAA
SEQ ID NO: 7 chicken beta-actin promoter 
TCGAGGTGAGCCCCACGTTCTGCTTCACTCTCCCCATCTCCCCCCCCT
CCCCACCCCCAATTTTGTATTTATTTATTTTTTAATTATTTTGTGCAGCGAT
GGGGGCGGGGGGGGGGGGGGGGCGCGCGCCAGGCGGGGCGGGGCGGGG
CGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGC
GGCCCTATAAAAAGCGAAGCGCGCGGCGGGCG
SEQ ID NO: 8 HSV-TK promoter 
AAATGAGTCTTCGGACCTCGCGGGGGCCGCTTAAGCGGTGGTTAGGG
TTTGTCTGACGCGGGGGGAGGGGGAAGGAACGAAACACTCTCATTCGGA
GGCGGCTCGGGGTTTGGTCTTGGTGGCCACGGGCACGCAGAAGAGCGCC
 
GCGATCCTCTTAAGCACCCCCCCGCCCTCCGTGGAGGCGGGGGTTTGGTC
GGCGGGTGGTAACTGGCGGGCCGCTGACTCGGGCGGGTCGCGCGCCCCA
GAGTGTGACCTTTTCGGTCTGCTCGCAGACCCCCGGGCGGCGCCGCCGCG
GCGGCGACGGGCTCGCTGGGTCCTAGGCTCCATGGGGACCGTATACGTGG
ACAGGCTCTGGAGCATCCGCACGACTGCGGTGATATTACCGGAGACCTTC
TGCGGGACGAGCCGGGTCACGCGGCTGACGCGGAGCGTCCGTTGGGCGA
CAAACACCAGGACGGGGCACAGGTACACTATCTTGTCACCCGGAGGCGC
GAGGGACTGCAGGAGCTTCAGGGAGTGGCGCAGCTGCTTCATCCCCGTGG
CCCGTTGCTCGCGTTTGCTGGCGGTGTCCCCGGAAGAAATATATTTGCAT
GTCTTTAGTTCTATGATGACACAAACCCCGCCCAGCGTCTTGTCATTGGCG
AATTCGAACACGCAGATGCAGTCGGGGCGGCGCGGTCCCAGGTCCACTTC
GCATATTAAGGTGACGCGTGTGGCCTCGAACACCGAGCGACCCTGCAGCG
ACCCGCTTAA
SEQ ID NO: 9 Ubc promoter 
GGCCTCCGCGCCGGGTTTTGGCGCCTCCCGCGGGCGCCCCCCTCCTC
ACGGCGAGCGCTGCCACGTCAGACGAAGGGCGCAGGAGCGTCCTGATCC
TTCCGCCCGGACGCTCAGGACAGCGGCCCGCTGCTCATAAGACTCGGCCT
TAGAACCCCAGTATCAGCAGAAGGACATTTTAGGACGGGACTTGGGTGA
CTCTAGGGCACTGGTTTTCTTTCCAGAGAGCGGAACAGGCGAGGAAAAGT
AGTCCCTTCTCGGCGATTCTGCGGAGGGATCTCCGTGGGGCGGTGAACGC
CGATGATTATATAAGGACGCGCCGGGTGTGGCACAGCTAGTTCCGTCGCA
GCCGGGATTTGGGTCGCGGTTCTTGTTTGTGGATCGCTGTGATCGTCACTT
GGTGAGTAGCGGGCTGCTGGGCTGGCCGGGGCTTTCGTGGCCGCCGGGCC
GCTCGGTGGGACGGAAGCGTGTGGAGAGACCGCCAAGGGCTGTAGTCTG
GGTCCGCGAGCAAGGTTGCCCTGAACTGGGGGTTGGGGGGAGCGCAGCA
AAATGGCGGCTGTTCCCGAGTCTTGAATGGAAGACGCTTGTGAGGCGGGC
TGTGAGGTCGTTGAAACAAGGTGGGGGGCATGGTGGGCGGCAAGAACCC
AAGGTCTTGAGGCCTTCGCTAATGCGGGAAAGCTCTTATTCGGGTGAGAT
GGGCTGGGGCACCATCTGGGGACCCTGACGTGAAGTTTGTCACTGACTGG
AGAACTCGGTTTGTCGTCTGTTGCGGGGGCGGCAGTTATGCGGTGCCGTT
GGGCAGTGCACCCGTACCTTTGGGAGCGCGCGCCCTCGTCGTGTCGTGAC
GTCACCCGTTCTGTTGGCTTATAATGCAGGGTGGGGCCACCTGCCGGTAG
GTGTGCGGTAGGCTTTTCTCCGTCGCAGGACGCAGGGTTCGGGCCTAGGG
TAGGCTCTCCTGAATCGACAGGCGCCGGACCTCTGGTGAGGGGAGGGAT
AAGTGAGGCGTCAGTTTCTTTGGTCGGTTTTATGTACCTATCTTCTTAAGT
AGCTGAAGCTCCGGTTTTGAACTATGCGCTCGGGGTTGGCGAGTGTGTTT
TGTGAAGTTTTTTAGGCACCTTTTGAAATGTAATCATTTGGGTCAATATGT
AATTTTCAGTGTTAGACTAGTAAATTGTCCGCTAAATTCTGGCCGTTTTTG
GCTTTTTTGTTAGAC
SEQ ID NO: 10 TRE promoter 
GAGTTTACTCCCTATCAGTGATAGAGAACGTATGTCGAGTTTACTCCC
TATCAGTGATAGAGAACGATGTCGAGTTTACTCCCTATCAGTGATAGAGA
ACGTATGTCGAGTTTACTCCCTATCAGTGATAGAGAACGTATGTCGAGTT
TACTCCCTATCAGTGATAGAGAACGTATGTCGAGTTTATCCCTATCAGTG
ATAGAGAACGTATGTCGAGTTTACTCCCTATCAGTGATAGAGAACGTATG
TCGAGGTAGGCGTGTACGGTGGGAGGCCTATATAAGCAGAGCTCGTTTAG
TGAACCGTCAGATCGCC
SEQ ID NO: 11 UAS promoter 
CGGAGTACTGTCCTCCGAGCGGAGTACTGTCCTCCGACTCGAGCGGA
GTACTGTCCTCCGATCGGAGTACTGTCCTCCGCGAATTCCGGAGTACTGT
CCTCCG
SEQ ID NO: 12 Ad promoter
   GGGGGGCTATAAAAGGGGGTGGGGGCGTTCGTCCTCACTCT
SEQ ID NO: 13 Ac5 promoter 
CATGAATGGCATCAACTCTGAATCAAATCTTTGCAGATGCACCTACT
TCTCATTTCCACTGTCACATCATTTTTCCAGATCTCGCTGCCTGTTATGTG
GCCCACAAACCAAGACACGTTTTATGGCCATTAAAGCTGGCTGATCGTCG
CCAAACACCAAATACATATCAATATGTACATTCGAGAAAGAAGCGATCA
AAGAAGCGTCTTCGGGCGAGTAGGAGAATGCGGAGGAGAAGGAGAACG
AGCTGATCTAGTATCTCTCCACAATCCAATGCCAACTGACCAACTGGCCA
TATTCGGAGCAATTTGAAGCCAATTTCCATCGCCTGGCGATCGCTCCATTC
TTGGCTATATGTTTTTCACCGTTCCCGGGGCCATTTTCAAAGACTCGTCGG
TAAGATAAGATTGTGTCACTCGCTGTCTCTCTTCATTTGTCGAAGAATGCT
GAGGAATTTCGCGATGACGTCGGCGAGTATTTTGAAGAATGAGAATAATT
TGTATTTATACGAAAATCAGTTAGTGGAATTTTCTACAAAAACATGTTAT
CTATAGATAATTTTGTTGCAAAATATGTTGACTATGACAAAGATTGTATG
TATATACCTTTAATGTATTCTCATTTTCTTATGTATTTATAATGGCAATGAT
GATACTGATGATATTTTAAGATGATGCCAGACCACAGGCTGATTTCTGCG
TCTTTTGCCGAACGCAGTGCATGTGCGGTTGTTGTTTTTTGGAATAGTTTC
AATTTTCGGACTGTCCGCTTTGATTTCAGTTTCTTGGCTTATTCAAAAAGC
AAAGTAAAGCCAAAAAAGCGAGATGGCAATACCAAATGCGGCAAAACG
GTAGTGGAAGGAAAGGGGTGCGGGGCAGCGGAAGGAAGGGTGGGGCGG
GGCGTGGCGGGGTCTGTGGCTGGGCGCGACGTCACCGACGTTGGAGCCA
CTCCTTTGACCATGTGTGCGTGTGTGTATTATTCGTGTCTCGCCACTCGCC
GGTTGTTTTTTTCTTTTTATCTCGCTCTCTCTAGCGCCATCTCGTACGCATG
CTCAACGCACCGCATGTTGCCGTGTCCTTTATGCGTCATTTTGGCTCGAAA
TAGGCAATTATTTAAACAAAGATTAGTCAACGAAAACGCTAAAATAAAT
AAGTCTACAATATGGTTACTTATTGCCATGTGTGTGCAGCCAACGATAGC
AACAAAAGCAACAACACAGTGGCTTTCCCTCTTTCACTTTTTGTTTGCAAG
CGCGTGCGAGCAAGACGGCACGACCGGCAAACGCAATTACGCTGACAAA
GAGCAGACGAAGTTTTGGCCGAAAAACATCAAGGCGCCTGATACGAATG
CATTTGCAATAACAATTGCGATATTTAATATTGTTTATGAAGCTGTTTGAC
TTCAAAACACACAAAAAAAAAAATAAAACAAATTATTTGAAAGAGAATT
AGGAATCGGACAGCTTATCGTTACGGGCTAACAGCACACCGAGACGAAA
TAGCTTACCTGACGTCACAGCCTCTGGAAGAACTGCCGCCAAGCAGAGAG
AGAGAGAAAAAGAGGGAGAGCAGCTTAGACCGCATGTGCTTGTGTGTGA
GGCGTCTCTCTCTTCGTCTCCTGTTTGCGCAAACGCATAGACTGCACTGAG
AAAATCGATTACCTATTTTTTATGAATGAATATTTGCACTATTACTATTCA
AAACTATTAAGATAGCAATCACATTCAATAGCCAAATACTATACCACCTG
AGCGATGCAACGAAATGATCAATTTGAGCAAAAATGCTGCATATTTAGGA
CGGCATCATTATAGAAATGCTTCTTGCTGTGTACTTTTCTCTCGTCTGGCA
GCTGTTTCGCCGTTATTGTTAAAACCGGCTTAAGTTAGGTGTGTTTTCTAC
GACTAGTGATGCCCCTACTAGAAGATGTGTGTTGCACAAATGTCCCTGAA
TAACCAATTTGAAGTGCAGATAGCAGTAAACGTAAGCTAATATGAATATT
 
ATTTAACTGTAATGTTTTAATATCGCTGGACATTACTAATAAACCCACTAT
AAACACATGTACATATGTATGTTTTGGCATACAATGAGTAGTTGGGGAAA
AAATGTGTAAAAGCACCGTGACCATCACAGCATAAAGATAACCAGCTGA
AGTATCGAATATGAGTAACCCCCAAATTGAATCACATGCCGCAACTGATA
GGACCCATGGAAGTACACTCTTCATGGCGATATACAAGACACACACAAG
CACGAACACCCAGTTGCGGAGGAAATTCTCCGTAAATGAAAACCCAATC
GGCGAACAATTCATACCCATATATGGTAAAAGTTTTGAACGCGACTTGAG
AGCGGAGAGCATTGCGGCTGATAAGGTTTTAGCGCTAAGCGGGCTTTATA
AAACGGGCTGCGGGACCAGTTTTCATATCACTACCGTTTGAGTTCTTGTG
CTGTGTGGATACTCCTCCCGACACAAAGCCGCTCCATCAGCCAGCAGTCG
TCTAATCCAGAGAC
SEQ ID NO: 14 Polyhedrin promoter 
ATCATGGAGATAATTAAAATGATAACCATCTCGCAAATAAATAAGTA
TTTTACTGTTTTCGTAACAGTTTTGTAATAAAAAAACCTATAAAT
SEQ ID NO: 15 p10 promoter 
GACCTTTAATTCAACCCAACACAATATATTATAGTTAAATAAGAATT
ATTATCAAATCATTTGTATATTAATTAAAATACTATACTGTAAATTACATT
TTATTTACAATC
SEQ ID NO: 16 CaMKIIa promoter 
CATTATGGCCTTAGGTCACTTCATCTCCATGGGGTTCTTCTTCTGATT
TTCTAGAAAATGAGATGGGGGTGCAGAGAGCTTCCTCAGTGACCTGCCCA
GGGTCACATCAGAAATGTCAGAGCTAGAACTTGAACTCAGATTACTAATC
TTAAATTCCATGCCTTGGGGGCATGCAAGTACGATATACAGAAGGAGTGA
ACTCATTAGGGCAGATGACCAATGAGTTTAGGAAAGAAGAGTCCAGGGC
AGGGTACATCTACACCACCCGCCCAGCCCTGGGTGAGTCCAGCCACGTTC
ACCTCATTATAGTTGCCTCTCTCCAGTCCTACCTTGACGGGAAGCACAAG
CAGAAACTGGGACAGGAGCCCCAGGAGACCAAATCTTCATGGTCCCTCTG
GGAGGATGGGTGGGGAGAGCTGTGGCAGAGGCCTCAGGAGGGGCCCTGC
TGCTCAGTGGTGACAGATAGGGGTGAGAAAGCAGACAGAGTCATTCCGT
CAGCATTCTGGGTCTGTTTGGTACTTCTTCTCACGCTAAGGTGGCGGTGTG
ATATGCACAATGGCTAAAAAGCAGGGAGAGCTGGAAAGAAACAAGGAC
AGAGACAGAGGCCAAGTCAACCAGACCAATTCCCAGAGGAAGCAAAGAA
ACCATTACAGAGACTACAAGGGGGAAGGGAAGGAGAGATGAATTAGCTT
CCCCTGTAAACCTTAGAACCCAGCTGTTGCCAGGGCAACGGGGCAATACC
TGTCTCTTCAGAGGAGATGAAGTTGCCAGGGTAACTACATCCTGTCTTTCT
CAAGGACCATCCCAGAATGTGGCACCCACTAGCCGTTACCATAGCAACTG
CCTCTTTGCCCCACTTAATCCCATCCCGTCTGTTAAAAGGGCCCTATAGTT
GGAGGTGGGGGAGGTAGGAAGAGCGATGATCACTTGTGGACTAAGTTTG
TTCGCATCCCCTTCTCCAACCCCCTCAGTACATCACCCTGGGGGAACAGG
GTCCACTTGCTCCTGGGCCCACACAGTCCTGCAGTATTGTGTATATAAGG
CCAGGGCAAAGAGGAGCAGGTTTTAAAGTGAAAGGCAGGCAGGTGTTGG
GGAGGCAGTTACCGGGGCAACGGGAACAGGGCGTTTCGGAGGTGGTTGC
CATGGGGACCTGGATGCTGACGAAGGCTCGCGAGGCTGTGAGCAGCCAC
AGTGCCCTGCTCAGAAGCCCCAAGCTCGTCAGTCAAGCCGGTTCTCCGTT
TGCACTCAGGAGCACGGGCAGGCGAGTGGCCCCTAGTTCTGGGGGCAGC
SEQ ID NO: 17 IL-2 promoter CATTTTGACACCCCCATAATATTTTTCCAGAATTAACAGTATAAATTG 
CATCTCTTGTTCAAGAGTTCCCTATCACTCTCTTTAATCACTACTCACAGT
AACCTCAACTCCTGC
SEQ ID NO: 18 RSV promoter 
AATGTAGTCTTATGCAATACTCTTGTAGTCTTGCAACATGGTAACGAT
GAGTTAGCAACATGCCTTACAAGGAGAGAAAAAGCACCGTGCATGCCGA
TTGGTGGAAGTAAGGTGGTACGATCGTGCCTTATTAGGAAGGCAACAGAC
GGGTCTGACATGGATTGGACGAACCACTGAATTGCCGCATTGCAGAGATA
TTGTATTTAAGTGCCTAGCTCGATACATAAAC
SEQ ID NO: 19 Gall promoter 
CGGATTAGAAGCCGCCGAGCGGGTGACAGCCCTCCGAAGGAAGACT
CTCCTCCGTGCGTCCTCGTCTTCACCGGTCGCGTTCCTGAAACGCAGATGT
GCCTCGCGCCGCACTGCTCCGAACAATAAAGATTCTACAATACTAGCTTT
TATGGTTATGAAGAGGAAAAATTGGCAGTAACCTGGCCCCACAAACCTTC
AAATGAACGAATCAAATTAACAACCATAGGATGATAATGCGATTAGTTTT
TTAGCCTTATTTCTGGGGTAATTAATCAGCGAAGCGATGATTTTTGATCTA
TTAACAGATATATAAATGCAAAAACTGCATAACCACTTTAACTAATACTT
TCAACATTTTCGGTTTGTATTACTTCTTATTCAAATGTAATAAAAGTATCA
ACAAAAAATTGTTAATATACCTCTATACTTTAACGTCAAGGAG
SEQ ID NO: 20 TEF1 promoter 
AATGTTTCTACTCCTTTTTTACTCTTCCAGATTTTCTCGGACTCCGCGC
ATCGCCGTACCACTTCAAAACACCCAAGCACAGCATACTAAATTTTCCCT
CTTTCTTCCTCTAGGGTGTCGTTAATTACCCGTACTAAAGGTTTGGAAAAG
AAAAAAGAGACCGCCTCGTTTCTTTTTCTTCGTCGAAAAAGGCAATAAAA
ATTTTTATCACGTTTCTTTTTCTTGAAATTTTTTTTTTTAGTTTTTTTCTCTT
TCAGTGACCTCCATTGATATTTAAGTTAATAAACGGTCTTCAATTTCTCAA
GTTTCAGTTTCATTTTTCTTGTTCTATTACAACTTTTTTTACTTCTTGTTCAT
TAGAAAGAAAGCATAGCAATCTAATCTAAG
SEQ ID NO: 21 TDH3 promoter 
TCATTATCAATACTGCCATTTCAAAGAATACGTAAATAATTAATAGT
AGTGATTTTCCTAACTTTATTTAGTCAAAAAATTAGCCTTTTAATTCTGCT
GTAACCCGTACATGCCCAAAATAGGGGGCGGGTTACACAGAATATATAA
CATCGTAGGTGTCTGGGTGAACAGTTTATTCCTGGCATCCACTAAATATA
ATGGAGCCCGCTTTTTAAGCTGGCATCCAGAAAAAAAAAGAATCCCAGC
ACCAAAATATTGTTTTCTTCACCAACCATCAGTTCATAGGTCCATTCTCTT
AGCGCAACTACAGAGAACAGGGGCACAAACAGGCAAAAAACGGGCACA
ACCTCAATGGAGTGATGCAACCTGCCTGGAGTAAATGATGACACAAGGC
AATTGACCCACGCATGTATCTATCTCATTTTCTTACACCTTCTATTACCTTC
TGCTCTCTCTGATTTGGAAAAAGCTGAAAAAAAAGGTTGAAACCAGTTCC
CTGAAATTATTCCCCTACTTGACTAATAAGTATATAAAGACGGTAGGTAT
TGATTGTAATTCTGTAAATCTATTTCTTAAACTTCTTAAATTCTACTTTTAT
AGTTAGTCTTTTTTTTAGTTTTAAAACACCAAGAACTTAGTTTCGAATAAA
CACACATAAACAAACAAA
SEQ ID NO: 22 ADH1 promoter 
ATCCTTTTGTTGTTTCCGGGTGTACAATATGGACTTCCTCTTTTCTGGC
AACCAAACCCATACATCGGGATTCCTATAATACCTTCGTTGGTCTCCCTA
ACATGTAGGTGGCGGAGGGGAGATATACAATAGAACAGATACCAGACAA
GACATAATGGGCTAAACAAGACTACACCAATTACACTGCCTCATTGATGG
 
TGGTACATAACGAACTAATACTGTAGCCCTAGACTTGATAGCCATCATCA
TATCGAAGTTTCACTACCCTTTTTCCATTTGCCATCTATTGAAGTAATAAT
AGGCGCATGCAACTTCTTTTCTTTTTTTTTCTTTTCTCTCTCCCCCGTTGTT
GTCTCACCATATCCGCAATGACAAAAAAATGATGGAAGACACTAAAGGA
AAAAATTAACGACAAAGACAGCACCAACAGATGTCGTTGTTCCAGAGCT
GATGAGGGGTATCTCGAAGCACACGAAACTTTTTCCTTCCTTCATTCACG
CACACTACTCTCTAATGAGCAACGGTATACGGCCTTCCTTCCAGTTACTTG
AATTTGAAATAAAAAAAAGTTTGCTGTCTTGCTATCAAGTATAAATAGAC
CTGCAATTATTAATCTTTTGTTTCCTCGTCATTGTTCTCGTTCCCTTTCTTC
CTTGTTTCTTTTTCTGCACAATATTTCAAGCTATACCAAGCATACAATCAA
CT
SEQ ID NO: 23 T7 promoter TAATACGACTCACTATAGG
SEQ ID NO: 24 T3 promoter AATTAACCCTCACTAAAGG
SEQ ID NO: 25 SP6 promoter ATTTAGGTGACACTATAGA
SEQ ID NO: 26 araBAD promoter 
AAAGCCATGACAAAAACGCGTAACAAAAGTGTCTATAATCACGGCA
GAAAAGTCCACATTGATTATTTGCACGGCGTCACACTTTGCTATGCCATA
GCATTTTTATCCATAAGATTAGCGGATCCTACCTGACGCTTTTTATCGCAA
CTCTCTACTGTTTCTCCAT
SEQ ID NO: 27 trp promoter 
GTACAATCTTGATCCGGAGCTTTTCTTTTTTTGCCGATTAAGAATTAA
TTCGGTCGAAAAAAGAAAAGGAGAGGGCCAAGAGGGAGGGCATTGGTG
ACTATTGAGCACGTGAGTATACGTGATTAAGCACACAAAGGCAGCTTGGA
GT
SEQ ID NO: 28 tac promoter TTGACAATTAATCATCGGCTCGTATAATG
SEQ ID NO: 29 lac promoter
   TTTACACTTTATGCTTCCGGCTCGTATGTTG
SEQ ID NO: 30 pL promoter 
TCTCACCTACCAAACAATGCCCCCCTGCAAAAAATAAATTCATATAA
AAAACATACAGATAACCATCTGCGGTGATAAATTATCTCTGGCGGTGTTG
ACATAAATACCACTGGCGGTGATACTGAGCACATCAGCAGGACGCACTG
ACCACCATGAAGGTGACGCTCTTAAAAATTAAGCCCTGAAGAAGGGCAG
CATTCAAAGCAGAAGGCTTTGGGGTGTGTGATACGAAACGAAGCATTGG
SEQ ID NO: 31 Nucleic acid sequence encoding β-galactosidase LacZ 
ATGACCATGATTACGGATTCACTGGCCGTCGTTTTACAACGTCGTGA
CTGGGAAAACCCTGGCGTTACCCAACTTAATCGCCTTGCAGCACATCCCC
CTTTCGCCAGCTGGCGTAATAGCGAAGAGGCCCGCACCGATCGCCCTTCC
CAACAGTTGCGCAGCCTGAATGGCGAATGGCGCTTTGCCTGGTTTCCGGC
ACCAGAAGCGGTGCCGGAAAGCTGGCTGGAGTGCGATCTTCCTGAGGCC
GATACTGTCGTCGTCCCCTCAAACTGGCAGATGCACGGTTACGATGCGCC
CATCTACACCAACGTAACCTATCCCATTACGGTCAATCCGCCGTTTGTTCC
CACGGAGAATCCGACGGGTTGTTACTCGCTCACATTTAATGTTGATGAAA
GCTGGCTACAGGAAGGCCAGACGCGAATTATTTTTGATGGCGTTAACTCG
GCGTTTCATCTGTGGTGCAACGGGCGCTGGGTCGGTTACGGCCAGGACAG
TCGTTTGCCGTCTGAATTTGACCTGAGCGCATTTTTACGCGCCGGAGAAA
ACCGCCTCGCGGTGATGGTGCTGCGTTGGAGTGACGGCAGTTATCTGGAA
GATCAGGATATGTGGCGGATGAGCGGCATTTTCCGTGACGTCTCGTTGCT
 
GCATAAACCGACTACACAAATCAGCGATTTCCATGTTGCCACTCGCTTTA
ATGATGATTTCAGCCGCGCTGTACTGGAGGCTGAAGTTCAGATGTGCGGC
GAGTTGCGTGACTACCTACGGGTAACAGTTTCTTTATGGCAGGGTGAAAC
GCAGGTCGCCAGCGGCACCGCGCCTTTCGGCGGTGAAATTATCGATGAGC
GTGGTGGTTATGCCGATCGCGTCACACTACGTCTGAACGTCGAAAACCCG
AAACTGTGGAGCGCCGAAATCCCGAATCTCTATCGTGCGGTGGTTGAACT
GCACACCGCCGACGGCACGCTGATTGAAGCAGAAGCCTGCGATGTCGGTT
TCCGCGAGGTGCGGATTGAAAATGGTCTGCTGCTGCTGAACGGCAAGCCG
TTGCTGATTCGAGGCGTTAACCGTCACGAGCATCATCCTCTGCATGGTCA
GGTCATGGATGAGCAGACGATGGTGCAGGATATCCTGCTGATGAAGCAG
AACAACTTTAACGCCGTGCGCTGTTCGCATTATCCGAACCATCCGCTGTG
GTACACGCTGTGCGACCGCTACGGCCTGTATGTGGTGGATGAAGCCAATA
TTGAAACCCACGGCATGGTGCCAATGAATCGTCTGACCGATGATCCGCGC
TGGCTACCGGCGATGAGCGAACGCGTAACGCGAATGGTGCAGCGCGATC
GTAATCACCCGAGTGTGATCATCTGGTCGCTGGGGAATGAATCAGGCCAC
GGCGCTAATCACGACGCGCTGTATCGCTGGATCAAATCTGTCGATCCTTC
CCGCCCGGTGCAGTATGAAGGCGGCGGAGCCGACACCACGGCCACCGAT
ATTATTTGCCCGATGTACGCGCGCGTGGATGAAGACCAGCCCTTCCCGGC
TGTGCCGAAATGGTCCATCAAAAAATGGCTTTCGCTACCTGGAGAGACGC
GCCCGCTGATCCTTTGCGAATACGCCCACGCGATGGGTAACAGTCTTGGC
GGTTTCGCTAAATACTGGCAGGCGTTTCGTCAGTATCCCCGTTTACAGGG
CGGCTTCGTCTGGGACTGGGTGGATCAGTCGCTGATTAAATATGATGAAA
ACGGCAACCCGTGGTCGGCTTACGGCGGTGATTTTGGCGATACGCCGAAC
GATCGCCAGTTCTGTATGAACGGTCTGGTCTTTGCCGACCGCACGCCGCA
TCCAGCGCTGACGGAAGCAAAACACCAGCAGCAGTTTTTCCAGTTCCGTT
TATCCGGGCAAACCATCGAAGTGACCAGCGAATACCTGTTCCGTCATAGC
GATAACGAGCTCCTGCACTGGATGGTGGCGCTGGATGGTAAGCCGCTGGC
AAGCGGTGAAGTGCCTCTGGATGTCGCTCCACAAGGTAAACAGTTGATTG
AACTGCCTGAACTACCGCAGCCGGAGAGCGCCGGGCAACTCTGGCTCAC
AGTACGCGTAGTGCAACCGAACGCGACCGCATGGTCAGAAGCCGGGCAC
ATCAGCGCCTGGCAGCAGTGGCGTCTGGCGGAAAACCTCAGTGTGACGCT
CCCCGCCGCGTCCCACGCCATCCCGCATCTGACCACCAGCGAAATGGATT
TTTGCATCGAGCTGGGTAATAAGCGTTGGCAATTTAACCGCCAGTCAGGC
TTTCTTTCACAGATGTGGATTGGCGATAAAAAACAACTGCTGACGCCGCT
GCGCGATCAGTTCACCCGTGCACCGCTGGATAACGACATTGGCGTAAGTG
AAGCGACCCGCATTGACCCTAACGCCTGGGTCGAACGCTGGAAGGCGGC
GGGCCATTACCAGGCCGAAGCAGCGTTGTTGCAGTGCACGGCAGATACA
CTTGCTGATGCGGTGCTGATTACGACCGCTCACGCGTGGCAGCATCAGGG
GAAAACCTTATTTATCAGCCGGAAAACCTACCGGATTGATGGTAGTGGTC
AAATGGCGATTACCGTTGATGTTGAAGTGGCGAGCGATACACCGCATCCG
GCGCGGATTGGCCTGAACTGCCAGCTGGCGCAGGTAGCAGAGCGGGTAA
ACTGGCTCGGATTAGGGCCGCAAGAAAACTATCCCGACCGCCTTACTGCC
GCCTGTTTTGACCGCTGGGATCTGCCATTGTCAGACATGTATACCCCGTAC
GTCTTCCCGAGCGAAAACGGTCTGCGCTGCGGGACGCGCGAATTGAATTA
TGGCCCACACCAGTGGCGCGGCGACTTCCAGTTCAACATCAGCCGCTACA
GTCAACAGCAACTGATGGAAACCAGCCATCGCCATCTGCTGCACGCGGA
AGAAGGCACATGGCTGAATATCGACGGTTTCCATATGGGGATTGGTGGCG
 
ACGACTCCTGGAGCCCGTCAGTATCGGCGGAATTCCAGCTGAGCGCCGGT
CGCTACCATTACCAGTTGGTCTGGTGTCAAAAATAA
SEQ ID NO: 32 Protein sequence of β-galactosidase LacZ 
MTMITDSLAVVLQRRDWENPGVTQLNRLAAHPPFASWRNSEEARTDRP
SQQLRSLNGEWRFAWFPAPEAVPESWLECDLPEADTVVVPSNWQMHGYDA
PIYTNVTYPITVNPPFVPTENPTGCYSLTFNVDESWLQEGQTRIIFDGVNSAFH
LWCNGRWVGYGQDSRLPSEFDLSAFLRAGENRLAVMVLRWSDGSYLEDQD
MWRMSGIFRDVSLLHKPTTQISDFHVATRFNDDFSRAVLEAEVQMCGELRD
YLRVTVSLWQGETQVASGTAPFGGEIIDERGGYADRVTLRLNVENPKLWSA
EIPNLYRAVVELHTADGTLIEAEACDVGFREVRIENGLLLLNGKPLLIRGVNR
HEHHPLHGQVMDEQTMVQDILLMKQNNFNAVRCSHYPNHPLWYTLCDRYG
LYVVDEANIETHGMVPMNRLTDDPRWLPAMSERVTRMVQRDRNHPSVIIWS
LGNESGHGANHDALYRWIKSVDPSRPVQYEGGGADTTATDIICPMYARVDE
DQPFPAVPKWSIKKWLSLPGETRPLILCEYAHAMGNSLGGFAKYWQAFRQY
PRLQGGFVWDWVDQSLIKYDENGNPWSAYGGDFGDTPNDRQFCMNGLVFA
DRTPHPALTEAKHQQQFFQFRLSGQTIEVTSEYLFRHSDNELLHWMVALDGK
PLASGEVPLDVAPQGKQLIELPELPQPESAGQLWLTVRVVQPNATAWSEAGH
ISAWQQWRLAENLSVTLPAASHAIPHLTTSEMDFCIELGNKRWQFNRQSGFL
SQMWIGDKKQLLTPLRDQFTRAPLDNDIGVSEATRIDPNAWVERWKAAGHY
QAEAALLQCTADTLADAVLITTAHAWQHQGKTLFISRKTYRIDGSGQMAITV
DVEVASDTPHPARIGLNCQLAQVAERVNWLGLGPQENYPDRLTAACFDRW
DLPLSDMYTPYVFPSENGLRCGTRELNYGPHQWRGDFQFNISRYSQQQLME
TSHRHLLHAEEGTWLNIDGFHMGIGGDDSWSPSVSAEFQLSAGRYHYQLVW
CQK*
SEQ ID NO: 33 Nucleic acid sequence encoding mCherry 
ATGGTGAGCAAGGGCGAGGAGGATAACATGGCCATCATCAAGGAGT
TCATGCGCTTCAAGGTGCACATGGAGGGCTCCGTGAACGGCCACGAGTTC
GAGATCGAGGGCGAGGGCGAGGGCCGCCCCTACGAGGGCACCCAGACCG
CCAAGCTGAAGGTGACCAAGGGTGGCCCCCTGCCCTTCGCCTGGGACATC
CTGTCCCCTCAGTTCATGTACGGCTCCAAGGCCTACGTGAAGCACCCCGC
CGACATCCCCGACTACTTGAAGCTGTCCTTCCCCGAGGGCTTCAAGTGGG
AGCGCGTGATGAACTTCGAGGACGGCGGCGTGGTGACCGTGACCCAGGA
CTCCTCCCTGCAGGACGGCGAGTTCATCTACAAGGTGAAGCTGCGCGGCA
CCAACTTCCCCTCCGACGGCCCCGTAATGCAGAAGAAGACCATGGGCTGG
GAGGCCTCCTCCGAGCGGATGTACCCCGAGGACGGCGCCCTGAAGGGCG
AGATCAAGCAGAGGCTGAAGCTGAAGGACGGCGGCCACTACGACGCTGA
GGTCAAGACCACCTACAAGGCCAAGAAGCCCGTGCAGCTGCCCGGCGCC
TACAACGTCAACATCAAGTTGGACATCACCTCCCACAACGAGGACTACAC
CATCGTGGAACAGTACGAACGCGCCGAGGGCCGCCACTCCACCGGCGGC
ATGGACGAGCTGTACAAGTAG
SEQ ID NO: 34 mCherry protein sequence 
MVSKGEEDNMAIIKEFMRFKVHMEGSVNGHEFEIEGEGEGRPYEGTQT
AKLKVTKGGPLPFAWDILSPQFMYGSKAYVKHPADIPDYLKLSFPEGFKWER
VMNFEDGGVVTVTQDSSLQDGEFIYKVKLRGTNFPSDGPVMQKKTMGWEA
SSERMYPEDGALKGEIKQRLKLKDGGHYDAEVKTTYKAKKPVQLPGAYNV
NIKLDITSHNEDYTIVEQYERAEGRHSTGGMDELYK*
SEQ ID NO: 35 Nucleic acid sequence encoding GFP 
ATGAGTAAAGGAGAAGAACTTTTCACTGGAGTTGTCCCAATTCTTGT
TGAATTAGATGGTGATGTTAATGGGCACAAATTTTCTGTCAGTGGAGAGG
GTGAAGGTGATGCAACATACGGAAAACTTACCCTTAAATTTATTTGCACT
ACTGGAAAACTACCTGTTCCATGGCCAACACTTGTCACTACTTTCTCTTAT
GGTGTTCAATGCTTTTCAAGATACCCAGATCATATGAAACGGCATGACTT
TTTCAAGAGTGCCATGCCCGAAGGTTATGTACAGGAAAGAACTATATTTT
TCAAAGATGACGGGAACTACAAGACACGTGCTGAAGTCAAGTTTGAAGG
TGATACCCTTGTTAATAGAATCGAGTTAAAAGGTATTGATTTTAAAGAAG
ATGGAAACATTCTTGGACACAAATTGGAATACAACTATAACTCACACAAT
GTATACATCATGGCAGACAAACAAAAGAATGGAATCAAAGTTAACTTCA
AAATTAGACACAACATTGAAGATGGAAGCGTTCAACTAGCAGACCATTAT
CAACAAAATACTCCAATTGGCGATGGCCCTGTCCTTTTACCAGACAACCA
TTACCTGTCCACACAATCTGCCCTTTCGAAAGATCCCAACGAAAAGAGAG
ACCACATGGTCCTTCTTGAGTTTGTAACAGCTGCTGGGATTACACATGGC
ATGGATGAACTATACAAATAG
SEQ ID NO: 36 GFP protein sequence 
MSKGEELFTGVVPILVELDGDVNGHKFSVSGEGEGDATYGKLTLKFICT
TGKLPVPWPTLVTTFSYGVQCFSRYPDHMKRHDFFKSAMPEGYVQERTIFFK
DDGNYKTRAEVKFEGDTLVNRIELKGIDFKEDGNILGHKLEYNYNSHNVYIM
ADKQKNGIKVNFKIRHNIEDGSVQLADHYQQNTPIGDGPVLLPDNHYLSTQS
ALSKDPNEKRDHMVLLEFVTAAGITHGMDELYK*
SEQ ID NO: 37 Nucleic acid sequence encoding EGFP 
ATGGTGAGCAAGGGCGAGGAGCTGTTCACCGGGGTGGTGCCCATCCT
GGTCGAGCTGGACGGCGACGTAAACGGCCACAAGTTCAGCGTGTCCGGC
GAGGGCGAGGGCGATGCCACCTACGGCAAGCTGACCCTGAAGTTCATCT
GCACCACCGGCAAGCTGCCCGTGCCCTGGCCCACCCTCGTGACCACCCTG
ACCTACGGCGTGCAGTGCTTCAGCCGCTACCCCGACCACATGAAGCAGCA
CGACTTCTTCAAGTCCGCCATGCCCGAAGGCTACGTCCAGGAGCGCACCA
TCTTCTTCAAGGACGACGGCAACTACAAGACCCGCGCCGAGGTGAAGTTC
GAGGGCGACACCCTGGTGAACCGCATCGAGCTGAAGGGCATCGACTTCA
AGGAGGACGGCAACATCCTGGGGCACAAGCTGGAGTACAACTACAACAG
CCACAACGTCTATATCATGGCCGACAAGCAGAAGAACGGCATCAAGGTG
AACTTCAAGATCCGCCACAACATCGAGGACGGCAGCGTGCAGCTCGCCG
ACCACTACCAGCAGAACACCCCCATCGGCGACGGCCCCGTGCTGCTGCCC
GACAACCACTACCTGAGCACCCAGTCCGCCCTGAGCAAAGACCCCAACG
AGAAGCGCGATCACATGGTCCTGCTGGAGTTCGTGACCGCCGCCGGGATC
ACTCTCGGCATGGACGAGCTGTACAAGTAA
SEQ ID NO: 38 EGFP protein sequence 
MVSKGEELFTGVVPILVELDGDVNGHKFSVSGEGEGDATYGKLTLKFIC
TTGKLPVPWPTLVTTLTYGVQCFSRYPDHMKQHDFFKSAMPEGYVQERTIFF
KDDGNYKTRAEVKFEGDTLVNRIELKGIDFKEDGNILGHKLEYNYNSHNVYI
MADKQKNGIKVNFKIRHNIEDGSVQLADHYQQNTPIGDGPVLLPDNHYLSTQ
SALSKDPNEKRDHMVLLEFVTAAGITLGMDELYK*
SEQ ID NO: 39 Nucleic acid sequence encoding dTomato 
ATGGTGAGCAAGGGCGAGGAGGTCATCAAAGAGTTCATGCGCTTCA
AGGTGCGCATGGAGGGCTCCATGAACGGCCACGAGTTCGAGATCGAGGG
CGAGGGCGAGGGCCGCCCCTACGAGGGCACCCAGACCGCCAAGCTGAAG
 
GTGACCAAGGGCGGCCCCCTGCCCTTCGCCTGGGACATCCTGTCCCCCCA
GTTCATGTACGGCTCCAAGGCGTACGTGAAGCACCCCGCCGACATCCCCG
ATTACAAGAAGCTGTCCTTCCCCGAGGGCTTCAAGTGGGAGCGCGTGATG
AACTTCGAGGACGGCGGTCTGGTGACCGTGACCCAGGACTCCTCCCTGCA
GGACGGCACGCTGATCTACAAGGTGAAGATGCGCGGCACCAACTTCCCCC
CCGACGGCCCCGTAATGCAGAAGAAGACCATGGGCTGGGAGGCCTCCAC
CGAGCGCCTGTACCCCCGCGACGGCGTGCTGAAGGGCGAGATCCACCAG
GCCCTGAAGCTGAAGGACGGCGGCCACTACCTGGTGGAGTTCAAGACCA
TCTACATGGCCAAGAAGCCCGTGCAACTGCCCGGCTACTACTACGTGGAC
ACCAAGCTGGACATCACCTCCCACAACGAGGACTACACCATCGTGGAAC
AGTACGAGCGCTCCGAGGGCCGCCACCACCTGTTCCTGTACGGCATGGAC
GAGCTGTACAAGTAA
SEQ ID NO: 40 dTomato protein sequence 
MVSKGEEVIKEFMRFKVRMEGSMNGHEFEIEGEGEGRPYEGTQTAKLK
VTKGGPLPFAWDILSPQFMYGSKAYVKHPADIPDYKKLSFPEGFKWERVMN
FEDGGLVTVTQDSSLQDGTLIYKVKMRGTNFPPDGPVMQKKTMGWEASTE
RLYPRDGVLKGEIHQALKLKDGGHYLVEFKTIYMAKKPVQLPGYYYVDTKL
DITSHNEDYTIVEQYERSEGRHHLFLYGMDELYK*
SEQ ID NO: 41 Nucleic acid sequence encoding BFP 
ATGGTGAGCAAGGGCGAGGAGCTGTTCACCGGGGTGGTGCCCATCCT
GGTCGAGCTGGACGGCGACGTAAACGGCCACAAGTTCAGCGTGTCCGGC
GAGGGCGAGGGCGATGCCACCTACGGCAAGCTGACCCTGAAGTTCATCT
GCACCACCGGCAAGCTGCCCGTGCCCTGGCCCACCCTCGTGACCACCCTG
AGCCACGGCGTCCAGTGCTTCAGCCGCTACCCCGACCACATGAAGCAGCA
CGACTTCTTCAAGTCCGCCATGCCCGAAGGCTACGTCCAGGAGCGCACCA
TCTTCTTCAAGGACGACGGCAACTACAAGACCCGCGCCGAGGTGAAGTTC
GAGGGCGACACCCTGGTGAACCGCATCGAGCTGAAGGGCATCGACTTCA
AGGAGGACGGCAACATCCTGGGGCACAAGCTGGAGTACAACTTCAACAG
CCACAACGTCTATATCATGGCCGACAAGCAGAAGAACGGCATCAAGGCG
AACTTCAAGATCCGCCACAACATCGAGGACGGCAGCGTGCAGCTCGCCG
ACCACTACCAGCAGAACACCCCCATCGGCGACGGCCCCGTGCTGCTGCCC
GACAGCCACTACCTGAGCACCCAGTCCGCCCTGAGCAAAGACCCCAACG
AGAAGCGCGATCACATGGTCCTGCTGGAGTTCGTGACCGCCGCCGGGATC
ACTCTCGGCATGGACGAGCTGTACAAGTAA
SEQ ID NO: 42 BFP protein sequence 
MVSKGEELFTGVVPILVELDGDVNGHKFSVSGEGEGDATYGKLTLKFIC
TTGKLPVPWPTLVTTLSHGVQCFSRYPDHMKQHDFFKSAMPEGYVQERTIFF
KDDGNYKTRAEVKFEGDTLVNRIELKGIDFKEDGNILGHKLEYNFNSHNVYI
MADKQKNGIKANFKIRHNIEDGSVQLADHYQQNTPIGDGPVLLPDSHYLSTQ
SALSKDPNEKRDHMVLLEFVTAAGITLGMDELYK*
SEQ ID NO: 43 Nucleic acid sequence encoding YFP 
ATGGTGAGCAAGGGCGAGGAGCTGTTCACCGGGGTGGTGCCCATCCT
GGTCGAGCTGGACGGCGACGTAAACGGCCACAAGTTCAGCGTGTCCGGC
GAGGGCGAGGGCGATGCCACCTACGGCAAGCTGACCCTGAAGTTCATCT
GCACCACCGGCAAGCTGCCCGTGCCCTGGCCCACCCTCGTGACCACCTTC
GGCTACGGCGTGCAGTGCTTCGCCCGCTACCCCGACCACATGCGCCAGCA
CGACTTCTTCAAGTCCGCCATGCCCGAAGGCTACGTCCAGGAGCGCACCA
 
TCTTCTTCAAGGACGACGGCAACTACAAGACCCGCGCCGAGGTGAAGTTC
GAGGGCGACACCCTGGTGAACCGCATCGAGCTGAAGGGCATCGACTTCA
AGGAGGACGGCAACATCCTGGGGCACAAGCTGGAGTACAACTACAACAG
CCACAACGTCTATATCATGGCCGACAAGCAGAAGAACGGCATCAAGGTG
AACTTCAAGATCCGCCACAACATCGAGGACGGCAGCGTGCAGCTCGCCG
ACCACTACCAGCAGAACACCCCCATCGGCGACGGCCCCGTGCTGCTGCCC
GACAACCACTACCTGAGCTACCAGTCCGCCCTGAGCAAAGACCCCAACG
AGAAGCGCGATCACATGGTCCTGCTGGAGTTCGTGACCGCCGCCGGGATC
ACTCTCGGCATGGACGAGCTGTACAAGTAA
SEQ ID NO: 44 YFP protein sequence 
MVSKGEELFTGVVPILVELDGDVNGHKFSVSGEGEGDATYGKLTLKFIC
TTGKLPVPWPTLVTTFGYGVQCFARYPDHMRQHDFFKSAMPEGYVQERTIF
FKDDGNYKTRAEVKFEGDTLVNRIELKGIDFKEDGNILGHKLEYNYNSHNV
YIMADKQKNGIKVNFKIRHNIEDGSVQLADHYQQNTPIGDGPVLLPDNHYLS
YQSALSKDPNEKRDHMVLLEFVTAAGITLGMDELYK*
SEQ ID NO: 45 Nucleic acid sequence encoding ZsGreen 
ATGGCTCAGTCAAAGCACGGTCTAACAAAAGAAATGACAATGAAAT
ACCGTATGGAAGGGTGCGTCGATGGACATAAATTTGTGATCACGGGAGA
GGGCATTGGATATCCGTTCAAAGGGAAACAGGCTATTAATCTGTGTGTGG
TCGAAGGTGGACCATTGCCATTTGCCGAAGACATATTGTCAGCTGCCTTT
ATGTACGGAAACAGGGTTTTCACTGAATATCCTCAAGACATAGCTGACTA
TTTCAAGAACTCGTGTCCTGCTGGTTATACATGGGACAGGTCTTTTCTCTT
TGAGGATGGAGCAGTTTGCATATGTAATGCAGATATAACAGTGAGTGTTG
AAGAAAACTGCATGTATCATGAGTCCAAATTTTATGGAGTGAATTTTCCT
GCTGATGGACCTGTGATGAAAAAGATGACAGATAACTGGGAGCCATCCT
GCGAGAAGATCATACCAGTACCTAAGCAGGGGATATTGAAAGGGGATGT
CTCCATGTACCTCCTTCTGAAGGATGGTGGGCGTTTACGGTGCCAATTCG
ACACAGTTTACAAAGCAAAGTCTGTGCCAAGAAAGATGCCGGACTGGCA
CTTCATCCAGCATAAGCTCACCCGTGAAGACCGCAGCGATGCTAAGAATC
AGAAATGGCATCTGACAGAACATGCTATTGCATCCGGATCTGCATTGCCC
TGA
SEQ ID NO: 46 ZsGreen protein sequence 
MAQSKHGLTKEMTMKYRMEGCVDGHKFVITGEGIGYPFKGKQAINLC
VVEGGPLPFAEDILSAAFMYGNRVFTEYPQDIADYFKNSCPAGYTWDRSFLF
EDGAVCICNADITVSVEENCMYHESKFYGVNFPADGPVMKKMTDNWEPSCE
KIIPVPKQGILKGDVSMYLLLKDGGRLRCQFDTVYKAKSVPRKMPDWHFIQH
KLTREDRSDAKNQKWHLTEHAIASGSALP*
SEQ ID NO: 47 Nucleic acid sequence encoding Firefly luciferase (FLuc) 
ATGGAAGATGCCAAAAACATTAAGAAAGGCCCAGCGCCATTCTACC
CACTCGAAGACGGGACCGCTGGCGAGCAGCTGCATAAAGCCATGAAGCG
CTACGCCCTGGTGCCCGGCACCATCGCCTTTACCGACGCACATATCGAGG
TGGACATTACCTACGCCGAGTACTTCGAGATGAGCGTTCGGCTGGCAGAA
GCTATGAAGCGCTATGGGCTGAATACAAACCATCGGATCGTGGTGTGTAG
CGAGAATAGCTTGCAGTTCTTCATGCCCGTGTTGGGTGCCCTGTTCATCGG
TGTGGCTGTGGCCCCAGCTAACGACATCTACAACGAGCGCGAGCTGCTGA
ACAGCATGGGCATCAGCCAGCCCACCGTCGTATTCGTGAGCAAGAAAGG
GCTGCAAAAGATCCTCAACGTGCAAAAGAAGCTACCGATCATACAAAAG
 
ATCATCATCATGGATAGCAAGACCGACTACCAGGGCTTCCAAAGCATGTA
CACCTTCGTGACTTCCCATTTGCCACCCGGCTTCAACGAGTACGACTTCGT
GCCCGAGAGCTTCGACCGGGACAAAACCATCGCCCTGATCATGAACAGT
AGTGGCAGTACCGGATTGCCCAAGGGCGTAGCCCTACCGCACCGCACCGC
TTGTGTCCGATTCAGTCATGCCCGCGACCCCATCTTCGGCAACCAGATCA
TCCCCGACACCGCTATCCTGAGCGTGGTGCCATTTCACCACGGCTTCGGC
ATGTTCACCACGCTGGGCTACTTGATCTGCGGCTTTCGGGTCGTGCTCATG
TACCGCTTCGAGGAAGAGCTATTCTTGCGCAGCTTGCAAGACTATAAGAT
TCAATCTGCCCTGCTGGTGCCCACACTATTTAGCTTCTTCGCTAAGAGCAC
TCTCATCGACAAGTACGACCTAAGCAACTTGCACGAGATCGCCAGCGGCG
GGGCGCCGCTCAGCAAGGAGGTAGGTGAGGCCGTGGCCAAACGCTTCCA
CCTACCAGGCATCCGCCAGGGCTACGGCCTGACAGAAACAACCAGCGCC
ATTCTGATCACCCCCGAAGGGGACGACAAGCCTGGCGCAGTAGGCAAGG
TGGTGCCCTTCTTCGAGGCTAAGGTGGTGGACTTGGACACCGGTAAGACA
CTGGGTGTGAACCAGCGCGGCGAGCTGTGCGTCCGTGGCCCCATGATCAT
GAGCGGCTACGTTAACAACCCCGAGGCTACAAACGCTCTCATCGACAAG
GACGGCTGGCTGCATAGCGGCGACATCGCCTACTGGGACGAGGACGAGC
ACTTCTTCATCGTGGACCGGCTGAAGAGCCTGATCAAATACAAGGGCTAC
CAGGTAGCCCCAGCCGAACTGGAGAGCATCCTGCTGCAACACCCCAACAT
CTTCGACGCCGGGGTCGCCGGCCTGCCCGACGACGATGCCGGCGAGCTGC
CCGCCGCAGTCGTCGTGCTGGAACACGGTAAAACCATGACCGAGAAGGA
GATCGTGGACTATGTGGCCAGCCAGGTTACAACCGCCAAGAAGCTGCGC
GGTGGTGTTGTGTTCGTGGACGAGGTGCCTAAAGGACTGACCGGCAAGTT
GGACGCCCGCAAGATCCGCGAGATTCTCATTAAGGCCAAGAAGGGCGGC
AAGATCGCCGTCTAG
SEQ ID NO: 48 Firefly luciferase (FLuc) protein sequence 
MEDAKNIKKGPAPFYPLEDGTAGEQLHKAMKRYALVPGTIAFTDAHIE
VDITYAEYFEMSVRLAEAMKRYGLNTNHRIVVCSENSLQFFMPVLGALFIGV
AVAPANDIYNERELLNSMGISQPTVVFVSKKGLQKILNVQKKLPIIQKIIIMDS
KTDYQGFQSMYTFVTSHLPPGFNEYDFVPESFDRDKTIALIMNSSGSTGLPKG
VALPHRTACVRFSHARDPIFGNQIIPDTAILSVVPFHHGFGMFTTLGYLICGFR
VVLMYRFEEELFLRSLQDYKIQSALLVPTLFSFFAKSTLIDKYDLSNLHEIASG
GAPLSKEVGEAVAKRFHLPGIRQGYGLTETTSAILITPEGDDKPGAVGKVVPF
FEAKVVDLDTGKTLGVNQRGELCVRGPMIMSGYVNNPEATNALIDKDGWL
HSGDIAYWDEDEHFFIVDRLKSLIKYKGYQVAPAELESILLQHPNIFDAGVAG
LPDDDAGELPAAVVVLEHGKTMTEKEIVDYVASQVTTAKKLRGGVVFVDE
VPKGLTGKLDARKIREILIKAKKGGKIAV*
SEQ ID NO: 49 Nucleic acid sequence encoding Renilla Luciferase (RLuc) 
ATGACCAGCAAAGTGTATGATCCGGAACAGCGCAAACGCATGATTA
CCGGCCCGCAGTGGTGGGCGCGCTGCAAACAGATGAACGTGCTGGATAG
CTTTATTAACTATTATGATAGCGAAAAACATGCGGAAAACGCGGTGATTT
TTCTGCATGGCAACGCGGCGAGCAGCTATCTGTGGCGGCATGTGGTGCCG
CATATTGAACCAGTGGCGCGCTGCATTATTCCGGATCTGATTGGCATGGG
CAAAAGCGGCAAAAGCGGCAACGGCAGCTATCGCCTGCTGGATCATTAT
AAATATCTGACCGCGTGGTTTGAACTGCTGAACCTGCCGAAAAAAATTAT
TTTTGTGGGCCATGATTGGGGCGCGTGCCTGGCGTTTCATTATAGCTATGA
ACATCAGGATAAAATTAAAGCGATTGTGCACGCGGAAAGCGTGGTGGAT
 
GTGATTGAAAGCTGGGATGAATGGCCGGATATTGAAGAAGATATTGCGCT
GATTAAAAGCGAAGAAGGCGAAAAAATGGTGCTGGAAAACAACTTTTTT
GTGGAAACCATGCTGCCGAGCAAAATTATGCGCAAACTGGAACCGGAAG
AATTTGCGGCGTATCTGGAACCGTTTAAAGAAAAAGGCGAAGTGCGCCG
CCCGACCCTGAGCTGGCCGCGCGAAATTCCGCTGGTGAAAGGCGGCAAA
CCGGATGTGGTCCAGATTGTGCGCAACTATAACGCGTATCTGCGCGCGAG
CGATGATCTGCCGAAAATGTTTATTGAAAGCGATCCGGGCTTTTTTAGCA
ACGCGATTGTGGAAGGCGCGAAAAAATTTCCGAACACCGAATTTGTGAA
AGTGAAGGGCCTGCATTTTAGCCAGGAAGATGCGCCGGATGAAATGGGC
AAATATATTAAGAGCTTTGTGGAACGCGTGCTGAAAAACGAACAGGGCT
AG
SEQ ID NO: 50 Renilla Luciferase (RLuc) protein sequence 
MTSKVYDPEQRKRMITGPQWWARCKQMNVLDSFINYYDSEKHAENAV
IFLHGNAASSYLWRHVVPHIEPVARCIIPDLIGMGKSGKSGNGSYRLLDHYK
YLTAWFELLNLPKKIIFVGHDWGACLAFHYSYEHQDKIKAIVHAESVVDVIE
SWDEWPDIEEDIALIKSEEGEKMVLENNFFVETMLPSKIMRKLEPEEFAAYLE
PFKEKGEVRRPTLSWPREIPLVKGGKPDVVQIVRNYNAYLRASDDLPKMFIE
SDPGFFSNAIVEGAKKFPNTEFVKVKGLHFSQEDAPDEMGKYIKSFVERVLK
NEQG*
SEQ ID NO: 51 Nucleic acid sequence encoding Gaussia Luciferase (GLuc) 
ATGGGAGTCAAAGTTCTGTTTGCCCTGATCTGCATCGCTGTGGCCGA
GGCCAAGCCCACCGAGAACAACGAAGACTTCAACATCGTGGCCGTGGCC
AGCAACTTCGCGACCACGGATCTCGATGCTGACCGCGGGAAGTTGCCCGG
CAAGAAGCTGCCGCTGGAGGTGCTCAAAGAGATGGAAGCCAATGCCCGG
AAAGCTGGCTGCACCAGGGGCTGTCTGATCTGCCTGTCCCACATCAAGTG
CACGCCCAAGATGAAGAAGTTCATCCCAGGACGCTGCCACACCTACGAA
GGCGACAAAGAGTCCGCACAGGGCGGCATAGGCGAGGCGATCGTCGACA
TTCCTGAGATTCCTGGGTTCAAGGACTTGGAGCCCATGGAGCAGTTCATC
GCACAGGTCGATCTGTGTGTGGACTGCACAACTGGCTGCCTCAAAGGGCT
TGCCAACGTGCAGTGTTCTGACCTGCTCAAGAAGTGGCTGCCGCAACGCT
GTGCGACCTTTGCCAGCAAGATCCAGGGCCAGGTGGACAAGATCAAGGG
GGCCGGTGGTGACTAA
SEQ ID NO: 52 Gaussia Luciferase (GLuc) protein sequence 
MGVKVLFALICIAVAEAKPTENNEDFNIVAVASNFATTDLDADRGKLPG
KKLPLEVLKEMEANARKAGCTRGCLICLSHIKCTPKMKKFIPGRCHTYEGDK
ESAQGGIGEAIVDIPEIPGFKDLEPMEQFIAQVDLCVDCTTGCLKGLANVQCS
DLLKKWLPQRCATFASKIQGQVDKIKGAGGD*
SEQ ID NO: 53 Nucleic acid sequence encoding Nano-Glo Luciferase (NLuc) 
ATGGTCTTCACACTCGAAGATTTCGTTGGGGACTGGCGACAGACAGC
CGGCTACAACCTGGACCAAGTCCTTGAACAGGGAGGTGTGTCCAGTTTGT
TTCAGAATCTCGGGGTGTCCGTAACTCCGATCCAAAGGATTGTCCTGAGC
GGTGAAAATGGGCTGAAGATCGACATCCATGTCATCATCCCGTATGAAGG
TCTGAGCGGCGACCAAATGGGCCAGATCGAAAAAATTTTTAAGGTGGTGT
ACCCTGTGGATGATCATCACTTTAAGGTGATCCTGCACTATGGCACACTG
GTAATCGACGGGGTTACGCCGAACATGATCGACTATTTCGGACGGCCGTA
TGAAGGCATCGCCGTGTTCGACGGCAAAAAGATCACTGTAACAGGGACC
CTGTGGAACGGCAACAAAATTATCGACGAGCGCCTGATCAACCCCGACG
 
GCTCCCTGCTGTTCCGAGTAACCATCAACGGAGTGACCGGCTGGCGGCTG
TGCGAACGCATTCTGGCGTAA
SEQ ID NO: 54 Nano-Glo Luciferase (NLuc) protein sequence 
MVFTLEDFVGDWRQTAGYNLDQVLEQGGVSSLFQNLGVSVTPIQRIVLS
GENGLKIDIHVIIPYEGLSGDQMGQIEKIFKVVYPVDDHHFKVILHYGTLVID
GVTPNMIDYFGRPYEGIAVFDGKKITVTGTLWNGNKIIDERLINPDGSLLFRV
TINGVTGWRLCERILA*
SEQ ID NO: 55 Nucleic acid sequence encoding Cypridina Luciferase (CLuc) 
ATGAAGACCTTAATTCTTGCCGTTGCATTAGTCTACTGCGCCACTGTT
CATTGCCAGGACTGTCCTTACGAACCTGATCCACCAAACACAGTTCCAAC
TTCCTGTGAAGCTAAAGAAGGAGAATGTATTGATAGCAGCTGTGGCACCT
GCACGAGAGACATACTATCAGATGGACTGTGTGAAAATAAACCAGGAAA
AACATGTTGCCGAATGTGTCAGTATGTAATTGAATGCAGAGTAGAGGCCG
CAGGATGGTTTAGAACATTCTATGGAAAGAGATTCCAGTTCCAGGAACCT
GGTACATACGTGTTGGGTCAAGGAACCAAGGGCGGCGACTGGAAGGTGT
CCATCACCCTGGAGAACCTGGATGGAACCAAGGGGGCTGTGCTGACCAA
GACAAGACTGGAAGTGGCTGGAGACATCATTGACATCGCTCAAGCTACTG
AGAATCCCATCACTGTAAACGGTGGAGCTGACCCTATCATCGCCAACCCG
TACACCATCGGCGAGGTCACCATCGCTGTTGTTGAGATGCCAGGCTTCAA
CATCACCGTCATTGAGTTCTTCAAACTGATCGTGATCGACATCCTCGGAG
GAAGATCTGTAAGAATCGCCCCAGACACAGCAAACAAAGGAATGATCTC
TGGCCTCTGTGGAGATCTTAAAATGATGGAAGATACAGACTTCACTTCAG
ATCCAGAACAACTCGCTATTCAGCCTAAGATCAACCAGGAGTTTGACGGT
TGTCCACTCTATGGAAATCCTGATGACGTTGCATACTGCAAAGGTCTTCT
GGAGCCGTACAAGGACAGCTGCCGCAACCCCATCAACTTCTACTACTACA
CCATCTCCTGCGCCTTCGCCCGCTGTATGGGTGGAGACGAGCGAGCCTCA
CACGTGCTGCTTGACTACAGGGAGACGTGCGCTGCTCCCGAAACTAGAGG
AACCTGCGTTTTGTCTGGACATACTTTCTACGATACATTTGACAAAGCAA
GATACCAATTCCAGGGTCCCTGCAAGGAGATTCTTATGGCCGCCGACTGT
TTCTGGAACACTTGGGATGTGAAGGTTTCACACAGGAATGTTGACTCTTA
CACTGAAGTAGAGAAAGTACGAATCAGGAAACAATCGACTGTAGTAGAA
CTCATTGTTGATGGAAAACAGATTCTGGTTGGAGGAGAAGCCGTGTCCGT
CCCGTACAGCTCTCAGAACACTTCCATCTACTGGCAAGATGGTGACATAC
TGACTACAGCCATCCTACCTGAAGCTCTGGTGGTCAAGTTCAACTTCAAG
CAACTGCTCGTCGTACATATTAGAGATCCATTCGATGGTAAGACTTGCGG
TATTTGCGGTAACTACAACCAGGATTTCAGTGATGATTCTTTTGATGCTGA
AGGAGCCTGTGATCTGACCCCCAACCCACCGGGATGCACCGAAGAACAG
AAACCTGAAGCTGAACGACTCTGCAATAGTCTCTTCGCCGGTCAAAGTGA
TCTTGATCAGAAATGTAACGTGTGCCACAAGCCTGACCGTGTCGAACGAT
GCATGTACGAGTATTGCCTGAGGGGACAACAGGGTTTCTGTGACCACGCA
TGGGAGTTCAAGAAAGAATGCTACATAAAGCATGGAGACACCCTAGAAG
TACCAGATGAATGCAAATAG
SEQ ID NO: 56 Cypridina Luciferase (CLuc) protein sequence 
MKTLILAVALVYCATVHCQDCPYEPDPPNTVPTSCEAKEGECIDSSCGT
CTRDILSDGLCENKPGKTCCRMCQYVIECRVEAAGWFRTFYGKRFQFQEPGT
YVLGQGTKGGDWKVSITLENLDGTKGAVLTKTRLEVAGDIIDIAQATENPIT
VNGGADPIIANPYTIGEVTIAVVEMPGFNITVIEFFKLIVIDILGGRSVRIAPDT
 
ANKGMISGLCGDLKMMEDTDFTSDPEQLAIQPKINQEFDGCPLYGNPDDVA
YCKGLLEPYKDSCRNPINFYYYTISCAFARCMGGDERASHVLLDYRETCAAP
ETRGTCVLSGHTFYDTFDKARYQFQGPCKEILMAADCFWNTWDVKVSHRN
VDSYTEVEKVRIRKQSTVVELIVDGKQILVGGEAVSVPYSSQNTSIYWQDGDI
LTTAILPEALVVKFNFKQLLVVHIRDPFDGKTCGICGNYNQDFSDDSFDAEG
ACDLTPNPPGCTEEQKPEAERLCNSLFAGQSDLDQKCNVCHKPDRVERCMY
EYCLRGQQGFCDHAWEFKKECYIKHGDTLEVPDECK*
SEQ ID NO: 57 CaMV poly(A) signal nucleic acid sequence 
TTTCTCCATAATAATGTGTGAGTAGTTCCCAGATAAGGGAATTAGGG
TTCCTATAGGGTTTCGCTCATGTGTTGAGCATATAAGAAACCCTTAGTATG
TATTTGTATTTGTAAAATACTTCTATCAATAAAATTTCTAATTCCTAAAAC
CAAAATCCAGTACTAAAATCCAGATC
SEQ ID NO: 58 EF-1α poly(A) signal nucleic acid sequence 
ATTATCCCTAATACCTGCCACCCCACTCTTAATCAGTGGTGGAAGAA
CGGTCTCAGAACTGTTTGTTTCAATTGGCCATTTAAGTTTAGTAGTAAAAG
ACTGGTTAATGATAACAATGCATCGTAAAACCTTCAGAAGGAAAGGAGA
ATGTTTTGTGGACCACTTTGGTTTTCTTTTTTGCGTGTGGCAGTTTTAAGTT
ATTAGTTTTTAAAATCAGTACTTTTTAATGGAAACAACTTGACCAAAAAT
TTGTCACAGAATTTTGAGACCCATTAAAAAAGTTAAATGAGAAACCTGTG
TGTTCCTTTGGTCAACACCGAGACATTTAGGTGAAAGACATCTAATTCTG
GTTTTACGAATCTGGAAACTTCTTGAAAATGTAATTCTTGAGTTAACACTT
CTGGGTGGAGAATAGGGTTGTTTTCCCCCCACATAATTGGAAGGGGAAGG
AATATCATTTAAAGCTATGGGAGGGTTTCTTTGATTACAACACTGGAGAG
AAATGCAGCATGTTGCTGATTGCCTGTCACTAAAACAGGCCAAAAACTGA
GTCCTTGGGTTGCATAGAAAGCT
SEQ ID NO: 59 bGH poly(A) signal nucleic acid sequence 
CTGTGCCTTCTAGTTGCCAGCCATCTGTTGTTTGCCCCTCCCCCGTGC
CTTCCTTGACCCTGGAAGGTGCCACTCCCACTGTCCTTTCCTAATAAAATG
AGGAAATTGCATC
SEQ ID NO: 60 hGH poly(A) signal nucleic acid sequence 
GGGTGGCATCCCTGTGACCCCTCCCCAGTGCCTCTCCTGGCCCTGGA
AGTTGCCACTCCAGTGCCCACCAGCCTTGTCCTAATAAAATTAAGTTGCA
TCATTTTGTCTGACTAGGTGTCCTTCTATAATATTATGGGGTGGAGGGGG
GTGGTATGGAGCAAGGGGCAAGTTGGGAAGACAACCTGTAGGGCCTGCG
GGGTCTATTGGGAACCAAGCTGGAGTGCAGTGGCACAATCTTGGCTCACT
GCAATCTCCGCCTCCTGGGTTCAAGCGATTCTCCTGCCTCAGCCTCCCGAG
TTGTTGGGATTCCAGGCATGCATGACCAGGCTCAGCTAATTTTTGTTTTTT
TGGTAGAGACGGGGTTTCACCATATTGGCCAGGCTGGTCTCCAACTCCTA
ATCTCAGGTGATCTACCCACCTTGGCCTCCCAAATTGCTGGGATTACAGG
CGTGAACCACTGCTCCCTTCCCTGTCCTT
SEQ ID NO: 61 SV40 poly(A) signal nucleic acid sequence 
AACTTGTTTATTGCAGCTTATAATGGTTACAAATAAAGCAATAGCAT
CACAAATTTCACAAATAAAGCATTTTTTTCACTGCATTCTAGTTGTGGTTT
GTCCAAACTCATCAATGTATCTTA
SEQ ID NO: 62 human β-globin poly(A) signal nucleic acid sequence 
GCTCGCTTTCTTGCTGTCCAATTTCTATTAAAGGTTCCTTTGTTCCCTA
AGTCCAACTACTAAACTGGGGGATATTATGAAGGGCCTTGAGCATCTGGA
 
TTCTGCCTAATAAAAAACATTTATTTTCATTGCAATGATGTATTTAAATTA
TTTCTGAATATTTTACTAAAAAGGGAATGTGGGAGGTCAGTGCATTTAAA
ACATAAAGAAATGAAGAGCTAGTTCAAACCTTGGGAAAATACACTATAT
CTTAAACTCCATGAAAGAAGGTGAGGCTGCAAACAGCTAATGCACATTG
GCAACAGCCCCTGATGCCTATGCCTTATTCATCCCTCAGAAAAGGATTCA
AGTAGAGGCTTGATTTGGAGGTTAAAGTTTTGCTATGCTGTATTTTA
SEQ ID NO: 63 Nucleic acid sequence encoding the nuclear localization signal peptide sequence
   CCAAAAAAGAAGAGAAAGGTA
SEQ ID NO: 64 Nuclear localization signal peptide protein sequence
   PKKKRKV
SEQ ID NO: 65 Nucleic acid sequence encoding the mitochondrial localization signal peptide sequence 
ATGCTGTTTAATCTGAGGATCCTGTTAAACAATGCAGCTTTTAGAAAT
GGTCACAACTTCATGGTTCGAAATTTTCGGTGTGGACAACCACTACAAAA
TAAAGTGCAG
SEQ ID NO: 66 Mitochondrial localization signal peptide protein sequence
   MLFNLRILLNNAAFRNGHNFMVRNFRCGQPLQNKVQ
SEQ ID NO: 67 Nucleic acid sequence encoding the nuclear export signal peptide sequence
   CTGCCCCCCCTGGAGCGGCTGACCCTG
SEQ ID NO: 68 Nuclear export signal peptide protein sequence
   LPPLERLTL
SEQ ID NO: 69 Nucleic acid sequence encoding ANG protein 
ATGGTGATGGGCCTGGGCGTTTTGTTGTTGGTCTTCGTGCTGGGTCTG
GGTCTGACCCCACCGACCCTGGCTCAGGATAACTCCAGGTACACACACTT
CCTGACCCAGCACTATGATGCCAAACCACAGGGCCGGGATGACAGATAC
TGTGAAAGCATCATGAGGAGACGGGGCCTGACCTCACCCTGCAAAGACA
TCAACACATTTATTCATGGCAACAAGCGCAGCATCAAGGCCATCTGTGAA
AACAAGAATGGAAACCCTCACAGAGAAAACCTAAGAATAAGCAAGTCTT
CTTTCCAGGTCACCACTTGCAAGCTACATGGAGGTTCCCCCTGGCCTCCAT
GCCAGTACCGAGCCACAGCGGGGTTCAGAAACGTTGTTGTTGCTTGTGAA
AATGGCTTACCTGTCCACTTGGATCAGTCAATTTTCCGTCGTCCG
SEQ ID NO: 70 ANG protein sequence
SEQ ID NO: 71 Nucleic acid sequence encoding ANG-S protein 
CAGGATAACTCCAGGTACACACACTTCCTGACCCAGCACTATGATGC
CAAACCACAGGGCCGGGATGACAGATACTGTGAAAGCATCATGAGGAGA
CGGGGCCTGACCTCACCCTGCAAAGACATCAACACATTTATTCATGGCAA
CAAGCGCAGCATCAAGGCCATCTGTGAAAACAAGAATGGAAACCCTCAC
AGAGAAAACCTAAGAATAAGCAAGTCTTCTTTCCAGGTCACCACTTGCAA
GCTACATGGAGGTTCCCCCTGGCCTCCATGCCAGTACCGAGCCACAGCGG
GGTTCAGAAACGTTGTTGTTGCTTGTGAAAATGGCTTACCTGTCCACTTGG
ATCAGTCAATTTTCCGTCGTCCG
SEQ ID NO: 72 ANG-S protein sequence
SEQ ID NO: 73 Nucleic acid sequence encoding RNASEL protein 
ATTGATGAAAAATACAAAATTGCTGATACTTCAGAAGGAGGCATCTA
CCTGGGGTTCTATGAGAAGCAAGAAGTAGCTGTGAAGACGTTCTGTGAGG
GCAGCCCACGTGCACAGCGGGAAGTCTCTTGTCTGCAAAGCAGCCGAGA
GAACAGTCACTTGGTGACATTCTATGGGAGTGAGAGCCACAGGGGCCACT
TGTTTGTGTGTGTCACCCTCTGTGAGCAGACTCTGGAAGCGTGTTTGGATG
TGCACAGAGGGGAAGATGTGGAAAATGAGGAAGATGAATTTGCCCGAAA
TGTCCTGTCATCTATATTTAAGGCTGTTCAAGAACTACACTTGTCCTGTGG
ATACACCCACCAGGATCTGCAACCACAAAACATCTTAATAGATTCTAAGA
AAGCTGCTCACCTGGCAGATTTTGATAAGAGCATCAAGTGGGCTGGAGAT
CCACAGGAAGTCAAGAGAGATCTAGAGGACCTTGGACGGCTGGTCCTCT
ATGTGGTAAAGAAGGGAAGCATCTCATTTGAGGATCTGAAAGCTCAAAG
TAATGAAGAGGTGGTTCAACTTTCTCCAGATGAGGAAACTAAGGACCTCA
TTCATCGTCTCTTCCATCCTGGGGAACATGTGAGGGACTGTCTGAGTGAC
CTGCTGGGTCATCCCTTCTTTTGGACTTGGGAGAGCCGCTATAGGACGCTT
CGGAATGTGGGAAATGAATCCGACATCAAAACACGAAAATCTGAAAGTG
AGATCCTCAGACTACTGCAACCTGGGCCTTCTGAACATTCCAAAAGTTTT
GACAAGTGGACGACTAAGATTAATGAATGTGTTATGAAAAAAATGAATA
AGTTTTATGAAAAAAGAGGCAATTTCTACCAGAACACTGTGGGTGATCTG
CTAAAGTTCATCCGGAATTTGGGAGAACACATTGATGAAGAAAAGCATA
AAAAGATGAAATTAAAAATTGGAGACCCTTCCCTGTATTTTCAGAAGACA
TTTCCAGATCTGGTGATCTATGTCTACACAAAACTACAGAACACAGAATA
TAGAAAGCATTTCCCCCAAACCCACAGTCCAAAC
SEQ ID NO: 74 RNASEL protein sequence 
IDEKYKIADTSEGGIYLGFYEKQEVAVKTFCEGSPRAQREVSCLQSSREN
SHLVTFYGSESHRGHLFVCVTLCEQTLEACLDVHRGEDVENEEDEFARNVLS
SIFKAVQELHLSCGYTHQDLQPQNILIDSKKAAHLADFDKSIKWAGDPQEVK
RDLEDLGRLVLYVVKKGSISFEDLKAQSNEEVVQLSPDEETKDLIHRLFHPGE
HVRDCLSDLLGHPFFWTWESRYRTLRNVGNESDIKTRKSESEILRLLQPGPSE
HSKSFDKWTTKINECVMKKMNKFYEKRGNFYQNTVGDLLKFIRNLGEHIDE
EKHKKMKLKIGDPSLYFQKTFPDLVIYVYTKLQNTEYRKHFPQTHSPN
SEQ ID NO: 75 Nucleic acid sequence encoding RRP44 protein 
CACTACTTGCTGCCCGACACTAATGTGTTACTGCACCAGATTGATGTT
CTTGAGGACCCTGCCATCAGGAATGTAATTGTGCTACAAACAGTTCTTCA
AGAAGTGAGAAATCGCAGTGCCCCCGTATATAAACGCATCCGAGATGTG
ACTAATAACCAAGAGAAGCATTTCTATACTTTCACTAATGAGCACCATAG
AGAAACCTATGTAGAACAAGAACAGGGAGAAAATGCTAATGACAGGAAT
GATAGAGCGATTCGAGTAGCAGCAAAATGGTACAATGAACATTTGAAAA
AAATGTCAGCAGACAACCAGCTGCAAGTTATCTTCATAACAAATGACAGG
AGAAACAAAGAG
SEQ ID NO: 76 RRP44 protein sequence 
HYLLPDTNVLLHQIDVLEDPAIRNVIVLQTVLQEVRNRSAPVYKRIRDVT
NNQEKHFYTFTNEHHRETYVEQEQGENANDRNDRAIRVAAKWYNEHLKKM
SADNQLQVIFITNDRRNKE
SEQ ID NO: 77 Nucleic acid sequence encoding RNASE1 protein 
AAGGAATCCCGGGCCAAGAAATTCCAGCGGCAGCATATGGACTCAG
ACAGTTCCCCCAGCAGCAGCTCCACCTACTGTAACCAAATGATGAGGCGC
CGGAATATGACACAGGGGCGGTGCAAACCAGTGAACACCTTTGTGCACG
AGCCCCTGGTAGATGTCCAGAATGTCTGTTTCCAGGAAAAGGTCACCTGC
AAGAACGGGCAGGGCAACTGCTACAAGAGCAACTCCAGCATGCACATCA
CAGACTGCCGCCTGACAAACGGCTCCAGGTACCCCAACTGTGCATACCGG
ACCAGCCCGAAGGAGAGACACATCATTGTGGCCTGTGAAGGGAGCCCAT
ATGTGCCAGTCCACTTTGATGCTTCTGTGGAGGACTCTACC
SEQ ID NO: 78 RNASE1 protein sequence 
KESRAKKFQRQHMDSDSSPSSSSTYCNQMMRRRNMTQGRCKPVNTFV
HEPLVDVQNVCFQEKVTCKNGQGNCYKSNSSMHITDCRLTNGSRYPNCAYR
TSPKERHIIVACEGSPYVPVHFDASVEDST
SEQ ID NO: 79 Nucleic acid sequence encoding PNPT1 protein 
GCTGTGGCCGTGGACTTAGGCAACAGGAAATTAGAAATATCTTCTGG
AAAGCTGGCCAGATTTGCAGATGGCTCTGCTGTAGTACAGTCAGGTGACA
CTGCAGTAATGGTCACAGCGGTCAGTAAAACAAAACCTTCCCCTTCCCAG
TTTATGCCTTTGGTGGTTGACTACAGACAAAAAGCTGCTGCAGCAGGTAG
AATTCCCACAAACTATCTGAGAAGAGAGATTGGTACTTCTGATAAAGAAA
TTCTAACAAGTCGAATAATAGATCGTTCAATTAGACCGCTCTTTCCAGCT
GGCTACTTCTATGATACACAGGTTCTGTGTAATCTGTTAGCAGTAGATGG
TGTAAATGAGCCTGATGTCCTAGCAATTAATGGCGCTTCCGTAGCCCTCT
CATTATCAGATATTCCTTGGAATGGACCTGTTGGGGCAGTACGAATAGGA
ATAATTGATGGAGAATATGTTGTTAACCCAACAAGAAAAGAAATGTCTTC
TAGTACTTTAAATTTAGTGGTTGCTGGAGCACCTAAAAGTCAGATTGTCA
TGTTGGAAGCCTCTGCAGAGAACATTTTACAGCAGGACTTTTGCCATGCT
ATCAAAGTGGGAGTGAAATATACCCAACAAATAATTCAGGGCATTCAGC
AGTTGGTAAAAGAAACTGGTGTTACCAAGAGGACACCTCAGAAGTTATTT
ACCCCTTCGCCAGAGATTGTGAAATATACTCATAAACTTGCTATGGAGAG
ACTCTATGCAGTTTTTACAGATTACGAGCATGACAAAGTTTCCAGAGATG
AAGCTGTTAACAAAATAAGATTAGATACGGAGGAACAACTAAAAGAAAA
ATTTCCAGAAGCCGATCCATATGAAATAATAGAATCCTTCAATGTTGTTG
CAAAGGAAGTTTTTAGAAGTATTGTTTTGAATGAATACAAAAGGTGCGAT
GGTCGGGATTTGACTTCACTTAGGAATGTAAGTTGTGAGGTAGATATGTT
TAAAACCCTTCATGGATCAGCATTATTTCAAAGAGGACAAACACAGGTGC
TTTGTACCGTTACATTTGATTCATTAGAATCTGGTATTAAGTCAGATCAAG
TTATAACAGCTATAAATGGGATAAAAGATAAAAATTTCATGCTGCACTAC
GAGTTTCCTCCTTATGCAACTAATGAAATTGGCAAAGTCACTGGTTTAAA
TAGAAGAGAACTTGGGCATGGTGCTCTTGCTGAGAAAGCTTTGTATCCTG
TTATTCCCCGAGATTTTCCTTTCACCATAAGAGTTACATCTGAAGTCCTAG
AGTCAAATGGGTCATCTTCTATGGCATCTGCATGTGGCGGAAGTTTAGCA
TTAATGGATTCAGGGGTTCCAATTTCATCTGCTGTTGCAGGCGTAGCAAT
AGGATTGGTCACCAAAACCGATCCTGAGAAGGGTGAAATAGAAGATTAT
CGTTTGCTGACAGATATTTTGGGAATTGAAGATTACAATGGTGACATGGA
CTTCAAAATAGCTGGCACTAATAAAGGAATAACTGCATTACAGGCTGATA
TTAAATTACCTGGAATACCAATAAAAATTGTGATGGAGGCTATTCAACAA
GCTTCAGTGGCAAAAAAGGAGATATTACAGATCATGAACAAAACTATTTC
 AAAACCTCGAGCATCTAGAAAAGAAAATGGA
SEQ ID NO: 80 PNPT1 protein sequence 
AVAVDLGNRKLEISSGKLARFADGSAVVQSGDTAVMVTAVSKTKPSPS
QFMPLVVDYRQKAAAAGRIPTNYLRREIGTSDKEILTSRIIDRSIRPLFPAGYF
YDTQVLCNLLAVDGVNEPDVLAINGASVALSLSDIPWNGPVGAVRIGIIDGE
YVVNPTRKEMSSSTLNLVVAGAPKSQIVMLEASAENILQQDFCHAIKVGVKY
TQQIIQGIQQLVKETGVTKRTPQKLFTPSPEIVKYTHKLAMERLYAVFTDYEH
DKVSRDEAVNKIRLDTEEQLKEKFPEADPYEIIESFNVVAKEVFRSIVLNEYK
RCDGRDLTSLRNVSCEVDMFKTLHGSALFQRGQTQVLCTVTFDSLESGIKSD
QVITAINGIKDKNFMLHYEFPPYATNEIGKVTGLNRRELGHGALAEKALYPVI
PRDFPFTIRVTSEVLESNGSSSMASACGGSLALMDSGVPISSAVAGVAIGLVT
KTDPEKGEIEDYRLLTDILGIEDYNGDMDFKIAGTNKGITALQADIKLPGIPIKI
VMEAIQQASVAKKEILQIMNKTISKPRASRKENG
SEQ ID NO: 81 Nucleic acid sequence encoding REXO2 protein 
ATGGTCTGGGTGGACCTGGAGATGACAGGATTGGACATTGAGAAGG
ACCAGATTATTGAGATGGCCTGTCTGATAACTGACTCTGATCTCAACATTT
TGGCTGAAGGTCCTAACCTGATTATAAAACAACCAGATGAGTTGCTGGAC
AGCATGTCAGATTGGTGTAAGGAGCATCACGGGAAGTCTGGCCTTACCAA
GGCAGTGAAGGAGAGTACAATTACATTGCAGCAGGCAGAGTATGAATTT
CTGTCCTTTGTACGACAGCAGACTCCTCCAGGGCTCTGTCCACTTGCAGG
AAATTCAGTTCATGAAGATAAGAAGTTTCTTGACAAATACATGCCCCAGT
TCATGAAACATCTTCATTATAGAATAATTGATGTGAGCACTGTTAAAGAA
CTGTGCAGACGCTGGTATCCAGAAGAATATGAATTTGCACCAAAGAAGG
CTGCTTCTCATAGGGCACTTGATGACATTAGTGAAAGCATCAAAGAGCTT
SEQ ID NO: 82 REXO2 protein sequence 
MVWVDLEMTGLDIEKDQIIEMACLITDSDLNILAEGPNLIIKQPDELLDS
MSDWCKEHHGKSGLTKAVKESTITLQQAEYEFLSFVRQQTPPGLCPLAGNSV
HEDKKFLDKYMPQFMKHLHYRIIDVSTVKELCRRWYPEEYEFAPKKAASHR
ALDDISESIKEL
SEQ ID NO: 83 Nucleic acid sequence encoding MRPL44 protein 
GATCTTCTCAAAACTGCATTTGTTAATAGCTGCTATATTAAAAGTGAG
GAGGCCAAACGCCAACAACTTGGGATAGAGAAAGAAGCTGTTCTTCTGA
ATCTTAAAAGTAATCAAGAACTATCCGAACAAGGGACATCTTTTTCACAG
ACTTGCCTTACACAGTTTCTTGAAGACGAGTACCCAGACATGCCCACTGA
AGGCATAAAAAATCTTGTTGACTTTCTCACTGGTGAGGAAGTCGTGTGTC
ACGTGGCTAGAAACTTGGCTGTGGAGCAGTTAACACTGAGTGAAGAATTC
CCAGTGCCCCCAGCTGTGTTACAGCAGACTTTCTTTGCAGTTATTGGAGCC
CTGTTACAGAGCAGTGGACCTGAGAGGACTGCACTTTTCATCAGGGACTT
CTTAATTACTCAAATGACTGGAAAAGAGCTC
SEQ ID NO: 84 MRPL44 protein sequence 
DLLKTAFVNSCYIKSEEAKRQQLGIEKEAVLLNLKSNQELSEQGTSFSQT
CLTQFLEDEYPDMPTEGIKNLVDFLTGEEVVCHVARNLAVEQLTLSEEFPVPP
AVLQQTFFAVIGALLQSSGPERTALFIRDFLITQMTGKEL
SEQ ID NO: 85 Nucleic acid sequence encoding LACTB2 protein 
ATGGCTGCTGTACTGCAGCGCGTCGAGCGGCTGTCCAATCGAGTCGT
GCGTGTGTTGGGCTGTAACCCGGGTCCCATGACCCTCCAAGGCACCAACA
CCTACCTAGTGGGGACCGGCCCCAGGAGAATCCTCATTGACACTGGAGAA
 
CCAGCAATTCCAGAATACATCAGCTGTTTAAAGCAGGCTCTAACTGAATT
TAACACAGCAATCCAGGAAATTGTAGTGACTCACTGGCACCGAGATCATT
CTGGAGGCATAGGAGATATTTGTAAAAGCATCAATAATGACACTACCTAT
TGCATTAAAAAACTCCCACGGAATCCTCAGAGAGAAGAAATTATAGGAA
ATGGAGAGCAACAATATGTTTATCTGAAAGATGGAGATGTGATTAAGACT
GAGGGAGCCACTCTAAGAGTTCTATATACCCCTGGCCACACTGATGATCA
CATGGCTCTACTCTTAGAAGAGGAAAATGCTATCTTTTCTGGAGATTGCA
TCCTAGGGGAAGGAACAACGGTATTTGAAGACCTCTATGATTATATGAAC
TCTTTAAAAGAGTTATTGAAAATCAAAGCTGATATTATATATCCAGGACA
TGGCCCAGTAATTCATAATGCTGAAGCTAAAATTCAACAATACATTTCTC
ACAGAAATATTCGAGAGCAGCAAATTCTTACATTATTTCGTGAGAACTTT
GAGAAATCATTTACAGTAATGGAGCTTGTAAAAATTATTTACAAGAATAC
TCCTGAGAATTTACATGAAATGGCTAAACATAATCTCTTACTTCATTTGAA
AAAACTAGAAAAAGAAGGAAAAATATTTAGCAACACAGATCCTGACAAG
AAATGGAAAGCTCATCTT
SEQ ID NO: 86 LACTB2 protein sequence 
MAAVLQRVERLSNRVVRVLGCNPGPMTLQGTNTYLVGTGPRRILIDTG
EPAIPEYISCLKQALTEFNTAIQEIVVTHWHRDHSGGIGDICKSINNDTTYCIK
KLPRNPQREEIIGNGEQQYVYLKDGDVIKTEGATLRVLYTPGHTDDHMALLL
EEENAIFSGDCILGEGTTVFEDLYDYMNSLKELLKIKADIIYPGHGPVIHNAEA
KIQQYISHRNIREQQILTLFRENFEKSFTVMELVKIIYKNTPENLHEMAKHNLL
LHLKKLEKEGKIFSNTDPDKKWKAHL
SEQ ID NO: 87 Nucleic acid sequence encoding TSEN15 protein 
ATGGAGGAGCGCGGCGATTCCGAGCCGACCCCCGGCTGCAGCGGCC
TGGGTCCGGGCGGTGTTCGCGGCTTTGGCGACGGCGGTGGAGCTCCTTCG
TGGGCCCCTGAGGACGCCTGGATGGGCACTCACCCTAAGTATCTAGAAAT
GATGGAATTAGATATAGGAGATGCCACCCAAGTTTATGTAGCGTTCTTGG
TTTACCTGGACCTCATGGAAAGCAAAAGCTGGCATGAAGTAAACTGTGTA
GGATTACCAGAACTCCAGCTCATCTGCCTTGTTGGTACTGAGATAGAAGG
GGAGGGGTTACAGACTGTGGTGCCTACCCCCATCACTGCTTCCCTCAGCC
ATAACAGGATAAGGGAGATCTTGAAGGCATCTCGAAAGTTGCAAGGTGA
TCCAGATTTGCCGATGTCTTTTACTTTGGCCATAGTGGAGTCTGATTCTAC
AATAGTCTATTATAAACTTACTGATGGATTTATGCTGCCAGACCCTCAGA
ATATTTCTCTTAGAAGA
SEQ ID NO: 88 TSEN15 protein sequence 
MEERGDSEPTPGCSGLGPGGVRGFGDGGAPSWAPEDAWMGTHPKYL
EMMELDIGDATQVYVAFLVYLDLMESKSWHEVNCVGLPELQLICLVGTEIE
GEGLQTVVPTPITASLSHNRIREILKASRKLQGDPDLPMSFTLAIVESDSTIVY
YKLTDGFMLPDPQNISLRR
SEQ ID NO: 89 Nucleic acid sequence encoding ZC3H12A protein 
GGTGGTGGCACCCCTAAGGCTCCCAACCTGGAGCCTCCACTCCCAGA
AGAGGAAAAGGAGGGCAGCGACCTGAGACCAGTGGTCATCGATGGGAGC
AACGTGGCCATGAGCCATGGGAACAAGGAGGTCTTCTCCTGCCGGGGCAT
CCTGCTGGCAGTGAACTGGTTTCTGGAGCGGGGCCACACAGACATCACAG
TGTTTGTGCCATCCTGGAGGAAGGAGCAGCCTCGGCCCGACGTGCCCATC
ACAGACCAGCACATCCTGCGGGAACTGGAGAAGAAGATCCTGGTGT
TCACACCATCACGACGCGTGGGTGGCAAGCGGGTGGTGTGCTATGACGAC
 
AGATTCATTGTGAAGCTGGCCTACGAGTCTGACGGGATCGTGGTTTCCAA
CGACACATACCGTGACCTCCAAGGCGAGCGGCAGGAGTGGAAGCGCTTC
ATCGAGGAGCGGCTGCTCATGTACTCCTTCGTCAATGACAAGTTTATGCC
CCCTGATGACCCACTGGGCCGGCACGGGCCCAGCCTGGACAACTTCCTGC
GTAAGAAGCCACTC
SEQ ID NO: 90 ZC3H12A protein sequence 
GGGTPKAPNLEPPLPEEEKEGSDLRPVVIDGSNVAMSHGNKEVFSCRGIL
LAVNWFLERGHTDITVFVPSWRKEQPRPDVPITDQHILRELEKKKILVFTPSR
RVGGKRVVCYDDRFIVKLAYESDGIVVSNDTYRDLQGERQEWKRFIEERLL
MYSFVNDKFMPPDDPLGRHGPSLDNFLRKKPL
SEQ ID NO: 91 XTEN linker peptide sequence
   SGSETPGTSESATPES
SEQ ID NO: 92 Linker peptide protein sequence
   GGGGS
SEQ ID NO: 93 (CTG)14 nucleic acid sequence
   CTGCTGCTGCTGCTGCTGCTGCTGCTGCTGCTGCTGCTGCTG
SEQ ID NO: 94 mCherry-(CTG)14 nucleic acid sequence 
ATGGTGAGCAAGGGCGAGGAGGATAACATGGCCATCATCAAGGAGT
TCATGCGCTTCAAGGTGCACATGGAGGGCTCCGTGAACGGCCACGAGTTC
GAGATCGAGGGCGAGGGCGAGGGCCGCCCCTACGAGGGCACCCAGACCG
CCAAGCTGAAGGTGACCAAGGGTGGCCCCCTGCCCTTCGCCTGGGACATC
CTGTCCCCTCAGTTCATGTACGGCTCCAAGGCCTACGTGAAGCACCCCGC
CGACATCCCCGACTACTTGAAGCTGTCCTTCCCCGAGGGCTTCAAGTGGG
AGCGCGTGATGAACTTCGAGGACGGCGGCGTGGTGACCGTGACCCAGGA
CTCCTCCCTGCAGGACGGCGAGTTCATCTACAAGGTGAAGCTGCGCGGCA
CCAACTTCCCCTCCGACGGCCCCGTAATGCAGAAGAAGACTATGGGCTGG
GAGGCCTCCTCCGAGCGGATGTACCCCGAGGACGGCGCCCTGAAGGGCG
AGATCAAGCAGAGGCTGAAGCTGAAGGACGGCGGCCACTACGACGCTGA
GGTCAAGACCACCTACAAGGCCAAGAAGCCCGTGCAGCTGCCCGGCGCC
TACAACGTCAACATCAAGTTGGACATCACCTCCCACAACGAGGACTACAC
CATCGTGGAACAGTACGAACGCGCCGAGGGCCGCCACTCCACCGGCGGC
ATGGACGAGCTGTACAAGTAGGTTGCTAGCTGCTGCTGCTGCTGCTG
CTGCTGCTGCTGCTGCTGCTGCTCGAG
SEQ ID NO: 95 PUF-ANG protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVDTGNGSQMV
MGLGVLLLVFVLGLGLTPPTLAQDNSRYTHFLTQHYDAKPQGRDDRYCESI
MRRRGLTSPCKDINTFIHGNKRSIKAICENKNGNPHRENLRISKSSFQVTTCKL
HGGSPWPPCQYRATAGFRNVVVACENGLPVHLDQSIFRRP
SEQ ID NO: 96 PUF-ANG-S protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
 
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVDTGNGSQQDN
SRYTHFLTQHYDAKPQGRDDRYCESIMRRRGLTSPCKDINTFIHGNKRSIKAI
CENKNGNPHRENLRISKSSFQVTTCKLHGGSPWPPCQYRATAGFRNVVVAC
ENGLPVHLDQSIFRRP
SEQ ID NO: 97 PUF-RNASEL protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVDTGNGSQIDE
KYKIADTSEGGIYLGFYEKQEVAVKTFCEGSPRAQREVSCLQSSRENSHLVTF
YGSESHRGHLFVCVTLCEQTLEACLDVHRGEDVENEEDEFARNVLSSIFKAV
QELHLSCGYTHQDLQPQNILIDSKKAAHLADFDKSIKWAGDPQEVKRDLEDL
GRLVLYVVKKGSISFEDLKAQSNEEVVQLSPDEETKDLIHRLFHPGEHVRDC
LSDLLGHPFFWTWESRYRTLRNVGNESDIKTRKSESEILRLLQPGPSEHSKSF
DKWTTKINECVMKKMNKFYEKRGNFYQNTVGDLLKFIRNLGEHIDEEKHKK
MKLKIGDPSLYFQKTFPDLVIYVYTKLQNTEYRKHFPQTHSPN
SEQ ID NO: 98 PUF-RRP44 protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVDTGNGSQHYL
LPDTNVLLHQIDVLEDPAIRNVIVLQTVLQEVRNRSAPVYKRIRDVTNNQEK
HFYTFTNEHHRETYVEQEQGENANDRNDRAIRVAAKWYNEHLKKMSADNQ
LQVIFITNDRRNKE
SEQ ID NO: 99 PUF-RNASE1 protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVDTGNGSQKES
RAKKFQRQHMDSDSSPSSSSTYCNQMMRRRNMTQGRCKPVNTFVHEPLVD
VQNVCFQEKVTCKNGQGNCYKSNSSMHITDCRLTNGSRYPNCAYRTSPKER
HIIVACEGSPYVPVHFDASVEDST
SEQ ID NO: 100 PUF-PNPT1 protein sequence GRSRLLEDFRNNRYPNLQLREIAGHIMEFSQDQHGSRFIELKLERATPAE 
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVDTGNGSQAVA
VDLGNRKLEISSGKLARFADGSAVVQSGDTAVMVTAVSKTKPSPSQFMPLV
VDYRQKAAAAGRIPTNYLRREIGTSDKEILTSRIIDRSIRPLFPAGYFYDTQVL
CNLLAVDGVNEPDVLAINGASVALSLSDIPWNGPVGAVRIGIIDGEYVVNPT
RKEMSSSTLNLVVAGAPKSQIVMLEASAENILQQDFCHAIKVGVKYTQQIIQ
GIQQLVKETGVTKRTPQKLFTPSPEIVKYTHKLAMERLYAVFTDYEHDKVSR
DEAVNKIRLDTEEQLKEKFPEADPYEIIESFNVVAKEVFRSIVLNEYKRCDGR
DLTSLRNVSCEVDMFKTLHGSALFQRGQTQVLCTVTFDSLESGIKSDQVITAI
NGIKDKNFMLHYEFPPYATNEIGKVTGLNRRELGHGALAEKALYPVIPRDFP
FTIRVTSEVLESNGSSSMASACGGSLALMDSGVPISSAVAGVAIGLVTKTDPE
KGEIEDYRLLTDILGIEDYNGDMDFKIAGTNKGITALQADIKLPGIPIKIVMEAI
QQASVAKKEILQIMNKTISKPRASRKENG
SEQ ID NO: 101 PUF-REXO2 protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVDTGNGSQMV
WVDLEMTGLDIEKDQIIEMACLITDSDLNILAEGPNLIIKQPDELLDSMSDWC
KEHHGKSGLTKAVKESTITLQQAEYEFLSFVRQQTPPGLCPLAGNSVHEDKK
FLDKYMPQFMKHLHYRIIDVSTVKELCRRWYPEEYEFAPKKAASHRALDDIS
ESIKEL
SEQ ID NO: 102 PUF-MRPL44 protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVDTGNGSQDLL
KTAFVNSCYIKSEEAKRQQLGIEKEAVLLNLKSNQELSEQGTSFSQTCLTQFL
EDEYPDMPTEGIKNLVDFLTGEEVVCHVARNLAVEQLTLSEEFPVPPAVLQQ
TFFAVIGALLQSSGPERTALFIRDFLITQMTGKEL
SEQ ID NO: 103 PUF-LACTB2 protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
 
RKIVMHKIRPHIATLRKYTYGKHILAKLEKYYMKNGVDLGVDTGNGSQMA
AVLQRVERLSNRVVRVLGCNPGPMTLQGTNTYLVGTGPRRILIDTGEPAIPE
YISCLKQALTEFNTAIQEIVVTHWHRDHSGGIGDICKSINNDTTYCIKKLPRNP
QREEIIGNGEQQYVYLKDGDVIKTEGATLRVLYTPGHTDDHMALLLEEENAI
FSGDCILGEGTTVFEDLYDYMNSLKELLKIKADIIYPGHGPVIHNAEAKIQQYI
SHRNIREQQILTLFRENFEKSFTVMELVKIIYKNTPENLHEMAKHNLLLHLKK
LEKEGKIFSNTDPDKKWKAHL
SEQ ID NO: 104 PUF-TSEN15 protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVDTGNGSQMEE
RGDSEPTPGCSGLGPGGVRGFGDGGGAPSWAPEDAWMGTHPKYLEMMELD
IGDATQVYVAFLVYLDLMESKSWHEVNCVGLPELQLICLVGTEIEGEGLQTV
VPTPITASLSHNRIREILKASRKLQGDPDLPMSFTLAIVESDSTIVYYKLTDGF
MLPDPQNISLRR
SEQ ID NO: 105 PUF-ZC3H12A protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVDTGNGSQGGG
TPKAPNLEPPLPEEEKEGSDLRPVVIDGSNVAMSHGNKEVFSCRGILLAVNW
FLERGHTDITVFVPSWRKEQPRPDVPITDQHILRELEKKKILVFTPSRRVGGKR
VVCYDDRFIVKLAYESDGIVVSNDTYRDLQGERQEWKRFIEERLLMYSFVND
KFMPPDDPLGRHGPSLDNFLRKKPL
SEQ ID NO: 106 ANG-PUF protein sequence 
MVMGLGVLLLVFVLGLGLTPPTLAQDNSRYTHFLTQHYDAKPQGRDDR
YCESIMRRRGLTSPCKDINTFIHGNKRSIKAICENKNGNPHRENLRISKSSFQV
TTCKLHGGSPWPPCQYRATAGFRNVVVACENGLPVHLDQSIFRRPVDTGNG
SQGRSRLLEDFRNNRYPNLQLREIAGHIMEFSQDQHGSRFIELKLERATPAER
QLVFNEILQAAYQLMVDVFGNYVIQKFFEFGSLEQKLALAERIRGHVLSLAL
QMYGSYVIRKALEFIPSDQQNEMVRELDGHVLKCVKDQNGSYVVEKCIECV
QPQSLQFIIDAFKGQVFALSTHPYGNRVIQRILEHCLPDQTLPILEELHQHTEQ
LVQDQYGSYVIRHVLEHGRPEDKSKIVAEIRGNVLVLSQHKFASNVVEKCVT
HASRTERAVLIDEVCTMNDGPHSALYTMMKDQYANYVVQKMIDVAEPGQR
KIVMHKIRPHIATLRKYTYGKHILAKLEKYYMKNGVDLG
SEQ ID NO: 107 ANG-S-PUF protein sequence 
QDNSRYTHFLTQHYDAKPQGRDDRYCESIMRRRGLTSPCKDINTFIHGN
KRSIKAICENKNGNPHRENLRISKSSFQVTTCKLHGGSPWPPCQYRATAGFRN
VVVACENGLPVHLDQSIFRRPVDTGNGSQGRSRLLEDFRNNRYPNLQLREIA
GHIMEFSQDQHGSRFIELKLERATPAERQLVFNEILQAAYQLMVDVFGNYVI
 
QKFFEFGSLEQKLALAERIRGHVLSLALQMYGSYVIRKALEFIPSDQQNEMV
RELDGHVLKCVKDQNGSYVVEKCIECVQPQSLQFIIDAFKGQVFALSTHPYG
NRVIQRILEHCLPDQTLPILEELHQHTEQLVQDQYGSYVIRHVLEHGRPEDKS
KIVAEIRGNVLVLSQHKFASNVVEKCVTHASRTERAVLIDEVCTMNDGPHSA
LYTMMKDQYANYVVQKMIDVAEPGQRKIVMHKIRPHIATLRKYTYGKHILA
KLEKYYMKNGVDLG
SEQ ID NO: 108 RNASE1-PUF protein sequence 
KESRAKKFQRQHMDSDSSPSSSSTYCNQMMRRRNMTQGRCKPVNTFV
HEPLVDVQNVCFQEKVTCKNGQGNCYKSNSSMHITDCRLTNGSRYPNCAYR
TSPKERHIIVACEGSPYVPVHFDASVEDSTVDTGNGSQGRSRLLEDFRNNRYP
NLQLREIAGHIMEFSQDQHGSRFIELKLERATPAERQLVFNEILQAAYQLMVD
VFGNYVIQKFFEFGSLEQKLALAERIRGHVLSLALQMYGSYVIRKALEFIPSD
QQNEMVRELDGHVLKCVKDQNGSYVVEKCIECVQPQSLQFIIDAFKGQVFA
LSTHPYGNRVIQRILEHCLPDQTLPILEELHQHTEQLVQDQYGSYVIRHVLEH
GRPEDKSKIVAEIRGNVLVLSQHKFASNVVEKCVTHASRTERAVLIDEVCTM
NDGPHSALYTMMKDQYANYVVQKMIDVAEPGQRKIVMHKIRPHIATLRKY
TYGKHILAKLEKYYMKNGVDLG
SEQ ID NO: 109 PNPT1-PUF protein sequence 
AVAVDLGNRKLEISSGKLARFADGSAVVQSGDTAVMVTAVSKTKPSPS
QFMPLVVDYRQKAAAAGRIPTNYLRREIGTSDKEILTSRIIDRSIRPLFPAGYF
YDTQVLCNLLAVDGVNEPDVLAINGASVALSLSDIPWNGPVGAVRIGIIDGE
YVVNPTRKEMSSSTLNLVVAGAPKSQIVMLEASAENILQQDFCHAIKVGVKY
TQQIIQGIQQLVKETGVTKRTPQKLFTPSPEIVKYTHKLAMERLYAVFTDYEH
DKVSRDEAVNKIRLDTEEQLKEKFPEADPYEIIESFNVVAKEVFRSIVLNEYK
RCDGRDLTSLRNVSCEVDMFKTLHGSALFQRGQTQVLCTVTFDSLESGIKSD
QVITAINGIKDKNFMLHYEFPPYATNEIGKVTGLNRRELGHGALAEKALYPVI
PRDFPFTIRVTSEVLESNGSSSMASACGGSLALMDSGVPISSAVAGVAIGLVT
KTDPEKGEIEDYRLLTDILGIEDYNGDMDFKIAGTNKGITALQADIKLPGIPIKI
VMEAIQQASVAKKEILQIMNKTISKPRASRKENGVDTGNGSQGRSRLLEDFR
NNRYPNLQLREIAGHIMEFSQDQHGSRFIELKLERATPAERQLVFNEILQAAY
QLMVDVFGNYVIQKFFEFGSLEQKLALAERIRGHVLSLALQMYGSYVIRKAL
EFIPSDQQNEMVRELDGHVLKCVKDQNGSYVVEKCIECVQPQSLQFIIDAFK
GQVFALSTHPYGNRVIQRILEHCLPDQTLPILEELHQHTEQLVQDQYGSYVIR
HVLEHGRPEDKSKIVAEIRGNVLVLSQHKFASNVVEKCVTHASRTERAVLID
EVCTMNDGPHSALYTMMKDQYANYVVQKMIDVAEPGQRKIVMHKIRPHIA
TLRKYTYGKHILAKLEKYYMKNGVDLG
SEQ ID NO: 110 REXO2-PUF protein sequence 
MVWVDLEMTGLDIEKDQIIEMACLITDSDLNILAEGPNLIIKQPDELLDS
MSDWCKEHHGKSGLTKAVKESTITLQQAEYEFLSFVRQQTPPGLCPLAGNSV
HEDKKFLDKYMPQFMKHLHYRIIDVSTVKELCRRWYPEEYEFAPKKAASHR
ALDDISESIKELVDTGNGSQGRSRLLEDFRNNRYPNLQLREIAGHIMEFSQDQ
HGSRFIELKLERATPAERQLVFNEILQAAYQLMVDVFGNYVIQKFFEFGSLEQ
KLALAERIRGHVLSLALQMYGSYVIRKALEFIPSDQQNEMVRELDGHVLKCV
KDQNGSYVVEKCIECVQPQSLQFIIDAFKGQVFALSTHPYGNRVIQRILEHCL
PDQTLPILEELHQHTEQLVQDQYGSYVIRHVLEHGRPEDKSKIVAEIRGNVLV
LSQHKFASNVVEKCVTHASRTERAVLIDEVCTMNDGPHSALYTMMKDQYA
NYVVQKMIDVAEPGQRKIVMHKIRPHIATLRKYTYGKHILAKLEKYYMKNG
 VDLG
SEQ ID NO: 111 ZC3H12A-PUF protein sequence 
GGGTPKAPNLEPPLPEEEKEGSDLRPVVIDGSNVAMSHGNKEVFSCRGIL
LAVNWFLERGHTDITVFVPSWRKEQPRPDVPITDQHILRELEKKKILVFTPSR
RVGGKRVVCYDDRFIVKLAYESDGIVVSNDTYRDLQGERQEWKRFIEERLL
MYSFVNDKFMPPDDPLGRHGPSLDNFLRKKPLVDTGNGSQGRSRLLEDFRN
NRYPNLQLREIAGHIMEFSQDQHGSRFIELKLERATPAERQLVFNEILQAAYQ
LMVDVFGNYVIQKFFEFGSLEQKLALAERIRGHVLSLALQMYGSYVIRKALE
FIPSDQQNEMVRELDGHVLKCVKDQNGSYVVEKCIECVQPQSLQFIIDAFKG
QVFALSTHPYGNRVIQRILEHCLPDQTLPILEELHQHTEQLVQDQYGSYVIRH
VLEHGRPEDKSKIVAEIRGNVLVLSQHKFASNVVEKCVTHASRTERAVLIDE
VCTMNDGPHSALYTMMKDQYANYVVQKMIDVAEPGQRKIVMHKIRPHIAT
LRKYTYGKHILAKLEKYYMKNGVDLG
SEQ ID NO: 112 Nucleic acid sequence encoding RNASET2 protein 
ATGCGCCCTGCAGCCCTGCGCGGGGCCCTGCTGGGCTGCCTCTGCCT
GGCGTTGCTTTGCCTGGGCGGTGCGGACAAGCGCCTGCGTGACAACCATG
AGTGGAAAAAACTAATTATGGTTCAGCACTGGCCTGAGACAGTATGCGA
GAAAATTCAAAACGACTGTAGAGACCCTCCGGATTACTGGACAATACATG
GACTATGGCCCGATAAAAGTGAAGGATGTAATAGATCGTGGCCCTTCAAT
TTAGAAGAGATTAAGGATCTTTTGCCAGAAATGAGGGCATACTGGCCTGA
CGTAATTCACTCGTTTCCCAATCGCAGCCGCTTCTGGAAGCATGAGTGGG
AAAAGCATGGGACCTGCGCCGCCCAGGTGGATGCGCTCAACTCCCAGAA
GAAGTACTTTGGCAGAAGCCTGGAACTCTACAGGGAGCTGGACCTCAAC
AGTGTGCTTCTAAAATTGGGGATAAAACCATCCATCAATTACTACCAAGT
TGCAGATTTTAAAGATGCCCTTGCCAGAGTATATGGAGTGATACCCAAAA
TCCAGTGCCTTCCACCAAGCCAGGATGAGGAAGTACAGACAATTGGTCAG
ATAGAACTGTGCCTCACTAAGCAAGACCAGCAGCTGCAAAACTGCACCG
AGCCGGGGGAGCAGCCGTCCCCCAAGCAGGAAGTCTGGCTGGCAAATGG
GGCCGCCGAGAGCCGGGGTCTGAGAGTCTGTGAAGATGGCCCAGTCTTCT
ATCCCCCACCTAAAAAGACCAAGCAT
SEQ ID NO: 113 RNASET2 protein sequence 
MRPAALRGALLGCLCLALLCLGGADKRLRDNHEWKKLIMVQHWPETV
CEKIQNDCRDPPDYWTIHGLWPDKSEGCNRSWPFNLEEIKDLLPEMRAYWP
DVIHSFPNRSRFWKHEWEKHGTCAAQVDALNSQKKYFGRSLELYRELDLNS
VLLKLGIKPSINYYQVADFKDALARVYGVIPKIQCLPPSQDEEVQTIGQIELCL
TKQDQQLQNCTEPGEQPSPKQEVWLANGAAESRGLRVCEDGPVFYPPPKKT
KH
SEQ ID NO: 114 Nucleic acid sequence encoding SUPV3L1 protein 
ATGTCCTTCTCCCGTGCCCTATTGTGGGCTCGGCTCCCGGCGGGGCGC
CAGGCTGGCCACCGGGCAGCCATCTGCTCTGCCCTTCGTCCCCACTTTGG
GCCCTTTCCCGGGGTTCTGGGGCAAGTTTCTGTCCTTGCCACCGCCTCCTC
CTCTGCCTCCGGTGGCTCCAAAATACCAAACACGTCCTTGTTCGTGCCCCT
GACTGTGAAACCTCAGGGCCCCAGCGCCGACGGCGACGTCGGGGCCGAG
CTAACCCGGCCTCTGGACAAGAATGAAGTAAAGAAGGTCTTAGACAAAT
TTTACAAGAGGAAAGAAATTCAGAAACTGGGTGCTGATTATGGACTTGAT
GCTCGTCTCTTCCACCAAGCTTTCATAAGCTTTAGAAATTATATTATGCAG
TCTCATTCCCTGGATGTGGACATTCACATTGTTTTGAATGATATTTGCTTC
 
GGTGCAGCTCATGCGGATGATTTATTCCCATTTTTCTTGAGACATGCCAAA
CAAATATTTCCTGTGTTGGACTGTAAGGATGATCTACGTAAAATCAGTGA
CTTAAGAATACCACCTAACTGGTACCCAGATGCTAGAGCCATGCAGCGGA
AGATAATATTTCATTCAGGCCCCACAAACAGTGGAAAGACTTATCACGCA
ATCCAGAAATACTTCTCAGCAAAGTCTGGAGTGTATTGTGGCCCTCTAAA
ATTACTGGCACATGAGATCTTCGAAAAGAGTAATGCTGCTGGTGTGCCAT
GTGACTTGGTGACAGGTGAAGAGCGTGTGACAGTTCAGCCAAATGGGAA
ACAGGCTTCACATGTTTCTTGTACAGTTGAGATGTGCAGTGTTACAACTCC
TTATGAAGTGGCTGTAATTGATGAAATTCAAATGATTAGAGATCCAGCCA
GAGGATGGGCCTGGACCAGAGCACTTCTAGGACTGTGTGCTGAAGAGGTT
CATTTGTGTGGAGAACCTGCTGCTATTGACCTGGTGATGGAGCTTATGTA
CACAACGGGGGAGGAAGTGGAGGTTCGAGACTATAAGAGGCTTACCCCC
ATTTCTGTGCTGGACCATGCACTAGAATCTTTAGATAACCTTCGGCCTGG
GGACTGCATTGTCTGTTTTAGCAAGAATGATATTTATTCTGTGAGTCGGCA
GATTGAAATTCGGGGATTAGAATCAGCTGTTATATATGGCAGTCTCCCAC
CTGGGACCAAACTTGCTCAAGCAAAAAAGTTTAATGATCCCAATGACCCA
TGCAAAATCTTGGTTGCTACAGATGCAATTGGCATGGGACTTAATTTGAG
CATAAGGAGAATTATTTTTTACTCCCTTATAAAGCCCAGTATCAATGAAA
AGGGAGAGAGAGAACTAGAACCAATCACAACCTCTCAAGCCCTGCAGAT
TGCTGGCAGAGCTGGCAGATTCAGCTCACGGTTTAAAGAAGGAGAGGTT
ACAACAATGAATCATGAAGATCTCAGTTTATTAAAGGAAATTTTGAAGAG
GCCTGTGGATCCTATAAGGGCAGCTGGTCTTCATCCAACTGCTGAGCAGA
TTGAAATGTTTGCCTACCATCTCCCTGATGCAACACTGTCCAATCTCATTG
ATATTTTTGTAGACTTTTCACAAGTTGATGGGCAGTATTTTGTCTGCAATA
TGGATGATTTTAAATTTTCTGCAGAGTTGATCCAGCATATTCCACTAAGTC
TGCGAGTGAGGTATGTTTTCTGCACAGCTCCTATCAACAAGAAGCAGCCT
TTTGTGTGTTCTTCACTGTTACAGTTTGCCAGGCAGTATAGCAGGAATGA
GCCCCTGACCTTTGCATGGTTACGCCGATACATCAAATGGCCTTTACTTCC
ACCTAAGAATATTAAAGACCTCATGGATCTTGAAGCTGTCCACGATGTCT
TGGATCTTTACTTGTGGCTAAGCTACCGATTTATGGATATGTTTCCAGATG
CCAGCCTTATTCGAGATCTCCAGAAAGAACTAGATGGTATTATCCAAGAT
GGTGTGCACAATATCACTAAATTGATTAAAATGTCTGAGACGCATAAGCT
GTTGAATTTGGAGGGCTTTCCATCAGGGAGCCAGTCACGATTGTCAGGAA
CCTTAAAGAGCCAAGCTAGAAGGACACGCGGCACCAAAGCTCTAGGGAG
TAAAGCTACTGAGCCACCCAGCCCCGATGCAGGAGAGCTGTCCCTTGCTT
CCAGATTGGTGCAGCAAGGACTCCTCACTCCAGACATGCTGAAACAGCTA
GAAAAAGAGTGGATGACACAACAAACTGAACACAACAAAGAAAAAACA
GAGTCTGGGACTCATCCAAAAGGGACGAGAAGAAAGAAGAAGGAACCTG
ATTCGGAC
SEQ ID NO: 115 SUPV3L1 protein sequence 
MSFSRALLWARLPAGRQAGHRAAICSALRPHFGPFPGVLGQVSVLATAS
SSASGGSKIPNTSLFVPLTVKPQGPSADGDVGAELTRPLDKNEVKKVLDKFY
KRKEIQKLGADYGLDARLFHQAFISFRNYIMQSHSLDVDIHIVLNDICFGAAH
ADDLFPFFLRHAKQIFPVLDCKDDLRKISDLRIPPNWYPDARAMQRKIIFHSG
PTNSGKTYHAIQKYFSAKSGVYCGPLKLLAHEIFEKSNAAGVPCDLVTGEER
VTVQPNGKQASHVSCTVEMCSVTTPYEVAVIDEIQMIRDPARGWAWTRALL
GLCAEEVHLCGEPAAIDLVMELMYTTGEEVEVRDYKRLTPISVLDHALESLD
 
NLRPGDCIVCFSKNDIYSVSRQIEIRGLESAVIYGSLPPGTKLAQAKKFNDPND
PCKILVATDAIGMGLNLSIRRIIFYSLIKPSINEKGERELEPITTSQALQIAGRAG
RFSSRFKEGEVTTMNHEDLSLLKEILKRPVDPIRAAGLHPTAEQIEMFAYHLP
DATLSNLIDIFVDFSQVDGQYFVCNMDDFKFSAELIQHIPLSLRVRYVFCTAPI
NKKQPFVCSSLLQFARQYSRNEPLTFAWLRRYIKWPLLPPKNIKDLMDLEAV
HDVLDLYLWLSYRFMDMFPDASLIRDLQKELDGIIQDGVHNITKLIKMSETH
KLLNLEGFPSGSQSRLSGTLKSQARRTRGTKALGSKATEPPSPDAGELSLASR
LVQQGLLTPDMLKQLEKEWMTQQTEHNKEKTESGTHPKGTRRKKKEPDSD
SEQ ID NO: 116 PUF-RNASET2 protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVEEHRRSTDKR
LRDNHEWKKLIMVQHWPETVCEKIQNDCRDPPDYWTIHGLWPDKSEGCNR
SWPFNLEEIKDLLPEMRAYWPDVIHSFPNRSRFWKHEWEKHGTCAAQVDAL
NSQKKYFGRSLELYRELDLNSVLLKLGIKPSINYYQVADFKDALARVYGVIP
KIQCLPPSQDEEVQTIGQIELCLTKQDQQLQNCTEPGEQPSPKQEVWLANGA
AESRGLRVCEDGPVFYPPPKKTKH
SEQ ID NO: 117 RNASET2-PUF protein sequence 
DKRLRDNHEWKKLIMVQHWPETVCEKIQNDCRDPPDYWTIHGLWPDK
SEGCNRSWPFNLEEIKDLLPEMRAYWPDVIHSFPNRSRFWKHEWEKHGTCA
AQVDALNSQKKYFGRSLELYRELDLNSVLLKLGIKPSINYYQVADFKDALAR
VYGVIPKIQCLPPSQDEEVQTIGQIELCLTKQDQQLQNCTEPGEQPSPKQEVW
LANGAAESRGLRVCEDGPVFYPPPKKTKHVEEHRRSTGRSRLLEDFRNNRYP
NLQLREIAGHIMEFSQDQHGSRFIELKLERATPAERQLVFNEILQAAYQLMVD
VFGNYVIQKFFEFGSLEQKLALAERIRGHVLSLALQMYGSYVIRKALEFIPSD
QQNEMVRELDGHVLKCVKDQNGSYVVEKCIECVQPQSLQFIIDAFKGQVFA
LSTHPYGNRVIQRILEHCLPDQTLPILEELHQHTEQLVQDQYGSYVIRHVLEH
GRPEDKSKIVAEIRGNVLVLSQHKFASNVVEKCVTHASRTERAVLIDEVCTM
NDGPHSALYTMMKDQYANYVVQKMIDVAEPGQRKIVMHKIRPHIATLRKY
TYGKHILAKLEKYYMKNGVDLG
SEQ ID NO: 118 PUF-SUPV3L1 protein sequence 
GRSRLLEDFRNNRYPNLQLREIAGHIMEFSQDQHGSRFIELKLERATPAE
RQLVFNEILQAAYQLMVDVFGNYVIQKFFEFGSLEQKLALAERIRGHVLSLA
LQMYGSYVIRKALEFIPSDQQNEMVRELDGHVLKCVKDQNGSYVVEKCIEC
VQPQSLQFIIDAFKGQVFALSTHPYGNRVIQRILEHCLPDQTLPILEELHQHTE
QLVQDQYGSYVIRHVLEHGRPEDKSKIVAEIRGNVLVLSQHKFASNVVEKCV
THASRTERAVLIDEVCTMNDGPHSALYTMMKDQYANYVVQKMIDVAEPGQ
RKIVMHKIRPHIATLRKYTYGKHILAKLEKYYMKNGVDLGVEEHRRSTVLD
HALESLDNLRPGDCIVCFSKNDIYSVSRQIEIRGLESAVIYGSLPPGTKLAQAK
KFNDPNDPCKILVATDAIGMGLNLSIRRIIFYSLIKPSINEKGERELEPITTSQAL
QIAGRAGRFSSRFKEGEVTTMNHEDLSLLKEILKRPVDPIRAAGLHPTAEQIE
SEQ ID NO: 119 SUPV3L1-PUF protein sequence

VLDHALESLDNLRPGDCIVCFSKNDIYSVSRQIEIRGLESAVIYGSLPPGT

KLAQAKKFNDPNDPCKILVATDAIGMGLNLSIRRIIFYSLIKPSINEKGERELEP
ITTSQALQIAGRAGRFSSRFKEGEVTTMNHEDLSLLKEILKRPVDPIRAAGLHP
TAEQIEVEEHRRSTGRSRLLEDFRNNRYPNLQLREIAGHIMEFSQDQHGSRFIE
LKLERATPAERQLVFNEILQAAYQLMVDVFGNYVIQKFFEFGSLEQKLALAE
RIRGHVLSLALQMYGSYVIRKALEFIPSDQQNEMVRELDGHVLKCVKDQNG
SYVVEKCIECVQPQSLQFIIDAFKGQVFALSTHPYGNRVIQRILEHCLPDQTLP
ILEELHQHTEQLVQDQYGSYVIRHVLEHGRPEDKSKIVAEIRGNVLVLSQHKF
ASNVVEKCVTHASRTERAVLIDEVCTMNDGPHSALYTMMKDQYANYVVQK
MIDVAEPGQRKIVMHKIRPHIATLRKYTYGKHILAKLEKYYMKNGVDLG

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

## Claims

1. A set of nucleic acid constructs, comprising: a first nucleic acid construct and a second nucleic acid construct;
wherein the first nucleic acid construct comprises a first expression cassette, the first expression cassette expresses a long RNA repeat sequence, and the first expression cassette comprises a repeat sequence unit of 3-6 nucleotides, wherein the number of repetitions of the repeat sequence is 41-2000, preferably 45-1500, and more preferably 50-1000;
and the second nucleic acid construct comprises a second expression cassette, the second expression cassette expresses a short RNA repeat sequence, and the second expression cassette comprises a repeat sequence unit of 3-6 nucleotides corresponding to the first expression cassette, wherein the number of repetitions of the repeat sequence is 2-40, preferably 5-40.

2. A recombinant vector comprising the set of nucleic acid constructs according to claim 1.

3. A genetically engineered cell comprising the set of nucleic acid constructs according to claim 1, or the recombinant vector according to claim 2.

4. A kit, comprising: a container and the set of nucleic acid constructs according to claim 1 located in the container.

5. A method for screening a drug for treating long RNA repeat sequence-associated diseases, comprising the steps of:
(a) co-introducing the set of nucleic acid constructs according to claim 1 and a drug to be screened into a cell;
(b) determining whether the drug to be screened is a drug for treating long RNA repeat sequence-associated diseases according to the intensity changes of the first detectable label and the second detectable label in the set of nucleic acid constructs.

6. A system for screening a drug for treating long RNA repeat sequence-associated diseases, wherein the system comprises:
(a) the set of nucleic acid constructs according to claim 1;
(b) a drug to be screened;
wherein, when the expression level or intensity of the first detectable label in the set of nucleic acid constructs is significantly reduced, and the expression level or intensity of the second detectable label is slightly changed or substantially unchanged, it indicates that the drug to be screened is a candidate or potential drug usable for treating long RNA repeat sequence-associated diseases.

7. A screening composition for screening a drug for treating long RNA repeat sequence-associated diseases, wherein the screening composition comprises:
(a) a drug to be screened;
(b) the set of nucleic acid constructs according to claim 1;
wherein, when the expression level or intensity of the first detectable label in the set of nucleic acid constructs is significantly reduced, and the expression level or intensity of the second detectable label is slightly changed or substantially unchanged, it indicates that the drug to be screened is a candidate or potential drug usable for treating long RNA repeat sequence-associated diseases.

8. Use of the system according to claim 6 or the screening composition according to claim 7, for screening a drug for treating long RNA repeat sequence-associated diseases.

9. A composition comprising:
(a) a drug for treating long RNA repeat sequence-associated diseases obtained by the method according to claim 5 or the system according to claim 6, wherein the drug for treating long RNA repeat sequence-associated diseases comprises an artificial RNA degrading enzyme; preferably, the RNA degrading enzyme has a structure of Formula IV or IV' from the N-terminus to the C-terminus of the protein:
D1-A-L1-B-D2 (IV);
D1-B-L1-A-D2 (IV')
wherein each "-" is independently a linker peptide or a peptide bond;
A is a PUF protein fragment;
B is the full length of an RNase or an RNA helicase, or an active fragment thereof;
L1 is absent or a linker peptide;
D1 is absent or a localization peptide;
D2 is absent or a localization peptide; the full length of the RNase or RNA helicase, or an active fragment thereof, is selected from the group consisting of: PIN (from SMG6, UNIPROT: Q86US8), ANG (UNIPROT: P03950), RNASEL (UNIPROT: Q05823), RRP44 (UNIPROT: Q9Y2L1), RNASE1 (UNIPROT: P07998), RNASET2 (UNIPROT: O00584), SUPV3L1 (UNIPROT: Q8IYB8), PNPT1 (UNIPROT: Q8TCS8), REXO2 (UNIPROT: Q9Y3B8), MRPL44 (UNIPROT: Q9H9J2), LACTB2 (UNIPROT: Q53H82), TSEN15 (UNIPROT: Q8WW01), ZC3H12A (UNIPROT: Q5D1E8), an active region thereof, and sequence combination thereof.

10. A method for preparing an animal model for screening a drug for treating long RNA repeat sequence-associated diseases, wherein the method comprises the steps of:
(a) providing a cell of a non-human mammal, and introducing the set of nucleic acid constructs according to claim 1 into the cell, thereby obtaining a cell that simultaneously expresses a long RNA repeat sequence and a short RNA repeat sequence;
(b) using the cell that simultaneously expresses a long RNA repeat sequence and a short RNA repeat sequence obtained in step (a), thereby preparing an animal model for screening a drug for treating long RNA repeat sequence-associated diseases.

11. Use of a non-human mammalian model prepared by the method according to claim 10, wherein the model is used for screening or identifying a substance (therapeutic agent) for treating long RNA repeat sequence-associated diseases.

12. A method for screening or identifying a potential therapeutic agent of a drug for treating long RNA repeat sequence-associated diseases, comprising the steps of:
(a) in a test group, administering a test drug to a non-human mammalian model prepared by the method according to claim 9 in the presence of the test drug, and analyzing the expression level or intensity of the first detectable label and the second detectable label of the animal model in the test group, respectively; and in a control group without administering the test drug but under otherwise identical conditions, analyzing the expression level or intensity of the first detectable label and the second detectable label of the animal model in the control group;
(b) comparing the expression level or intensity of the first detectable label and the second detectable label of the animal models between the test group and the control group, wherein, compared with the control group, if the expression level or intensity of the first detectable label is significantly reduced and the expression level or intensity of the second detectable label is substantially unchanged in the animal model administered with the test drug, it indicates that this test compound can be used as a potential therapeutic agent for treating long RNA repeat sequence-associated diseases.

13. Use of the genetically engineered cell according to claim 3, for preparing a biological agent for constructing a non-human mammalian animal model for screening a drug for treating long RNA repeat sequence-associated diseases.
